(19) Europäisches Patentamt
European Patent Office
Office européen des brevets

(11) Veröffentlichungsnummer: **0 025 017**
**A1**

(12) # EUROPÄISCHE PATENTANMELDUNG

(21) Anmeldenummer: 80810261.0

(22) Anmeldetag: 22.08.80

(51) Int. Cl.³: C 07 D 501/20
C 07 D 501/24, C 07 D 501/59
A 61 K 31/545
//C07D285/08

(30) Priorität: 28.08.79 CH 7798 79
04.07.80 CH 5144/80

(43) Veröffentlichungstag der Anmeldung:
11.03.81 Patentblatt 81/10

(84) Benannte Vertragsstaaten:
AT BE CH DE FR GB IT LI LU NL SE

(71) Anmelder: CIBA-GEIGY AG
Patentabteilung Postfach
CH-4002 Basel(CH)

(72) Erfinder: Csendes, Ivan, Dr.
Finkenstrasse 9
CH-4104 Oberwil(CH)

(72) Erfinder: Müller, Beat, Dr.
Vogesenstrasse 31
CH-4106 Therwil(CH)

(72) Erfinder: Scartazzini, Riccardo, Dr.
Conrad Ferdinand Meyer-Strasse 38
CH-4059 Basel(CH)

(54) **Polyazathiaverbindungen, Verfahren zu ihrer Herstellung, pharmazeutische Präparate enthaltend solche Verbindungen und Verwendung von letzteren.**

(57) 7ß-Aminothiadiazolylacetylamino -3-cephem-4 -carbonsäure-Verbindungen, insbesondere der Formel

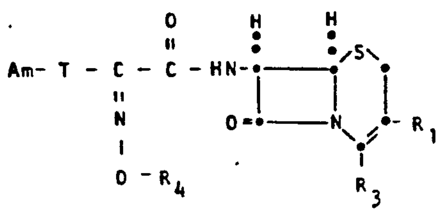

worin $R_1$ insbesondere Wasserstoff, Methoxy, Chlor, Heterocyclylthio, Carbamoylpyridiniomethyl, Azidomethyl oder Acylmethyl, $R_3$ insbesondere Carboxy oder physiologisch spaltbares, verestertes Carboxy, $R_4$ insbesondere Wasserstoff, Niederalkyl, Carbamoyl oder N-substitutiertes Carbamoyl, Am in erster Linie Amino, und T einen zweiwertigen Thiadiazolrest darstellen, oder Salz von solchen Verbindungen, zeigen antibiotische Eigenschaften gegen gram-positive und gram-negative Mikroorganismen. Ebenfalls umfasst sind Zwischenprodukte.

Verfahren zur Herstellung der Verbindungen sind auch Teil der Erfindung.

EP 0 025 017 A1

Croydon Printing Company Ltd.

CIBA-GEIGY AG 4-12493/+
Basel (Schweiz)

Polyazathiaverbindungen, Verfahren zu ihrer Herstellung, pharmazeutische Präparate enthaltend solche Verbindungen und Verwendung von letzteren.

Die Erfindung betrifft neue 7β-Aminothiadiazolylacetamido-3-cephem-4-carbonsäureverbindungen, Verfahren zu ihrer Herstellung, pharmazeutische Präparate, die solche Verbindungen enthalten, und ihre Verwendung zur Herstellung von pharmazeutischen Präparaten oder als pharmakologisch wirksame Verbindungen.

Die Erfindung betrifft insbesondere 7β-Aminothiadiazolylacetylamino-3-cephem-4-carbonsäureverbindungen der Formel

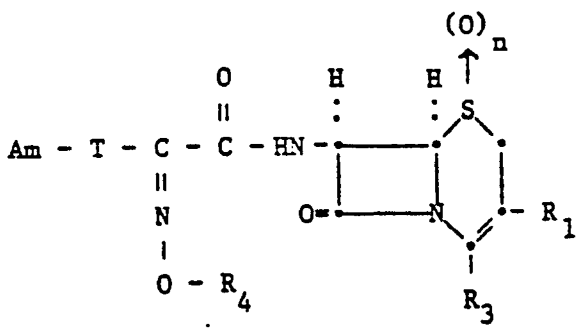

(I),

worin n für 0 oder 1 steht, $R_1$ für Wasserstoff, veräthertes oder verestertes Hydroxy, veräthertes Mercapto oder den Rest der Formel $-CH_2-R_2$ steht, worin $R_2$ Acyl, gegebenenfalls funktionell abgewandeltes Carboxy, eine Ammoniogruppe oder Azido darstellt, $R_3$ Carboxy oder in physiologisch spaltbarer oder geschützter Form vorliegendes funktionell abgewandeltes Carboxy bedeutet, Am für gegebenenfalls substituiertes Amino steht, T einen zweiwertigen, über die Ringkohlenstoffatome mit Am und dem Kohlenstoffatom der Gruppe der Formel $-C(=N-OR_4)-$ verbundenen Thiadiazolrest bedeutet, und $R_4$ Wasserstoff, gegebenenfalls substituiertes Niederalkyl oder Cycloalkyl oder gegebenenfalls N-substituiertes Carbamoyl darstellt, und Salze von solchen Verbindungen, die eine salzbildende Gruppierung aufweisen, Verfahren zur Herstellung dieser Verbindungen, solche Verbindungen enthaltende pharmazeutische Mittel und ihre Verwendung zur Herstellung von pharmazeutischen Präparaten oder als pharmakologisch wirksame Verbindungen.

In der obigen Formel I steht n in erster Linie für 0, kann aber auch 1 bedeuten. In letzterem Fall kann die entsprechende Sulfoxidverbindung in der $\alpha$- oder $\beta$-Form vorliegen.

Veräthertes Hydroxy ist in erster Linie Niederalkoxy, während verestertes Hydroxy durch eine anorganische oder organische Säure verestertes Hydroxy, in erster Linie Halogen, ferner Niederalkanoyloxy oder Niederalkylsulfonyloxy darstellt.

Veräthertes Mercapto ist in erster Linie Heterocyclylthio, worin Heterocyclyl vorzugsweise einen fünf- oder sechsgliedrigen, gegebenenfalls teilweise gesättigten aromatischen Heterocyclylrest darstellt, der mindestens ein Ringstickstoffatom und gegebenenfalls weitere Ringstickstoff-, Ringsauerstoff- und/oder Ringschwefelatome enthält und über ein Ringkohlenstoffatom mit dem Thioschwefel verbunden ist, und der gegebenenfalls substituiert sein kann, kann aber auch für gegebenenfalls, z.B. durch Hydroxy, Amino oder Carboxy, substituiertes Niederalkylmercapto stehen.

Acyl als Rest $R_2$ kann für Formyl stehen, ist aber vorzugsweise gegebenenfalls substituiertes Hydrocarbylcarbonyl, worin Hydrocarbyl einen aliphatischen, cycloaliphatischen, aromatischen oder araliphatischen Kohlenwasserstoffrest bedeutet und insbesondere für Niederalkyl, Cycloalkyl, Phenyl bzw. Phenylniederalkyl steht, oder Heterocyclylcarbonyl, wobei Heterocyclyl vorzugsweise monocyclisches, fünf- oder sechsgliedriges aromatisches Heterocyclyl mit einem Stickstoff-, Sauerstoff- oder Schwefelatom als Ringglied ist.

Funktionell abgewandeltes Carboxy, insbesondere als Rest $R_2$, aber auch als Rest $R_3$, ist verestertes oder amidiertes Carboxy, wobei verestertes Carboxy z.B. Niederalkoxycarbonyl oder, insbesondere als

physiologisch spaltbares verestertes Carboxy $R_3$, Acyloxymethoxycarbonyl, worin Acyl z.B. den Rest einer organischen Carbonsäure, in erster Linie einer gegebenenfalls, z.B. durch Amino, substituierten Niederalkancarbonsäure bedeutet, oder worin Acyloxymethyl Teil eines Lactons bildet, wie Niederalkanoyloxymethoxycarbonyl oder Aminoniederalkanoyloxymethoxycarbonyl, oder Phthalidyloxycarbonyl, ferner Aryloxycarbonyl, insbesondere benz-Indanyloxycarbonyl, und amidiertes Carboxy z.B. gegebenenfalls N-niederalkyliertes Carbamoyl sein kann. In geschützter Form vorliegendes funktionell abgewandeltes Carboxy, insbesondere als $R_3$, ist in leicht spaltbarer Form vorliegendes und in freies Carboxy überführbares funktionell abgewandeltes Carboxy.

Ammonio ist in erster Linie gegebenenfalls substituiertes Pyridinio, wobei Substituenten z.B. Niederalkyl oder Carbamoyl sein können.

Gegebenenfalls substituiertes Amino ist in erster Linie geschütztes, d.h. leicht spaltbares und in freies Amino überführbares substituiertes Amino, insbesondere entsprechendes Acylamino, kann aber auch gegebenenfalls geschütztes sekundäres oder tertiäres, z.B. durch Niederalkyl mono- oder disubstituiertes Amino, insbesondere Methylamino, sein.

Ein zweiwertiger Thiadiazolrest T ist in erster Linie ein 1,2,4-Thiadiazolrest, der in 3- und 5-Stellung mit der Gruppe Am bzw. der Gruppe der Formel $-C(=N-O-R_4)-$ verbunden ist, wobei die Gruppe Am vorzugsweise mit der 5-Stellung eines 1,2,4-Thiadiazolrestes T verknüpft ist. Weitere Thiadiazolreste T sind 1,2,3-Thiadiazolreste, in welchem die 4- und 5-Stellungen mit der Gruppe Am bzw. der Gruppe der Formel $-C(=N)-O-R_4)-$ verbunden sind, sowie die symmetrischen 1,3,4- und 1,2,5-Thiadiazolreste, die jeweils durch die beiden Ringkohlenstoffatome mit der Gruppe Am bzw. der Gruppe der Formel $-C(=N-O-R_4)-$ verbunden sind. Dabei kann die durch eine Aminogruppe substituierte, zweiwertige Thiadiazolgruppe auch in tautomerer Form als ein durch eine Iminogruppe substituierte, zweiwertige Dihydro-thiadiazolgruppe oder als Gemisch der beiden

Formen vorliegen. Das Gleichgewicht zwischen den beiden Tautomeren hängt von der Art der gegebenenfalls substituierten Aminogruppe Am, sowie von äusseren Faktoren, wie Temperatur, Lösungsmittel oder pH-Wert ab. Im Rahmen der vorliegenden Erfindung wird die Thiadiazolgruppe in der Beschreibung und in den Ansprüchen nur als Thiadiazolgruppe bezeichnet, obgleich die Dihydro-thiadiazolform ebenfalls umfasst sein soll.

Die Gruppe der Formel $=N-O-R_4$ kann in der syn(oder Z)-Form oder in der anti(oder E)-Form vorliegen, wobei die syn(oder Z)-Form bevorzugt ist.

Eine Niederalkylgruppe $R_4$ enthält vorzugsweise 1-4 C-Atome und ist beispielsweise Aethyl, Propyl, Butyl oder insbesondere Methyl.

Eine Cycloalkylgruppe $R_4$ enthält vorzugsweise 3-8, in erster Linie 3-6 Ringglieder, und ist z.B. Cyclobutyl, Cyclopentyl oder Cyclohexyl, und insbesondere Cyclopropyl.

Substituenten von substituiertem Niederalkyl oder Cycloalkyl $R_4$ sind u.a. gegebenenfalls veräthertes Hydroxy, z.B. Niederalkoxy, primäres, sekundäres oder tertiäres Amino Am, z.B. Amino oder Diniederalkylamino, gegebenenfalls funktionell abgewandeltes, inkl. verestertes, amidiertes oder geschütztes Carboxyl oder Sulfo, sowie gegebenenfalls durch Niederalkyl N-substituiertes Ureidocarbonyl. Bevorzugt sind die substituierten Niederalkyl- und Cycloalkylgruppen durch eine Carboxyl- oder Sulfogruppe substituiert, wobei diese bevorzugt am Kohlenstoffatom, das mit dem Sauerstoffatom der Oxyiminogruppe verbunden ist, stehen.

Solche substituierte Niederalkyl- und Cycloalkylgruppen $R_4$ sind beispielsweise 2-Aminoäthyl, 2-Dimethylaminoäthyl, Carboxymethyl, 1- oder 2-Carboxyäthyl, 1-, 2- oder 3-Carboxyprop-1-yl, 1-, 2- oder

3-Carboxyprop-2-yl, 1-Carboxybut-1-yl, 1-Carboxycycloprop-1-yl und 1-Carboxycyclobut-1-yl, sowie entsprechende durch Sulfo substituierte Niederalkyl- und Cycloalkylgruppen.

Die Carboxy- und Sulfogruppen im Rest $R_4$ können beispielsweise durch Niederalkyl, wie Methyl oder Aethyl, oder eine der physiologisch abspaltbaren Gruppen, z.B. Pivaloyloxymethyl, verestert oder durch ein Amin, wie $NH_3$ oder ein primäres oder sekundäres Amin, wie ein Mono- oder Diniederalkylamin, wie Methyl- oder Aethylamin, oder Dimethyl- oder Diäthylamin amidiert sein, oder wie etwa für $R_3$ angegeben, geschützt sein.

Gegebenenfalls N-substituiertes Carbamoyl als Rest $R_4$ ist eine Gruppe -C(=O)-NHR, worin R Wasserstoff, Niederalkyl, z.B. Methyl, Aethyl oder 1- oder 2-Propyl, gegebenenfalls geschütztes Carboxyniederalkyl, z.B. Carboxymethyl, 1- oder 2-Carboxyäthyl oder 1-, 2- oder 3-Carboxypropyl, worin Carboxy durch eine der üblichen Carboxylschutzgruppen geschützt, beispielsweise durch Niederalkyl, z.B. Methyl, Aethyl, n- oder iso-Propyl, oder n- oder tert.-Butyl verestert sein kann, gegebenenfalls geschütztes Sulfoniederalkyl, z.B. Sulfomethyl, 1- oder 2-Sulfoäthyl oder 1-, 2- oder 3-Sulfopropyl, worin Sulfo durch eine der üblichen Sulfoschutzgruppen geschützt, beispielsweise durch Niederalkyl, z.B. Methyl oder Aethyl, verestert sein kann, gegebenenfalls geschütztes Hydroxyniederalkyl, z.B. Hydroxymethyl, 2-Hydroxyäthyl oder 2- oder 3-Hydroxypropyl, worin Hydroxy durch eine der üblichen Hydroxyschutzgruppen geschützt ist, z.B. acyliert, wie acetyliert, sein kann, gegebenenfalls geschütztes Aminoniederalkyl, z.B. 2-Aminoäthyl, 2- oder 3-Aminopropyl oder 2-, 3- oder 4-Aminobutyl, worin Amino durch eine der üblichen Aminoschutzgruppen geschützt, z.B. acyliert, wie acetyliert, sein kann, Arylniederalkyl, beispielsweise Phenylniederalkyl, z.B. Benzyl oder 1- oder 2-Phenyläthyl, Halogenniederalkyl, beispielsweise Fluor-, Chlor- oder Bromniederalkyl, z.B. 2-Chloräthyl, 3-Chlorpropyl- oder 4-Chlorbutyl, oder worin R Aryl, z.B. Phenyl oder durch Niederalkyl, z.B. Methyl, Niederalkoxy, z.B. Methoxy, Halogen, z.B. Chlor oder Nitro, ein- bis dreifach substituiertes Phenyl, bedeutet.

In der vorliegenden Beschreibung bedeutet der im Zusammenhang mit Definitionen von Gruppen bzw. Verbindungen verwendete Ausdruck "nieder", dass die entsprechenden Gruppen bzw. Verbindungen, sofern nicht ausdrücklich anders definiert, bis und mit 7, bevorzugt bis und mit 4 Kohlenstoffatome enthalten.

Die vor- und nachstehend verwendeten Allgemeindefinitionen haben im Rahmen der vorliegenden Beschreibung vorzugsweise die folgenden Bedeutungen:

Niederalkoxy ist z.B. Methoxy, ferner Aethoxy, n-Propyloxy, Isopropyloxy, n-Butyloxy, Isobutyloxy oder tert.-Butyloxy, sowie n-Pentyloxy, n-Hexyloxy oder n-Heptyloxy.

Halogen ist insbesondere Chlor, kann jedoch auch für Brom, ferner für Fluor oder Jod stehen.

Niederalkanoyloxy ist z.B. Acetyloxy oder Propionyloxy, während Niederalkylsulfonyloxy z.B. Methylsulfonyloxy oder Aethylsulfonyloxy ist.

Ein Heterocyclylrest in einer Heterocyclylthiogruppe, der einen fünf- oder sechsgliedrigen, gegebenenfalls teilweise gesättigten aromatischen Heterocyclylrest darstellt, der mindestens ein Ringstickstoffatom und gegebenenfalls weitere Stickstoff-, Sauerstoff- oder Schwefelatome als Ringglieder enthält, ist vorzugsweise ein entsprechender monocyclischer Rest, der gegebenenfalls substituiert sein kann. Solche Reste enthalten, zusätzlich zum Ringstickstoffatom vorzugsweise 1-3 weitere Stickstoffatome und/oder ein Sauerstoff- oder Schwefelatom als Ringglieder. Substituenten sind u.a. gegebenenfalls, wie durch gegebenenfalls veräthertes oder verestertes, inkl. geschütztes Hydroxy, z.B. Niederalkoxy, eine saure Gruppe, z.B. gegebenenfalls funktionell abgewandeltes, inkl. geschütztes Carboxy oder Sulfo, z.B. Carbamoyl, oder eine basische Gruppe, wie eine Aminogruppe, z.B. Amino oder Diniederalkylamino, ferner geschütztes Amino, substituiertes Niederalkyl, gegebenenfalls, wie durch Niederalkyl, Nitro, Niederalkoxy und/oder Halogen substituiertes Phenyl, oder Thienyl, z.B. 2-Thienyl, gegebenenfalls veräthertes oder verestertes, inkl. gegebenenfalls geschütztes Hydroxy, z.B. Niederalkoxy oder Halogen, gegebenenfalls funktionell abgewandeltes, wie verestertes Carboxy, z.B. Carboxy oder Niederalkoxycarbonyl, oder Oxo und/oder N-Oxido.

Diniederalkylamino ist z.B. Dimethylamino, Diäthylamino oder Di-n-propylamino.

Niederalkyl ist z.B. Methyl, Aethyl, n-Propyl, Isopropyl, n-Butyl, Isobutyl, sek.-Butyl oder tert.-Butyl, ferner n-Pentyl, Neopentyl, n-Hexyl oder n-Heptyl.

Niederalkoxycarbonyl ist z.B. Methoxycarbonyl oder Aethoxycarbonyl.

Heterocyclylthiogruppen mit entsprechenden fünfgliedrigen heterocyclischen Resten sind demgemäss u.a. gegebenenfalls substituiertes Diazolylthio, wie Imidazolylthio, z.B. 2-Imidazolylthio, gegebenenfalls, z.B. durch Niederalkyl, Carboxyniederalkyl und/oder

Phenyl, substituiertes Triazolylthio, z.B. 1H-1,2,3-Triazol-4-ylthio, 1-Methyl-1H-1,2,3-triazol-4-ylthio, 1H-1,2,4-Triazol-3-ylthio, 5-Methyl-1H-1,2,4-triazol-3-ylthio, 3-Methyl-1-phenyl-1H-1,2,4-triazol-5-ylthio, 4,5-Dimethyl-4H-1,2,4-triazol-3-ylthio oder 4-Carboxymethyl- oder 4-Phenyl-4H-1,2,4-triazol-3-ylthio, gegebenenfalls, z.B. durch Niederalkyl, Carboxyniederalkyl, Sulfoniederalkyl, Aminoniederalkyl, Diniederalkylamino-niederalkyl oder gegebenenfalls Substituenten, wie Halogen, enthaltendes Phenyl, substituiertes Tetrazolylthio, z.B. 1H-Tetrazol-5-ylthio, 1-Methyl-1H-tetrazol-5-ylthio, 1-Carboxymethyl-1H-tetrazol-5-ylthio, 1-(2-Carboxyäthyl)-1H-tetrazol-5-ylthio, 1-Sulfomethyl-1H-tetrazol-5-ylthio, 1-(2-Sulfoäthyl)-1H-tetrazol-5-ylthio, 1-(2-Dimethylaminoäthyl)-1H-tetrazol-5-ylthio, 1-Phenyl-1H-tetrazol-5-ylthio oder 1-(4-Chlorphenyl)-1H-tetrazol-5-ylthio, gegebenenfalls, z.B. durch Niederalkyl oder Thienyl, substituiertes Thiazolylthio oder Isothiazolylthio, z.B. 2-Thiazolylthio, 4-(2-Thienyl)-2-thiazolylthio, 4,5-Dimethyl-2-thiazolylthio, 3-Isothiazolylthio, 4-Isothiazolylthio oder 5-Isothiazolylthio, gegebenenfalls z.B. durch Niederalkyl, substituiertes Thiadiazolylthio, z.B. 1,2,3-Thiadiazol-4-ylthio, 1,2,3-Thiadiazol-5-ylthio, 1,3,4-Thiadiazol-2-ylthio, 2-Methyl-1,3,4-thiadiazol-5-ylthio, 1,2,4-Thiadiazol-5-ylthio, 3-Methyl-1,2,4-thiadiazol-5-ylthio oder 1,2,5-Thiadiazol-3-ylthio, Thiatriazolylthio, z.B. 1,2,3,4-Thiatriazolyl-5-ylthio, gegebenenfalls, z.B. durch Niederalkyl und/oder Phenyl, substituiertes Oxazolylthio oder Isoxazolylthio, z.B. 5-Oxazolylthio, 4-Methyl-5-oxazolylthio, 2-Oxazolylthio, 4,5-Diphenyl-2-oxazolylthio oder 3-Methyl-5-isoxazolylthio, oder gegebenenfalls, z.B. durch Niederalkyl, oder gegebenenfalls Substituenten, wie Nitro, enthaltendes Phenyl oder Thienyl, substituiertes Oxadiazolylthio, z.B. 1,2,4-Oxadiazol-5-ylthio, 2-Methyl-1,3,4-oxadiazol-5-ylthio, 2-Phenyl-1,3,4-oxadiazol-5-ylthio, 5-(4-Nitrophenyl)-1,3,4-oxadiazol-2-ylthio, oder 2-(2-Thienyl)-1,3,4-oxadiazol-5-ylthio.

Heterocyclylthiogruppen mit entsprechenden sechsgliedrigen heterocyclischen Resten sind demgemäss u.a. gegebenenfalls, z.B. durch Halogen, substituiertes 1-Oxidopyridylthio, z.B. 1-Oxido-2-pyridylthio oder 4-Chlor-1-oxido-2-pyridylthio, gegebenenfalls, z.B. durch Hydroxy, substituiertes Pyridazinylthio, z.B. 3-Hydroxy-6-pyridazinylthio, gegebenenfalls, z.B. durch Niederalkyl, Niederalkoxy oder Halogen, substituiertes N-Oxido-pyridazinylthio, z.B. 2-Oxido-6-pyridazinylthio, 3-Chlor-1-oxido-6-pyridazinylthio, 3-Methyl-2-oxido-6-pyridazinylthio, 3-Methoxy-1-oxido-6-pyridazinylthio, 3-Aethoxy-1-oxido-6-pyridazinylthio, 3-n-Butyloxy-1-oxido-6-pyridazinylthio oder 3-(2-Aethylhexyloxy)-1-oxido-6-pyridazinylthio, gegebenenfalls, z.B. durch Niederalkyl, Amino, Diniederalkylamino, Oxo oder Carboxy, substituiertes 2-Oxo-1,2-dihydro-pyrimidinylthio, z.B. 2-Oxo-1,2-dihydro-4-pyrimidinylthio, 6-Methyl-2-oxo-1,2-dihydro-4-pyrimidinylthio, 5-Methyl-2-oxo-1,2-dihydro-4-pyrimidinylthio, 6-Amino-2-oxo-1,2-dihydro-4-pyrimidinylthio, 6-Dimethylamino-2-oxo-1,2-dihydro-4-pyrimidinylthio-, 5-Carboxy-2-oxo-1,2-dihydro-4-pyrimidinylthio oder 6-Carboxy-2-oxo-1,2-dihydro-4-pyrimidinylthio, oder gegebenenfalls, z.B. durch Niederalkyl, und mit bis zu zwei Oxo substituiertes Triazinylthio, insbesondere N-Niederalkyl-5,6-dioxo-1,2,4-triazin-3-ylthio, z.B. 1-, 2- oder 4-Methyl-5,6-dioxo-1,2,4-triazin-3-ylthio.

Niederalkylthio ist z.B. Methylthio, Aethylthio, n-Propylthio, Isopropylthio oder n-Butylthio.

Cycloalkyl enthält vorzugsweise 3-8, in erster Linie 5 oder 6, als Gruppe $R_4$ vorzugsweise 3 Ringglieder, und ist z.B. Cyclopentyl oder Cyclohexyl, ferner Cyclopropyl, sowie Cycloheptyl.

Phenylniederalkyl ist z.B. Benzyl, 1- oder 2-Phenyläthyl oder 3-Phenylpropyl.

In einer Heterocyclylcarbonylgruppe $R_2$ ist der Heterocyclylrest z.B. vorzugsweise ein monocyclischer, fünf- oder sechsgliedriger aromatischer Heterocyclylrest, der mindestens ein Stickstoff-, Sauerstoff- oder Schwefelatom als Ringglied enthält, und ist in erster Linie gegebenenfalls substituiertes, z.B. Niederalkyl, Niederalkoxy und/oder Halogen enthaltendes Pyridyl, Thienyl oder Furyl, kann aber auch einer der oben genannten, in einer Heterocyclylthiogruppe vorkommenden heterocyclischen Reste sein.

Thienyl ist in erster Linie 2-Thienyl, und Furyl vorzugsweise 2-Furyl.

Gegebenenfalls substituiertes Niederalkanoyloxymethoxycarbonyl kann z.B. Amino als Substituenten enthalten, und ist z.B. Acetyloxymethoxycarbonyl oder Pivaloyloxymethoxycarbonyl, ferner (1-Aminoniederalkanoyl)-oxymethoxycarbonyl, wie Glycyloxymethoxycarbonyl, L-Valyloxymethoxycarbonyl oder L-Leucyloxymethoxycarbonyl.

Phthalidyloxycarbonyl ist z.B. 2-Phthalidyloxycarbonyl, während benz-Indanyloxycarbonyl z.B. 5-Indanyloxycarbonyl sein kann.

Wie oben erwähnt, können in Verbindungen der Formel I vorhandene funktionelle Gruppen, insbesondere Carboxyl- und Amino-, ferner Hydroxy- und Sulfogruppen, gegebenenfalls durch Schutzgruppen geschützt sein, wobei üblicherweise die in der Penicillin-, Cephalosporin- und Peptidchemie verwendeten Schutzgruppen zur Anwendung kommen.

Solche Schutzgruppen sind leicht, das heisst ohne dass unerwünschte Nebenreaktionen stattfinden, beispielsweise solvolytisch,

reduktiv, photolytisch oder auch unter physiologischen Bedingungen, abspaltbar.

Schutzgruppen dieser Art, sowie ihre Abspaltung sind beispielsweise beschrieben in "Protective Groups in Organic Chemistry", Plenum Press, London, New York, 1973, ferner in "The Peptides", Vol. I, Schröder and Lubke, Academic Press, London, New York, 1965, sowie in "Methoden der organischen Chemie", Houben-Weyl, 4.Auflage, Bd. 15/1, Georg Thieme Verlag, Stuttgart, 1974.

So sind Carboxylgruppen, wie die Carboxylgruppe $R_3$, z.B. in üblicherweise in veresterter Form geschützt, wobei solche Estergruppierungen unter schonenden Bedingungen leicht spaltbar sind. In dieser Art geschützte Carboxylgruppen enthalten als veresternde Gruppen in erster Linie in 1-Stellung verzweigte oder in 1- oder 2-Stellung geeignet substituierte Niederalkylgruppen. Bevorzugte, in veresterter Form vorliegende Carboxylgruppen sind u.a. tert.-Niederalkoxycarbonyl, z.B. tert.-Butyloxycarbonyl, Arylmethoxycarbonyl, mit einem oder zwei Arylresten, wobei diese gegebenenfalls, z.B. durch Niederalkyl, wie tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellen, wie gegebenenfalls, z.B. wie oben erwähnt, substituiertes Benzyloxycarbonyl, z.B. 4-Nitro-benzyloxycarbonyl oder 4-Methoxy-benzyloxycarbonyl, oder gegebenenfalls, z.B. wie oben erwähnt, substituiertes Diphenylmethoxycarbonyl, z.B. Diphenylmethoxycarbonyl oder Di-(4-methoxyphenyl)-methoxycarbonyl, 1-Niederalkoxyniederalkoxycarbonyl, wie Methoxymethoxycarbonyl, 1-Methoxyäthoxycarbonyl oder 1-Aethoxymethoxycarbonyl, 1-Niederalkylthioniederalkoxycarbonyl, wie 1-Methylthiomethoxycarbonyl oder 1-Aethylthioäthoxycarbonyl, Aroylmethoxycarbonyl, worin die Aroylgruppe gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichloräthoxycarbonyl, 2-Bromäthoxycarbonyl oder 2-Jodäthoxycarbonyl, oder 2-(trisubstituiertes Silyl)-äthoxycarbonyl,

worin die Substituenten unabhängig voneinander je einen gegebenenfalls substituierten, z.B. durch Niederalkyl, Niederalkoxy, Aryl, Halogen und/oder Nitro substituierten, aliphatischen, araliphatischen, cycloaliphatischen oder aromatischen Kohlenwasserstoffrest mit z.B. bis zu 15 Kohlenstoffatomen, wie entsprechendes, gegebenenfalls substituiertes Niederalkyl, Phenylniederalkyl, Cycloalkyl oder Phenyl bedeuten, z.B. 2-Triniederalkylsilyläthoxycarbonyl, wie 2-Trimethylsilyläthoxycarbonyl oder 2-(Di-n-butyl-methyl-silyl)-äthoxycarbonyl, oder 2-Triarylsilyläthoxycarbonyl, wie 2-Triphenylsilyläthoxycarbonyl.

Weitere, in veresterter Form vorliegende geschützte Carboxylgruppen sind entsprechende Silyloxycarbonyl-, insbesondere organische Silyloxycarbonylgruppen, ferner entsprechende Stannyloxycarbonylgruppen. In diesen enthält das Silicium- bzw. Zinnatom vorzugsweise Niederalkyl, insbesondere Methyl, ferner Niederalkoxy, z.B. Methoxy, und/oder Halogen, z.B. Chlor, als Substituenten. Geeignete Silyl- bzw. Stannylschutzgruppen sind in erster Linie Triniederalkylsilyl, insbesondere Trimethylsilyl, ferner Dimethyl-tert.butylsilyl, Niederalkoxyniederalkyl-halogen-silyl, z.B. Methoxy-methyl-chlor-silyl, oder Diniederalkyl-halogen-silyl, z.B. Dimethyl-chlor-silyl, oder entsprechend substituierte Stannylverbindungen, z.B. Tri-n-butylstannyl.

Bevorzugte geschützte Carboxylgruppen sind tert.-Niederalkoxycarbonyl, wie tert.-Butyloxycarbonyl, und in erster Linie gegebenenfalls, z.B. wie oben erwähnt, substituiertes Benzyloxycarbonyl, wie 4-Nitro-benzyloxycarbonyl, oder Diphenylmethoxycarbonyl.

Eine geschützte Aminogruppe, wie eine entsprechende Gruppe Am, kann z.B. in Form einer leicht spaltbaren Acylamino-, Arylmethylamino-, verätherten Mercaptoamino-, 2-Acyl-niederalk-1-en-1-yl-amino-, Silyl- oder Stannylaminogruppe oder als Azidogruppe vorliegen.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls, z.B. durch Halogen oder Aryl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl, oder Propionyl, Halogen-niederalkanoyl, wie 2-Halogenacetyl, insbesondere 2-Chlor-, 2-Brom-, 2-Jod-, 2,2,2-Trifluor- oder 2,2,2-Trichloracetyl, gegebenenfalls, z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung der Niederalkylrestes verzweigtes oder in 1- oder 2-Stellung geeignet substituiertes Niederalkoxycarbonyl, insbesondere tert.-Niederalkoxycarbonyl, z.B. tert.-Butyloxycarbonyl, Arylmethoxycarbonyl mit einem oder zwei Arylresten, die vorzugsweise gegebenenfalls, z.B. durch Niederalkyl, insbesondere tert.-Niederalkyl, wie tert.-Butyl, Niederalkoxy, wie Methoxy, Hydroxy, Halogen, z.B. Chlor, und/oder Nitro, mono- oder polysubstituiertes Phenyl darstellen, wie gegebenenfalls substituiertes Benzyloxycarbonyl, z.B. 4-Nitro-benzyloxycarbonyl, oder substituiertes Diphenylmethoxycarbonyl, z.B. Benzhydryloxycarbonyl oder Di-(4-methoxyphenyl)-methoxycarbonyl, Aroylmethoxycarbonyl, worin die Aroylgruppe vorzugsweise gegebenenfalls,z.B. durch Halogen, wie Brom, substituiertes Benzoyl darstellt, z.B. Phenacyloxycarbonyl, 2-Halogen-niederalkoxycarbonyl, z.B. 2,2,2-Trichloräthoxycarbonyl, 2-Bromäthoxycarbonyl oder 2-Jodäthoxycarbonyl, oder 2-(trisubstituiertes Silyl)-äthoxycarbonyl, worin die Substituenten unabhängig voneinander je einen gegebenenfalls substituierten, z.B. durch Niederalkyl, Niederalkoxy, Aryl, Halogen oder Nitro, substituierten, aliphatischen, araliphatischen, cycloaliphatischen oder aromatischen Kohlenwasserstoffrest mit z.B. bis zu 15 C-Atomen, wie entsprechendes, gegebenenfalls substituiertes Niederalkyl, Phenylniederalkyl, Cycloalkyl oder Phenyl, bedeuten, z.B. 2-Triniederalkylsilyläthoxycarbonyl, wie 2-Trimethylsilyläthoxycarbonyl oder 2-(Di-n-butyl-methyl-silyl)-äthoxycarbonyl, oder 2-Triarylsilyläthoxycarbonyl, wie 2-Triphenylsilyläthoxycarbonyl.

Weitere, als Aminoschutzgruppen in Frage kommende Acylreste sind auch entsprechende Reste organischer Phosphor-, Phosphon- oder Phosphinsäuren, wie Diniederalkylphosphoryl, z.B. Dimethylphosphoryl, Diäthylphosphoryl, Di-n-propylphosphoryl oder Diisopropylphosphoryl, Dicycloalkylphosphoryl, z.B. Dicyclohexylphosphoryl, gegebenenfalls substituiertes Diphenylphosphoryl, z.B. Diphenylphosphoryl, gegebenenfalls, z.B. durch Nitro substituiertes Di-phenylniederalkylphosphoryl, z.B. Dibenzylphosphoryl oder Di-4-nitrobenzylphosphoryl, gegebenenfalls substituiertes Phenyloxy-phenyl-phosphonyl, z.B. Phenyloxy-phenyl-phosphonyl, Diniederalkylphosphinyl, z.B. Diäthylphosphinyl, oder gegebenenfalls substituiertes Diphenylphosphinyl, z.B. Diphenylphosphinyl.

In einer Arylmethylaminogruppe, die eine Mono-, Di- oder insbesondere Triarylmethylamino darstellt, sind die Arylreste insbesondere gegebenenfalls substituierte Phenylreste. Solche Gruppen sind beispielsweise Benzyl-, Diphenylmethyl- und insbesondere Tritylamino.

Eine verätherte Mercaptogruppe in einer mit einem solchen Rest geschützten Aminogruppe ist in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor, und/oder Nitro substituiertes Phenyl ist. Eine entsprechende Aminoschutzgruppe ist z.B. 4-Nitrophenylthio.

In einem als Aminoschutzgruppe verwendbaren 2-Acyl-niederalk-1-en-1-yl-rest ist Acyl z.B. der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters. Entsprechende Schutzgruppen sind in erster Linie 1-Niederalkanoyl-prop-1-en-

2-yl, z.B. 1-Acetyl-prop-1-en-2-yl, oder 1-Niederalkoxycarbonyl-prop-1-en-2-yl, z.B. 1-Aethoxycarbonyl-prop-1-en-2-yl.

Eine Silyl- oder Stannylaminogruppe ist in erster Linie eine organische Silyl- bzw. Stannylaminogruppe, worin das Silicium- bzw. Zinnatom vorzugsweise Niederalkyl, insbesondere Methyl, ferner Niederalkoxy, z.B. Methoxy, und/oder Halogen, z.B. Chlor, als Substituenten enthält. Entsprechende Silyl- oder Stannylgruppen sind in erster Linie Triniederalkylsilyl, insbesondere Trimethylsilyl, ferner Dimethyl-tert.-butyl-silyl, Niederalkoxy-niederalkyl-halogen-silyl, z.B. Methoxy-methyl-chlor-silyl, oder Diniederalkyl-halogensilyl, z.B. Dimethyl-chlor-silyl, oder entsprechend substituiertes Stannyl, z.B. Tri-n-butylstannyl.

Eine Aminogruppe kann auch in protonierter Form geschützt werden; als entsprechende Anionen kommen in erster Linie diejenigen von starken anorganischen Säuren, wie von Halogenwasserstoffsäuren, z.B. das Chlor- oder Bromanion, oder von organischen Sulfonsäuren, wie p-Toluolsulfonsäure, in Frage.

Bevorzugte Aminoschutzgruppen sind Acylreste von Kohlensäurehalbestern, insbesondere tert.-Butyloxycarbonyl, gegebenenfalls, z.B. wie angegeben, substituiertes Benzyloxycarbonyl, z.B. 4-Nitro-benzyloxycarbonyl, oder Diphenylmethoxycarbonyl, oder 2-Halogen-niederalkoxycarbonyl, wie 2,2,2-Trichloräthoxycarbonyl, ferner Trityl oder Formyl.

Hydroxyschutzgruppen sind z.B. Acylreste, wie gegebenenfalls, z.B. durch Halogen, substituiertes Niederalkanoyl, wie 2,2-Dichloracetyl, oder insbesondere die im Zusammenhang mit einer geschützten Aminogruppe genannten Acylreste von Kohlensäurehalbestern, insbesondere 2,2,2-Trichloräthoxycarbonyl, oder organischen Silyl- oder Stannylreste, ferner leicht abspaltbare veräthernde Gruppen, wie tert.-Niederalkyl, z.B. tert.-Butyl, 2-oxa- oder 2-thia-aliphatische oder -cycloaliphatische Kohlenwasserstoffreste, in erster Linie 1-Niederalkoxy-niederalkyl oder 1-Niederalkylthio-niederalkyl, z.B. Methoxymethyl, 1-Methoxyäthyl, 1-Aethoxy-äthyl, 1-Methylthiomethyl,

1-Methylthio-äthyl oder 1-Aethylthioäthyl, oder 2-Oxa- oder 2-Thiacycloalkyl mit 5-7 Ringatomen, z.B. 2-Tetrahydrofuryl oder 2-Tetrahydropyranyl oder entsprechende Thiaanaloge, sowie gegebenenfalls substituiertes 1-Phenylniederalkyl, wie gegebenenfalls substituiertes Benzyl oder Diphenylmethyl, wobei als Substituenten der Phenylreste z.B. Halogen, wie Chlor, Niederalkoxy, wie Methoxy und/oder Nitro in Frage kommen.

Eine geschützte Sulfogruppe ist in erster Linie eine veresterte, wie mit einem aliphatischen, cycloaliphatischen, cycloaliphatisch-aliphatischen, aromatischen oder araliphatischen Alkohol, z.B. einem Niederalkanol, oder mit einem Silyl- oder Stannylrest, wie Triniederalkylsilyl, veresterte Sulfogruppe. In einer Sulfogruppe kann die Hydroxygruppe beispielsweise, wie die Hydroxygruppe in einer veresterten Carboxygruppe veräthert sein.

Salze von erfindungsgemässen Verbindungen sind in erster Linie pharmazeutisch verwendbare, nicht-toxische Salze, wie diejenigen von Verbindungen der Formel I mit sauren Gruppen, z.B. mit einer freien Carboxyl- und Sulfogruppe. Solche Salze sind in erster Linie Metall- oder Ammoniumsalze, wie Alkalimetall- und Erdalkalimetall-, z.B. Natrium-, Kalium-, Magnesium- oder Calciumsalze, sowie Amoniumsalze mit Ammoniak oder geeigneten organischen Aminen, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung in Frage kommen, wie Niederalkylamine, z.B. Triäthylamin, Hydroxyniederalkylamine, z.B. 2-Hydroxyäthylamin, Bis-(2-hydroxyäthyl)-amin oder Tris-(2-hydroxyäthyl)-amin, basische aliphatische Ester von Carbonsäuren, z.B. 4-Aminobenzoesäure-2-diäthylaminoäthylester, Niederalkylenamine, z.B. 1-Aethyl-piperidin, Cycloalkylamine, z.B. Dicyclohexylamin, oder Benzylamine, z.B. N,N'-Dibenzyl-äthylendiamin, ferner Basen vom Pyridintyp, z.B. Pyridin, Collidin oder Chinolin. Verbindungen der Formel I mit einer basischen Gruppen

können Säureadditionssalze, z.B. mit anorganischen Säuren, wie Salzsäure, Schwefelsäure oder Phosphorsäure, oder mit geeigneten organischen Carbon- oder Sulfonsäuren, z.B. Trifluoressigsäure, sowie mit Aminosäuren, wie Arginin und Lysin, bilden. Bei Anwesenheit von mehreren sauren oder basischen Gruppen können Mono- oder Polysalze gebildet werden. Verbindungen der Formel I mit einer sauren, z.B. freien Carboxylgruppe, und einer freien basischen, z.B. einer Aminogruppe, können auch in Form von inneren Salzen, d.h. in zwitterionischer Form vorliegen, oder es kann ein Teil des Moleküls als inneres Salz, und ein anderer Teil als normales Salz vorliegen.

Zur Isolierung oder Reinigung können auch pharmazeutisch ungeeignete Salze Verwendung finden. Zur therapeutischen Anwendung gelangen nur die pharmazeutisch verwendbaren, nicht toxischen Salze, die deshalb bevorzugt werden.

Die Verbindungen der Formel I, worin die funktionellen Gruppen in freier, d.h. nicht in geschützter Form vorliegen, wobei jedoch Carboxylgruppen gegebenenfalls in physiologisch spaltbarer, veresterter Form vorliegen, und ihre pharmazeutisch verwendbaren, nicht-toxischen Salze sind wertvolle, antibiotisch wirksame Substanzen, die insbesondere als antibakterielle Antibiotika verwendet werden können. Beispielsweise sind sie in vitro gegen gram-positive und gram-negative Mikroorganismen, wie gegen Kokken, z.B. Staphylokokken, wie Staphylococcus aureus, Streptokokken, wie Streptococcus pyogenes, Streptococcus faecalis und Streptococcus pneumoniae, und Neisseria, z.B. Neisseria gonorrhoeae oder Neisseria meningitidis, in Minimalkonzentrationen von bis zu 0.05 µg/ml, gegen Haemophilius influenza in Minimalkonzentrationen von bis zu 0.5 µg/ml, und insbesondere gegen Enterobacteriaceae, z.B. gegen Escherichia coli, Klebsiella sp., Serratia sp., wie Serratia marcescens, Enterobacter sp., Salmonella sp., oder Proteus sp., inkl. Indol-positive Proteus sp., in Minimalkonzentrationen von bis zu 0.01 µg/ml, sowie gegen Pseudomonas sp., wie Pseudomonas aeruginosa, in Minimalkonzentrationen von bis zu 0.01 µg/ml,

ferner gegen andere gram-negative und gram-positive Stäbchenbakterien wirksam. In vivo, bei subkutaner Anwendung an der Maus, sind sie ebenfalls gegen gram-positive und gram-negative Bakterien, insbesondere gegen Enterobacteriaeceae, wie den obgenannten, in Minimaldosen von unter 1 mg/kg, und gegen gram-positive Kokken, z.B. Staphylococcus aureus, in Minimaldosen von unter 30 mg/kg wirksam.

Die neuen Verbindungen können deshalb entsprechend, z.B. in Form von antibiotisch wirksamen Präparaten, zur Behandlung von durch gram-positive oder gram-negative Bakterien und Kokken, insbesondere durch von Enterobakterien, wie Serratia marcescens oder Escherichia coli, verursachten Infektionen Verwendung finden.

Verbindungen der Formel I, worin die funktionellen Gruppen geschützt sind, werden als Zwischenprodukte zur Herstellung von Verbindungen der Formel I verwendet, worin die funktionellen Gruppen in freier Form vorliegen.

Hervorzuhebende Gruppen sind Verbindungen der Formel I, worin Am eine gegebenenfalls geschützte sekundäre oder eine tertiäre Aminogruppe darstellt und n, $R_1$, $R_3$, $R_4$ und T die unter Formel I angegebenen Bedeutungen haben, oder worin $R_4$ für Wasserstoff, substituiertes Niederalkyl oder Cycloalkyl, oder gegebenenfalls N-substituiertes Carbamoyl steht und n, Am, $R_1$, $R_3$ und $R_4$ die unter Formel I gegebenen Bedeutungen haben, oder worin $R_1$ für veräthertes oder verestertes Hydroxy, veräthertes Mercapto oder den Rest der Formel $-CH_2-R_2$ steht, worin $R_2$ Acyl, gegebenenfalls funktionell abgewandeltes Carboxy, eine Ammoniogruppe oder Azido darstellt und n, Am, T, $R_3$ und $R_4$ die unter Formel I genannten Bedeutungen haben, und die Salze von solchen Verbindungen mit salzbildenden Gruppen.

Die Erfindung betrifft in erster Linie Verbindungen der Formel I, worin n für 0 oder 1 steht, $R_1$ für Wasserstoff, Niederalkoxy, Halogen, Niederalkylthio, Heterocyclylthio, worin Heterocyclyl für einen fünf- oder sechsgliedrigen monocyclischen, gegebenenfalls partiell gesättigten, aromatischen Heterocyclylrest steht, der mindestens 1 Ringstickstoffatom und gegebenenfalls 1-3 zusätzliche Ringstickstoffatome und/oder ein Ringsauerstoff- oder Ringschwefelatom enthält, und der durch gegebenenfalls Substituenten, wie Hydroxy, Amino, Diniederalkylamino, Carboxy, Carbamoyl oder Sulfo enthaltendes Niederalkyl, durch gegebenenfalls Substituenten, z.B. Niederalkyl, Niederalkoxy und/oder Halogen, enthaltendes Phenyl, oder durch Thienyl substituiert sein kann, oder für den Rest der Formel $-CH_2-R_2$ steht, worin $R_2$ Niederalkanoyl, Carboxy, Niederalkoxycarbonyl oder gegebenenfalls durch Carbamoyl substituiertes Pyridinio bedeutet, $R_3$ Carboxy, Niederalkanoyloxymethoxycarbonyl oder (2-Amino-niederalkanoyl)-oxymethoxycarbonyl, ferner geschütztes Carboxy darstellt, Am für Amino, Niederalkylamino oder Diniederalkylamino, ferner für geschütztes Amino steht, T einen zweiwertigen, über die beiden Ringkohlenstoffatome gebundenen Thiadiazolrest bedeutet, und $R_4$ Wasserstoff, gegebenenfalls durch Niederalkoxy, Amino, Diniederalkylamino oder Carboxy substituiertes Niederalkyl oder Cycloalkyl, oder gegebenenfalls N-niederalkyliertes Carbamoyl steht, wobei der Rest der Formel $=N-O-R_4$ die syn(oder Z)- oder anti(oder E)-Form aufweisen kann, sowie Salze, insbesondere pharmazeutisch verwendbare Salze von solchen Verbindungen der Formel I, die salzbildende Gruppen aufweisen.

Die Erfindung betrifft insbesondere Verbindungen der Formel I, worin n für 0 steht, $R_1$ für Wasserstoff, Niederalkoxy, z.B. Methoxy. Halogen, z.B. Chlor, Heterocyclylthio, worin Heterocyclyl gegebenenfalls durch Niederalkyl, z.B. Methyl, substituiertes Oxadiazolyl oder Thiadiazolyl, wie 1,3,4-Oxadiazol-2-yl oder 1,3,4-Thiadiazol-2-yl, gegebenenfalls durch Niederalkyl, z.B. Methyl, substituiertes Triazolyl, wie 1,2,3-Triazol-4-yl, oder gegebenenfalls durch Niederalkyl, z.B. Methyl oder Aethyl, das Diniederalkylamino, z.B. Dimethylamino, Carboxy, Carbamoyl oder Sulfo als Substituenten enthalten kann, substituiertes 1H-Tetrazol-5-yl bedeutet, oder einen Rest der Formel $-CH_2-R_2$ steht, worin $R_2$ gegebenenfalls durch Carbamoyl substituiertes Pyridinio bedeutet, $R_3$ Carboxy oder Niederalkanoyloxymethoxycarbonyl, z.B. Pivaloyloxymethoxycarbonyl, ferner geschütztes Carboxy

darstellt, Am für Amino, Methylamino, ferner geschütztes Amino steht, T den zweiwertigen, über die beiden Ringkohlenstoffatome gebundenen 1,2,4-Thiadiazolrest bedeutet, wobei das Ringkohlenstoffatom der 5-Stellung vorzugsweise mit der Gruppe Am verbunden ist, und $R_4$ Wasserstoff, Niederalkyl, z.B. Methyl, Carboxyniederalkyl, z.B. 2-Carboxy-2-propyl, Carbamoyl, Niederalkylcarbamoyl, z.B. N-Methylcarbamoyl, oder Aminoniederalkyl, z.B. 2-Aminoäthyl, darstellt, wobei der Rest der Formel $=N-O-R_4$ vorzugsweise die syn(oder Z)-Form aufweist, sowie Salze, insbesondere pharmazeutisch verwendbare Salze von solchen Verbindungen der Formel I, die salzbildende Gruppen aufweisen.

Besonders hervorzuheben sind die Verbindungen der Formel I, in welcher n für 0 steht, $R_1$ für Wasserstoff, Niederalkoxy, insbesondere Methoxy, Halogen, insbesondere Chlor oder den Rest der Formel $-CH_2R_2$ steht, worin $R_2$ gegebenenfalls Carbamoyl enthaltendes Pyridinio, insbesondere 4-Carbamoylpyridinio, bedeutet, $R_3$ Carboxy oder Niederalkanoyloxymethoxycarbonyl, insbesondere Pivaloyloxymethoxycarbonyl bedeutet, Am Amino oder Methylamino ist, T den zweiwertigen 1,2,4-Thiadiazolrest darstellt, der mit dem Ringkohlenstoffatom der 5-Stellung mit der Aminogruppe Am und mit dem Ringkohlenstoffatom der 3-Stellung mit der Gruppe der Formel $-C(=N-O-R_4)-$ verbunden ist, und $R_4$ für Wasserstoff, Niederalkyl, insbesondere Methyl, Carboxyniederalkyl, z.B.2-Carboxy-2-propyl, Carbamoyl oder Niederalkylcarbamoyl, z.B. N-Methylcarbamoyl, steht, wobei die Gruppe der Formel $=N-O-R_4$ die syn(oder Z)-Form aufweist, sowie Salze, in erster Linie pharmazeutisch verwendbare Salze von solchen Verbindungen der Formel I.

Die Erfindung betrifft insbesondere die in den Beispielen beschriebenen Verbindungen der Formel I, sowie Salze, besonders pharmazeutisch verwendbaren Salze von solchen Verbindungen mit salzbildenden Gruppen.

Die Verbindungen der vorliegenden Erfindung werden nach an sich bekannten Verfahren erhalten.

Verbindungen der Formel I und Salze von solchen Verbindungen, die eine salzbildende Gruppe aufweisen, werden beispielsweise hergestellt, indem man

a) in einer Verbindung der Formel

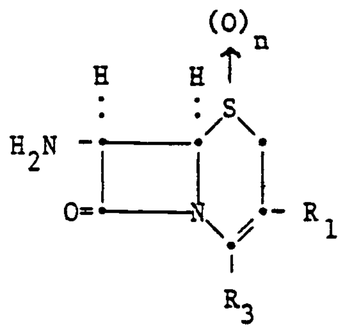

(II),

worin die 7β-Aminogruppe gegebenenfalls durch eine, die Acylierung erlaubende Gruppe geschützt ist, und worin weitere gegebenenfalls vorhandene funktionelle Gruppen geschützt sein können, die 7β-Aminogruppe durch Behandeln mit einem, den Acylrest einer Carbonsäure der Formel

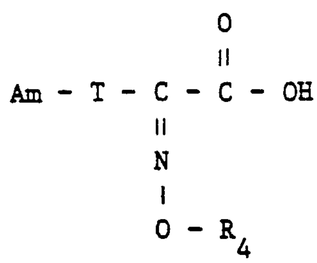

(III)

einführenden Acylierungsmittel, worin weitere, gegebenenfalls vorhandene funktionelle Gruppen in geschützter Form vorliegen können, acyliert, oder

b) eine 2-Cephemverbindung der Formel

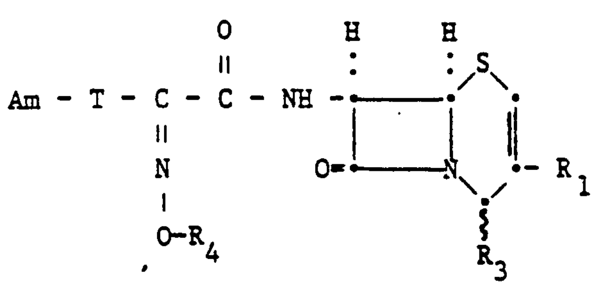

(IV)

worin gegebenenfalls vorhandene funktionelle Gruppen in geschützter Form vorliegen können, zur entsprechenden 3-Cephemverbindung isomerisiert, oder

c) eine Verbindung der Formel

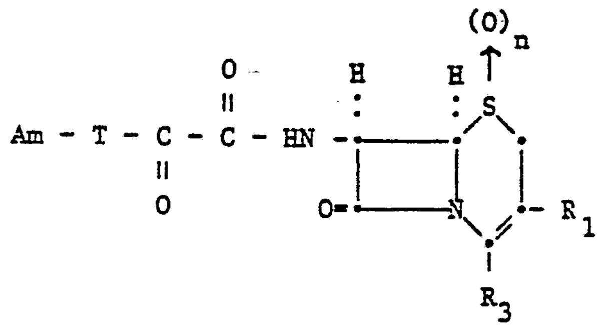

(V)

worin gegebenenfalls vorhandene funktionelle Gruppen in geschützter Form vorliegen können, mit einem gegebenenfalls O-substituierten Hydroxylamin der Formel $H_2N-O-R_4$ (VI) umsetzt, oder

d) zur Herstellung einer Verbindung der Formel I, worin $R_4$ Wasserstoff bedeutet, eine Verbindung der Formel

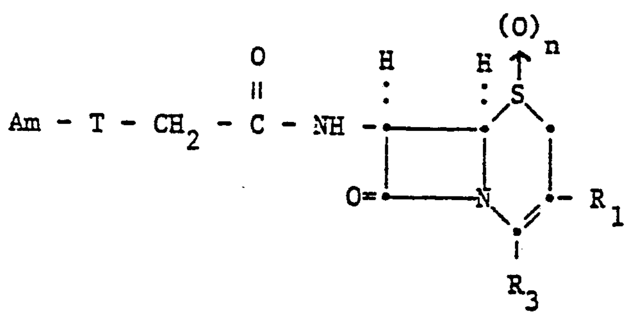

(VII),

worin gegebenenfalls vorhandene funktionelle Gruppen in geschütz- Form vorliegen können, mit einem Nitrosierungsmittel behandelt, oder

e) zur Herstellung einer Verbindung der Formel I, worin Am eine freie primäre oder sekundäre Aminogruppe bedeutet, und T für den zweiwertigen 1,2,4-Thiadiazolrest steht, dessen 5-Stellung mit der Aminogruppe Am und dessen 3-Stellung mit der Gruppe der Formel $-C(=N-O-R_4)-$ verbunden ist, eine Verbindung der Formel

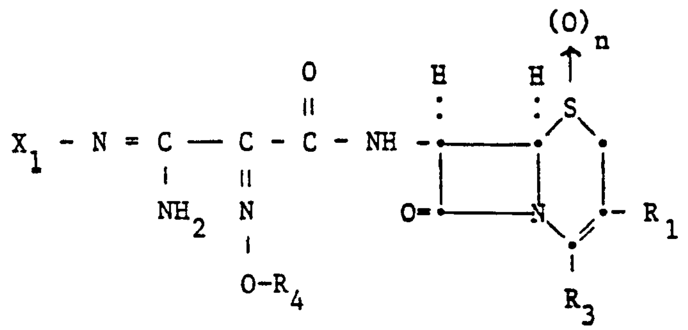

(VIII)

worin weitere, gegebenenfalls vorhandene funktionelle Gruppen in geschützter Form vorliegen können, und worin $X_1$ Halogen oder Hydroxy bedeutet, mit einem Salz der Rhodanwasserstoffsäure bzw. mit einem Isorhodanwasserstoffsäureester, oder worin $X_1$ Wasserstoff ist, mit Rhodan behandelt, oder

f) zur Herstellung einer Verbindung der Formel I, worin $R_1$ für Wasserstoff steht,

fa) in einer Verbindung der Formel

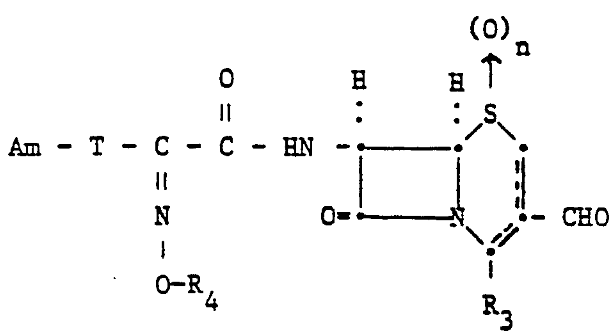

(IX)

worin die Doppelbindung in 2,3- oder in 3,4-Stellung sein kann, und worin gegebenenfalls vorhandene funktionelle Gruppen in geschützter Form vorliegen können, die Formylgruppe mittels Decarbonylierung durch Wasserstoff ersetzt, oder

fb) in einer Verbindung der Formel

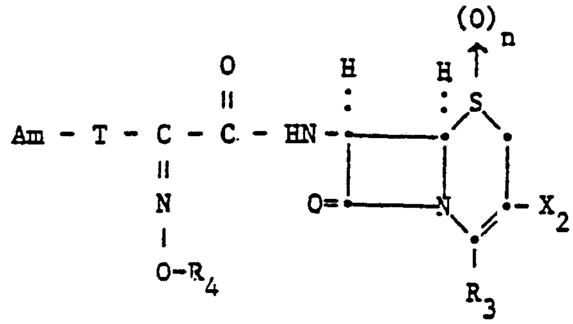

(X)

worin $X_2$ Hydroxy oder gegebenenfalls substituiertes Amino darstellt, und worin weitere gegebenenfalls vorhandene funktionelle Gruppen in geschützter Form vorliegen können, oder eine entsprechende tautomere Verbindung davon, $X_2$ durch Wasserstoff ersetzt, oder

fc) eine Verbindung der Formel

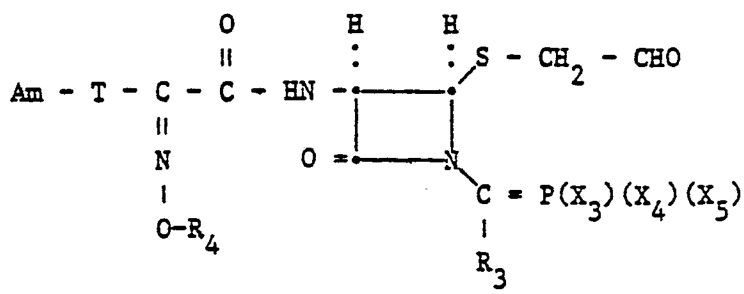

(XI)

worin $X_3$, $X_4$ und $X_5$ jeweils gegebenenfalls substituierte Kohlenwasserstoffreste darstellen, und worin weitere gegebenenfalls vorhandene funktionelle Gruppen in geschützter Form vorliegen, unter Abspaltung einer Verbindung der Formel $O = P(X_3)(X_4)(X_5)$ (XII) ringschliesst, oder

g) zur Herstellung einer Verbindung der Formel I, worin $R_1$ für veräthertes oder verestertes Hydroxy steht,

ga) eine Verbindung der Formel

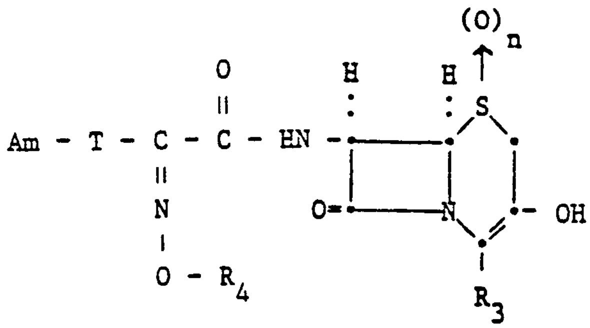

(XIII)

oder die entsprechende Tautomere 3-Oxo-cepham-Verbindung, worin $R_3$ funktionell abgewandeltes Carboxyl darstellt, und weitere gegebenenfalls vorhandene funktionelle Gruppen in geschützter Form vorliegen können, mit einem veräthernden oder veresternden Mittel behandelt, oder

gb) eine Verbindung der Formel

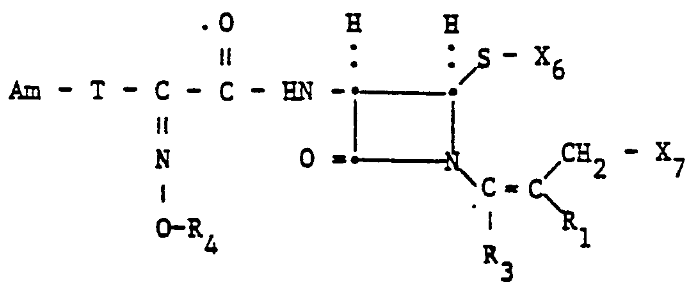

(XIV)

worin $X_6$ eine Abgangsgruppe darstellt, $X_7$ Wasserstoff oder Halogen bedeutet, und $R_1$ für veräthertes oder verestertes Hydroxy steht, und worin weitere gegebenenfalls vorhandene funktionelle Gruppen in geschützter Form vorliegen können, unter Abspaltung der Gruppen $X_6$ und $X_7$ ringschliesst, oder

h) zur Herstellung einer Verbindung der Formel I, worin $R_1$ den Rest der Formel $-CH_2-R_2$ darstellt, worin $R_2$ eine Ammoniogruppe bedeutet, eine Verbindung der Formel

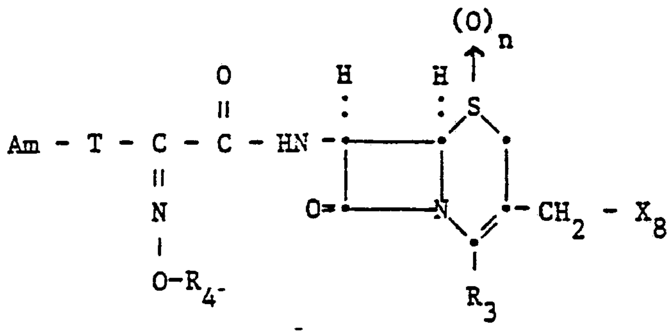

(XV)

worin $X_8$ einen durch nucleophile Substitution ersetzbaren Rest darstellt, und worin gegebenenfalls vorhandene funktionelle Gruppen in geschützter Form vorliegen können, mit einer organischen tertiären Aminbase umsetzt, oder

i) zur Herstellung einer Verbindung der Formel I, worin $R_1$ für den Rest der Formel $-CH_2-R_2$ darstellt, worin $R_2$ Acyl oder gegebenenfalls funktionell abgewandeltes Carboxy darstellt, eine Verbindung der Formel XIII oder eine entsprechende tautomere 3-Oxo-cepham-Verbindung, worin die Gruppe $R_3$ als funktionell abgewandelte Carboxylgruppe vorliegt, und worin andere, gegebenenfalls vorhandene funktionelle Gruppen in geschützter Form vorliegen können, mit einem Ylid der Formel $X_9^{\oplus}-^{\ominus}CH-R_2$ (XVI), worin $X_9^{\oplus}$ eine dreifach substituierte Phosphoniumgruppe oder eine zweifach veresterte Phosphonogruppe zusammen mit einem Kation bedeutet, und worin $R_2$ für Acyl oder eine gegebenenfalls funktionell abgewandelte Carboxylgruppe steht, umsetzt,

j) zur Herstellung von Verbindungen der Formel I, worin Am eine primäre, sekundäre oder tertiäre Aminogruppe ist, eine Verbindung der Formel

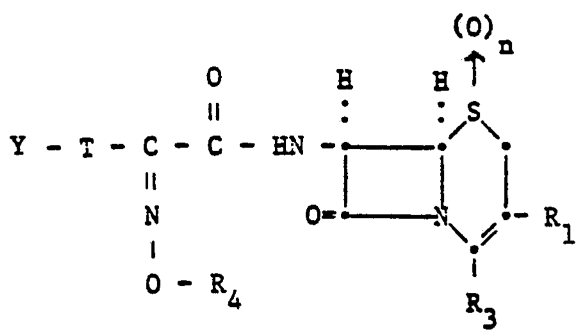

(XVII) ,

worin Y Halogen ist und $R_1$, $R_3$ und $R_4$ die oben angegebenen Bedeutungen haben, mit Ammoniak oder einem primären oder sekundären Amin Am-H, oder einem Metallamid davon, umsetzt, und, wenn erwünscht, eine erfindungsgemäss erhältliche Verbindung der Formel I in eine andere Verbindung der Formel I umwandelt, und/oder, wenn erwünscht oder notwendig, in einer erfindungsgemäss erhältlichen Verbindung der Formel I eine in geschützter Form vorliegende funktionelle Gruppe in die freie funktionelle Gruppe überführt, und/oder,
wenn erwünscht, ein erfindungsgemäss erhältliches Salz in die freie Verbindung oder in ein anderes Salz überführt, und/oder, wenn erwünscht, eine erfindungsgemäss erhältliche freie Verbindung mit einer salzbildenden Gruppe in ein Salz überführt,und/oder, wenn erwünscht, ein erfindungsgemäss erhältliches Gemisch von isomeren Verbindungen der Formel I in die einzelnen Isomeren auftrennt.

Verfahren a) (Acylierung): Gegebenenfalls vorhandene, die Aminogruppe in einem Ausgangsmaterial der Formel II substituierende und deren Acylierung erlaubende Reste in einem Ausgangsmaterial der Formel II sind beispielsweise organische Silyl- oder Stannylgruppen, ferner auch Ylidengruppen, die zusammen mit der Aminogruppe eine Schiff'sche Base bilden. Die genannten organischen Silyl- oder Stannylgruppen sind z.B. diejenigen, die auch mit einer Carboxylgruppe $R_3$ eine geschützte Carboxylgruppe zu bilden vermögen. Es sind dies in erster Linie Triniederalkylsilyl, insbesondere Trimethylsilyl. Bei der Silylierung oder Stannylierung zum Schutz einer Carboxylgruppe $R_3$ in einem Ausgangsmaterial der Formel II, kann, bei Verwendung eines Ueberschusses des Silylierungs- oder Stannylierungsmittels, die Aminogruppe ebenfalls silyliert oder stannyliert werden. Die genannten Ylidengruppen sind in erster Linie 1-Aryl-niederalkyliden-, insbesondere 1-Arylmethylengruppen, worin Aryl besonders für einen carbocyclischen, in erster Linie monocyclischen Arylrest, z.B. für gegebenenfalls, wie durch Niederalkyl, Hydroxy, Niederalkoxy und/oder Nitro, substituiertes Phenyl steht.

Weitere, in einem Ausgangsmaterial der Formel II vorhandene funktionelle Gruppen können, z.B. durch die obgenannten Schutzgruppen, geschützt sein. Vorzugsweise sind die an der Acylierungsreaktion nicht teilnehmenden funktionellen Gruppen, insbesondere gegebenenfalls vorhandene acylierbare Amino-, Hydroxy- und Mercaptogruppen, entsprechend geschützt.

In einem Acylrest der Säure der Formel III kann eine vorhandene Aminogrupe auch in ionischer Form geschützt sein, d.h. das Ausgangsmaterial der Formel III mit einer solchen Aminogruppe kann in Form eines Säureadditionssalzes, vorzugsweise mit einer starken anorganischen Säure, wie einer Halogenwasserstoffsäure, z.B. Salzsäure, oder Schwefelsäure verwendet werden.

Den Acylrest einer Carbonsäure der Formel III einführende Acylierungsmittel sind beispielsweise die Carbonsäure selbst oder reaktionsfähige funktionelle Derivate davon.

Falls eine freie Säure der Formel III, worin vorhandene funktionelle Gruppen ausser der an der Reaktion teilnehmenden Carboxylgruppe geschützt sein können, zur Acylierung eingesetzt wird, wird die Reaktion üblicherweise in Gegenwart von geeigneten Kondensationsmitteln, wie Carbodiimiden, beispielsweise N,N'-Diäthyl-, N,N'-Dipropyl-, N,N'-Dicyclohexyl- oder N-Aethyl-N'-3-dimethylaminopropylcarbodiimid, geeigneten Carbonylverbindungen, beispielsweise Carbonyldiimidazol, oder 1,2-Oxazoliumverbindungen, wie 2-Aethyl-5-phenyl-1,2-oxazolium-3'-sulfonat oder 2-tert.-Butyl-5-methyl-1,2-oxazoliumperchlorat, oder einer geeigneten Acylaminoverbindung, z.B. 2-Aethoxy-1-äthoxycarbonyl-1,2-dihydro-chinolin, durchgeführt.

Die Kondensationsreaktion wird vorzugsweise in einem wasserfreien Reaktionsmedium, vorzugsweise in Gegenwart eines Lösungs- oder Verdünnungsmittels, z.B. Methylenchlorid, Dimethylformamid, Acetonitril oder Tetrahydrofuran, wenn erwünscht oder notwendig,

unter Kühlen oder Erwärmen, z.B. in einem Temperaturbereich von etwa -40°C bis etwa +100°C, bevorzugt von etwa -20°C bis etwa +50° C und/oder in einer Inertgas-, z.B. Stickstoffatmosphäre durchgeführt.

Ein reaktionsfähiges, Amid-bildendes funktionelles Derivat einer Säure der Formel III, worin vorhandene funktionelle Gruppen ausser der an der Reaktion beteiligten Carboxylgruppe geschützt sein können, ist in erster Linie ein Anhydrid einer solchen Säure, inklusive und vorzugsweise ein gemischtes Anhydrid. Gemischte Anhydride sind z.B. diejenigen mit anorganischen Säuren, wie Halogenwasserstoffsäuren, d.h. die entsprechenden Säurehalogenide, z.B. -chloride oder -bromide, ferner mit Stickstoffwasserstoffsäure, d.h. die entsprechenden Säureazide, mit einer phosphorhaltigen Säure, z.B. Phosphorsäure, Diäthylphosphorsäure oder phosphoriger Säure, oder mit einer schwefelhaltigen Säure, z.B. Schwefelsäure, oder mit Cyanwasserstoffsäure. Weitere gemischte Anhydride sind z.B. diejenigen mit organischen Carbonsäuren, wie mit gegebenenfalls, z.B. durch Halogen, wie Fluor oder Chlor, substituierten Niederalkancarbonsäuren, z.B. Pivalinsäure oder Trichloressigsäure, oder mit Halbestern, insbesondere Niederalkylhalbestern der Kohlensäure, wie dem Aethyl- oder Isobutylhalbester der Kohlensäure, oder mit organischen, insbesondere aliphatischen oder aromatischen, Sulfonsäuren, z.B. p-Toluolsulfonsäure.

Weitere zur Reaktion mit der Aminogruppe geeignete Derivate einer Säure der Formel III, worin vorhandene funktionelle Gruppen ausser der an der Reaktion teilnehmenden Carboxylgruppe geschützt sein können, sind aktivierte Ester, wie Ester mit vinylogen Alkoholen (d.h. Enolen, wie vinylogen Niederalkenolen), oder Iminomethylesterhalogeniden, wie Dimethyliminomethylesterchlorid (hergestellt aus der Carbonsäure und z.B. Dimethyl-(1-chlor-äthyliden)-imminium-chlorid der Formel $(CH_3)_2N^{\oplus}{=}C(Cl)CH_3\ Cl^{\ominus}$, das man z.B. aus N,N-Dimethyl-acetamid und Phosgen erhalten kann), oder Arylester, wie vorzugsweise geeignet, z.B. durch Halogen, wie Chlor, und/oder Nitro, substituierte Phenylester, z.B. 4-Nitro-phenyl-, 2,3-Dinitrophenyl- oder 2,3,4,5,6-Pentachlor-phenylester, N-heteroaromatische Ester, wie N-Benztriazol-, z.B. 1-Benztriazolester, oder N-Diacyliminoester, wie N-Succinylimino- oder N-Phthalyliminoester.

Die Acylierung mit einem Säurederivat einer Säure der Formel III, wie einem entsprechenden Anhydrid, insbesondere einem Säurehalogenid, wird bevorzugt in Anwesenheit eines säurebindenden Mittels, beispielsweise einer organischen Base, wie eines organischen Amins, z.B. eines tertiären Amins, wie Triniederalkylamin, z.B. Trimethylamin, Triäthylamin oder Aethyl-diisopropylamin, oder N,N-Diniederalkyl-anilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder eines Oxirans, beispielsweise eines 1,2-Niederalkylenoxids, wie Aethylenoxid oder Propylenoxid, durchgeführt.

Die obigen Acylierungen werden vorzugsweise in einem inerten, vorzugsweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essigsäureäthylester, oder einem Nitril, z.B. Acetonitril, oder einer Mischung davon, wenn notwendig oder erwünscht, bei erniedrigter oder erhöhter Temperatur, z.B. in einem Temperaturbereich von etwa -40°C bis etwa +100°C, bevorzugt von etwa -10°C bis etwa +50°C, und/oder in einer Inertgas-, z.B. Stickstoffatmosphäre.

Die Acylierung kann auch durch Verwendung eines geeigneten Derivates der Säure der Formel III in Gegenwart einer geeigneten Acylase erfolgen. Solche Acylasen sind bekannt und können von einer Anzahl von Mikroorganismen gebildet werden, z.B. durch Acetobacter, wie Acetobacter aurantium, Achromobacter, wie Achromobacter aeris, Aeromonas, wie Aeromonas hydrophila, oder Bacillus, wie Bacillus megaterium 400. In einer solchen enzymatischen Acylierung werden insbesondere Amide, Ester oder Thioester, wie Niederalkyl-, z.B. Methyl- oder Aethylester, der Carbonsäure der Formel III als entsprechende Derivate eingesetzt. Ueblicherweise wird eine solche Acylierung in einem, den entsprechenden Mikroorganismus enthaltenden Nährmedium, in einem Filtrat der Kulturbrühe oder, gegebenenfalls nach Isolierung der Acylase, inklusive nach Adsorption an einen Träger, in einem wässrigen, gegebenenfalls einen Puffer enthaltenden Medium, z.B. in einem Temperaturbereich von etwa +20°C bis etwa +40°C, bevorzugt bei etwa +37° C, durchgeführt.

Ein in der Acylierungsreaktion eingesetztes Derivat einer Säure der Formel III kann, wenn erwünscht, in situ gebildet werden. So kann man z.B. ein gemischtes Anhydrid durch Behandeln einer Säure der Formel III (gegebenenfalls mit entsprechend geschützten funktionellen Gruppen) oder eines geeigneten Salzes davon, wie eines Ammoniumsalzes, z.B. mit einer organischen Base, wie Pyridin oder 4-Methylmorpholin, oder eines Metall-, z.B. Alkalimetallsalzes, mit einem geeigneten Säurederivat, wie einem entsprechenden Säurehalogenid einer gegebenenfalls substituierten Niederalkalcarbonsäure, z.B. Trichloracetylchlorid, mit einem Halbester eines Kohlensäurehalbhalogenids, z.B. Chlorameisensäureäthylester oder -isobutylester, oder mit einem Halogenid einer Diniederalkylphosphorsäure, z.B. Diäthylphosphorbromidat (das man durch Umsetzen von Triäthylphosphit mit Brom bilden kann) erhalten und das so erhältliche gemischte Anhydrid ohne Isolierung in der Acylierungsreaktion verwenden.

Verfahren b) (Isomerisierung): In einem 2-Cephem-Ausgangsmaterial der Formel IV weist die gegebenenfalls geschützte Carboxylgruppe $R_3$ in der 4-Stellung vorzugsweise die α-Konfiguration auf.

2-Cephem-Verbindungen der Formel IV können isomerisiert werden, indem man sie mit einem schwach-basischen Mittel behandelt und die entsprechende 3-Cephem-Verbindung isoliert. Geeignete Isomerisierungsmittel sind z.B. organische, stickstoffhaltige Basen, insbesondere tertiäre heterocyclische Basen aromatischen Charakters, in erster Linie Basen des Pyridin-Typs, wie Pyridin selber, sowie Picoline, Collidine oder Lutidine, ferner Chinolin, tertiäre aromatische Basen, z.B. solche des Anilin-Typs, wie N,N-Diniederalkylaniline, z.B. N,N-Dimethylanilin oder N,N-Diäthylanilin, oder tertiäre aliphatische, azacycloaliphatische oder araliphatische Basen, wie N,N,N-Triniederalkylamine, z.B. N,N,N-Trimethylamin oder N,N-Diisopropyl-N-äthylamin, N-Niederalkyl-azacycloalkane, z.B. N-Methyl-piperidin, oder N-Phenylniederalkyl-N,N-diniederalkyl-amine, z.B. N-Benzyl-N,N-dimethylamin, sowie Gemische von solchen basischen Mitteln, wie das Gemisch einer Base vom Pyridintyp und eines N,N,N-Tri-niederalkylamins, z.B. Pyridin und Triäthylamin. Ferner können auch anorganische oder organische Salze von Basen, insbesondere von mittelstarken bis starken Basen mit schwachen Säuren, wie Alkalimetall-, oder Ammoniumsalze von Niederalkancarbonsäuren, z.B. Natriumacetat, Triäthylammoniumacetat oder N-Methyl-piperidinacetat, sowie andere analoge Basen oder Gemische von solchen basischen Mitteln verwendet werden.

Bei der Isomerisierung von 2-Cephem-Verbindungen der Formel IV mit basischen Mitteln arbeitet man vorzugsweise in wasserfreiem Medium, in An- oder Abwesenheit eines Lösungsmittels, wie eines gegebenenfalls halogenierten, z.B. chlorierten, aliphatischen, cycloaliphatischen oder aromatischen Kohlenwasserstoffs, oder eines Lösungsmittelgemisches, wobei als Reaktonsmittel verwendete, unter den Reaktonsbedingungen flüssige Basen gleichzeitig auch als Lösungsmittel dienen können, gegebenenfalls unter Kühlen oder unter Erwärmen, vorzugsweise in einem Temperaturbereich von etwa -30°C bis etwa +100°C, in einer Inertgas-, z.B. Stickstoffatmosphäre, und/oder in einem geschlossenen Gefäss.

So erhältliche 3-Cephem-Verbindungen der Formel I lassen sich in an sich bekannter Weise, z.B. durch Adsorption und/oder Kristallisation, von gegebenenfalls noch vorhandenen 2-Cephem-Ausgangsstoffen abtrennen.

Die Isomerisierung von 2-Cephem-verbindungen der Formel IV wird vorzugsweise durchgeführt, indem man diese in 1-Stellung oxidiert, wenn erwünscht, gegebenenfalls Isomerengemische der gebildeten 1-Oxide trennt, und die so erhältlichen 1-Oxide der entsprechenden 3-Cephemverbindungen, wenn erwünscht, reduziert.

Als geeignete Oxidationsmittel für die Oxidation in 1-Stellung von 2-Cephem-verbindungen der Formel IV kommen anorganische Persäuren, die ein Reduktionspotential von wenigstens +1,5 Volt aufweisen und aus nicht-metallischen Elementen bestehen, organische Persäuren oder Gemische aus Wasserstoffperoxid und Säuren, insbesondere organische Carbonsäuren, mit einer Dissoziationskonstante von wenigstens $10^{-5}$ in Frage. Geeignete anorganische Persäuren sind Perjod- und Perschwefelsäure. Organische Persäuren sind entsprechende Percarbon- und Persulfonsäuren, die als solche zugesetzt oder durch Verwendung von wenigstens einem Aequivalent Wasserstoffperoxyd und einer Carbonsäure in situ gebildet werden können. Dabei ist es zweckmässig, einen grossen Ueberschuss der Carbonsäure anzuwenden, wenn z.B. Essigsäure als Lösungsmittel verwendet wird. Geeignete Persäuren sind z.B. Perameisensäure, Peressigsäure, Trifluorperessigsäure, Permaleinsäure, Perbenzoesäure, 3-Chlorperbenzoesäure, Monoperphthalsäure oder p-Toluolpersulfonsäure.

Die Oxidation kann ebenfalls unter Verwendung von Wasserstoffperoxid mit katalytischen Mengen einer Säure mit einer Dissoziationskonstante von wenigstens $10^{-5}$ durchgeführt werden, wobei man niedrige Konzentrationen, z.B. 1-2% und weniger, aber auch grössere Mengen der Säure einsetzen kann. Dabei hängt die Wirksamkeit des Gemisches in erster Linie von der Stärke der Säure ab. Geeignete Gemische sind z.B. solche von Wasserstoffperoxid mit Essigsäure, Perchlorsäure oder Trifluoressigsäure.

Die obige Oxidation kann in Gegenwart von geeigneten Katalysatoren durchgeführt werden. So kann z.B. die Oxidation mit Percarbonsäuren durch die Anwesenheit einer Säure mit einer Dissoziationskonstante von wenigstens $10^{-5}$ katalysiert werden, wobei ihre Wirksamkeit von ihrer Stärke abhängt. Als Katalysatoren geeignete Säuren sind z.B. Essigsäure, Perchlorsäure und Trifluoressigsäure. Ueblicherweise verwendet man mindestens äquimolare Mengen des Oxidationsmittels, vorzugsweise einen geringen Ueberschuss von etwa 10% bis etwa 20%, wobei man auch grössere Ueberschüsse, d.h. bis zur 10-fachen Menge des Oxidationsmittels oder darüber, verwenden kann. Die Oxidation wird unter milden Bedingungen , z.B. bei Temperaturen von etwa -50°C bis etwa +100°C, vorzugsweise von etwa -10°C bis etwa +40°C durchgeführt.

Die Reduktion der 1-Oxide von 3-Cephem-Verbindungen kann in an sich bekannter Weise durch Behandeln mit einem Reduktionsmittel, wenn notwendig, in Anwesenheit eines aktivierenden Mittels, durchgeführt werden. Als Reduktionsmittel kommen beispielsweise in Betracht: Katalytisch aktivierter Wasserstoff, wobei Edelmetallkatalysatoren verwendet werden, welche Palladium, Platin oder Rhodium enthalten, und die man gegebenenfalls zusammen mit einem geeigneten Trägermaterial, wie Kohle oder Bariumsulfat, einsetzt; reduzierende Zinn-, Eisen-, Kupfer- oder Mangankationen, welche in Form von entsprechenden Verbindungen oder Komplexen anorganischer oder organischer Art,

z.B. als Zinn-II-chlorid, -fluorid, -acetat oder -formiat, Eisen-II-chlorid, -sulfat, -oxalat oder -succinat, Kupfer-I-chlorid, -benzoat oder -oxyd, oder Mangan-II-chlorid, -sulfat, -acetat oder -oxyd, oder als Komplexe, z.B. mit Aethylendiamintetraessigsäure oder Nitrolotriessigsäure, verwendet werden; reduzierende Dithionit-, Jod- oder Eisen-II-cyanid-anionen, welche in Form von entsprechenden anorganischen oder organischen Salzen, wie Alkalimetall-, z.B. Natrium- oder Kaliumdithionit, Natrium- oder Kaliumjodid oder -eisen-II-cyanid, oder in Form der entsprechenden Säuren, wie Jodwasserstoffsäure, verwendet werden; reduzierende trivalente anorganische oder organische Phosphorverbindungen, wie Phosphine, ferner Ester, Amide und Halogenide der phosphinigen, phosphonigen oder phosphorigen Säure, sowie diesen Phosphorsauerstoffverbindungen entsprechende Phosphor-Schwefelverbindungen, worin organische Reste in erster Linie aliphatische, aromatische oder araliphatische Reste, z.B. gegebenenfalls substituiertes Niederalkyl, Phenyl oder Phenylniederalkyl darstellen, wie z.B. Triphenylphosphin, Tri-n-butylphosphin, Diphenylphosphinigsäuremethylester, Diphenylchlorphosphin, Phenyldichlorphosphin, Benzolphosphonigsäuredimethylester, Butanphosphonigsäuremethylester, Phosphorigsäuretriphenylester, Phosphorigsäuretrimethylester, Phosphortrichlorid, Phosphortribromid, etc.; reduzierende Halogensilanverbindungen, die mindestens ein an das Siliciumatom gebundenes Wasserstoffatom aufweisen, und die ausser Halogen, wie Chlor, Brom oder Jod, auch organische Reste, wie aliphatische oder aromatische Gruppen, z.B. gegebenenfalls substituiertes Niederalkyl oder Phenyl, aufweisen können, wie Chlorsilan, Bromsilan, Di- oder Trichlorsilan, Di- oder Tribromsilan, Diphenylchlorsilan, oder Dimethylchlorsilan, sowie auch Halogensilanverbindungen, worin alle Wasserstoffe durch organische Reste ersetzt sind, wie Triniederalkylhalogensilan, z.B. Trimethylchlorsilan oder Trimethyljodsilan, oder cyclische, Schwefel-enthaltende Silane, wie 1,3-Dithia-2,4-disilacyclobutane oder 1,3,5-Trithia-2,4,6-trisila-cyclohexane, deren Siliciumatome durch Kohlenwasserstoffreste, wie insbesondere Niederalkyl substituiert sind,

z.B. 2,2,4,4-Tetramethyl-1,3-dithia-2,4-disilacyclobutan oder 2,2,4,4,6,6-Hexamethyl-1,3,5-trithia-2,4,6-trisilacyclohexan, etc.; reduzierende quaternäre Chlormethylen-iminiumsalze, insbesondere -chloride oder -bromide, worin die Iminiumgruppe durch einen bivalenten oder zwei monovalente organische Reste, wie gegebenenfalls substituiertes Niederalkylen bzw. Niederalkyl substituiert ist, wie N-Chlormethylen-N,N-diäthyliminiumchlorid oder N-Chlormethylen-pyrrolidiniumchlorid; oder komplexe Metallhydride, wie Natriumborhydride, in Gegenwart von geeigneten Aktivierungsmitteln, wie Cobalt-II-chlorid, sowie Borandichlorid.

Als aktivierende Mittel, die zusammen mit denjenigen der obgenannten Reduktionsmittel verwendet werden, welche selber nicht Lewissäure-Eigenschaften aufweisen, d.h. in erster Linie zusammen mit den Dithionit-, Jod- oder Eisen-II-cyanid- und den nicht-halogenhaltigen trivalenten Phosphor-Reduktionsmitteln oder bei der katalytischen Reduktion eingesetzt werden, sind insbesondere organische Carbon- und Sulfonsäurehalogenide, ferner Schwefel-, Phosphor- oder Siliciumhalogenide mit gleicher oder grösserer Hydrolysenkonstante zweiter Ordnung als Benzoylchlorid, z.B. Phosgen, Oxalylchlorid, Essigsäurechlorid oder -bromid, Chloressigsäurechlorid; Pivalinsäurechlorid, 4-Methoxybenzoesäurechlorid, 4-Cyanbenzoesäurechlorid, p-Toluolsulfonsäurechlorid, Methansulfonsäurechlorid, Thionylchlorid, Phosphoroxychlorid, Phosphortrichlorid, Phosphortribromid, Phenyldichlorphosphin, Benzolphosphonigsäuredichlorid, Dimethylchlorsilan oder Trichlorsilan, ferner geeignete Säureanhydride, wie Trifluoressigsäureanhydrid, oder cyclische Sultone, wie Aethansulton, Propansulton, 1,3-Butansulton oder 1,3-Hexansulton zu erwähnen.

Die Reduktion wird vorzugsweise in Gegenwart von Lösungsmitteln oder Gemischen davon durchgeführt, deren Auswahl in erster Linie durch die Löslichkeit der Ausgangsstoffe und die Wahl des Reduktionsmittels

bestimmt wird, so z.B. Niederalkancarbonsäuren oder Ester davon, wie Essigsäure und Essigsäureäthylester, bei der katalytischen Reduktion, und z.B. gegebenenfalls substituierte, wie halogenierte oder nitrierte, aliphatische, cycloaliphatische, aromatische oder araliphatische Kohlenwasserstoffe, z.B. Benzol, Methylenchlorid, Chloroform oder Nitromethan, geeignete Säurederivate, wie Niederalkancarbonsäureester oder -nitrile, z.B. Essigsäureäthylester oder Acetonitril, oder Amide von anorganischen oder organischen Säuren, z.B. Dimethylformamid oder Hexamethylphosphoramid, Aether, z.B. Diäthyläther, Tetrahydrofuran oder Dioxan, Ketone, z.B. Aceton, oder Sulfone, insbesondere aliphatische Sulfone, z.B. Dimethylsulfon oder Tetramethylensulfon, etc., zusammen mit den chemischen Reduktionsmitteln, wobei diese Lösungsmittel vorzugsweise kein Wasser enthalten. Dabei arbeitet man gewöhnlicherweise bei Temperaturen von etwa -20°C bis etwa 100°C, wobei bei Verwendung von sehr reaktionsfähigen Aktivierungsmitteln die Reaktion auch bei tieferen Temperaturen durchgeführt werden kann.

Verfahren c) (Bildung der gegebenenfalls substituierten Hydroximinogruppe): In einem Ausgangsmaterial der Formel V, worin gegebenenfalls vorhandene funktionelle Gruppen geschützt sein können, kann die nicht zur Amidgruppierung gehörende Oxogruppe in der 7β-Acylamidogruppe durch Behandeln mit einem gegebenenfalls O-substituierten Hydroxylamin der Formel VI in eine gegebenenfalls O-substituierte Hydroxyiminogruppe übergeführt werden. Die Reaktion eines Ausgangsmaterials der Formel V mit einer Hydroxylaminverbindung der Formel VI wird in üblicher Weise durchgeführt, z.B. indem man die beiden Reaktionspartner in einem Lösungsmittel, wie Wasser oder einem organischen Lösungsmittel, wie einem Alkohol, z.B. Methanol, bei leicht erhöhter oder erniedrigter Temperatur, z.B. in einem Temperaturbereich von etwa -20°C bis etwa +100°C, bevorzugt von etwa 0°C bis etwa 30°C, gegebenenfalls in einer Inertgas-, wie Stickstoffatmosphäre, umsetzt. Die Hydroxylaminverbindung der Formel VI kann auch in situ,

z.B. aus einem ihrer Salze, wie einem Hydrohalogenid, wie Hydrochlorid, durch Behandeln mit einer anorganischen Base, wie einem Alkalimetallhydroxid, z.B. Natriumhydroxid, oder einer organischen Base, wie einem tertiären Amin, z.B. einem Triniederalkylamin, wie Triäthylamin oder Aethyl-diisopropylamin, oder einer heterocyclischen tertiären Base, wie Pyridin, in Freiheit gesetzt werden.

Verfahren d) (Bildung der unsubstituierten Hydroximinogruppe): In einem Ausgangsmaterial der Formel VII sind die funktionellen Gruppen vorzugsweise geschützt. Geeignete Nitrosierungsmittel sind salpetrige Säure und davon abgeleitete Derivate, wie Nitrosylhalogenide, z.B. Nitrosylchlorid oder Nitrosylbromid, Salze der salpetrigen Säure, wie Alkalimetallsalze, z.B. Natrium- oder Kaliumnitrit, oder insbesondere Ester der salpetrigen Säure, wie entsprechende Niederalkylester, z.B. Butyl-, Pentyl- oder insbesondere Isoamylnitrit. Verwendet man als Nitrosierungsmittel ein Salz der salpetrigen Säure, so wird die Reaktion vorzugsweise in Gegenwart einer starken anorganischen oder organischen Säure, z.B. Chlorwasserstoffsäure, Schwefelsäure, Ameisensäure oder Essigsäure, durchgeführt. Bei Verwendung eines Esters der salpetrigen Säure wird die Reaktion vorzugsweise in Gegenwart einer starken Base, wie eines Alkalimetallalkanolates durchgeführt.

Die Nitrosierung wird in einem geeigneten Lösungsmittel, wie Wasser, einer Carbonsäure, z.B. Essigsäure, einem Niederalkanol, z.B. Methanol oder Aethanol, einem Aether, z.B. Dioxan oder Tetrahydrofuran, oder einem Kohlenwasserstoff, z.B. Hexan oder Benzol, oder in Mischungen davon, wenn notwendig unter Kühlen oder Erwärmen, insbesondere in einem Temperaturbereich von etwa -15°C bis etwa Raumtemperatur, und/oder in einer Inertgasatmosphäre durchgeführt.

Verfahren e) (Bildung der Aminothiadiazolylgruppe): In einem Ausgangsmaterial der Formel VIII ist $X_1$ als Halogen beispielsweise Chlor oder Jod, bevorzugt jedoch Brom. Geeignete Salze der Rhodanwasserstoffsäure sind beispielsweise entsprechende Alkali-, Erdalkali- oder auch Schwermetallsalze, z.B. das Natrium-, Kalium-, Magnesium- oder Bleirhodanid, oder auch Ammoniumrhodanide, deren Ammoniumteil von Ammoniak oder von organischen Stickstoffbasen abgeleitet ist, z.B. Ammonium- oder Mono-, Di- Tri- oder Tetra-niederalkylammoniumrhodanid, worin Niederalkyl z.B. Methyl, Aethyl oder Isopropyl bedeutet.

Geeignete Isorhodanwasserstoffsäureester sind beispielsweise Isorhodanwasserstoffsäureniederalkylester, die auch als Niederalkylisothiocyanate bezeichnet werden, wie Methyl-, Aethyl-, Propyl- oder Butylisothiocyanat.

Die Kondensation von Verbindungen der Formel VIII mit einem Salz der Rhodanwasserstoffsäure oder mit Rhodan ergibt Verbindungen der Formel I, worin Am eine freie, primäre Aminogruppe ist, während die Kondensation mit einem Isorhodanwasserstoffsäureester eine Verbindung der Formel I ergibt, worin Am eine freie sekundäre Aminogruppe, beispielsweise eine Niederalkylaminogruppe ist.

Die Reaktion wird bevorzugt in einem inerten Lösungsmittel, wie einem Niederalkanol, z.B. Methanol, Aethanol, Propanol oder Isopropanol, einem Aether, wie Diäthyläther, Dioxan oder Tetrahydrofuran, einem Amid, wie Dimethylformamid, einem aliphatischen oder aromatischen Kohlenwasserstoff, wie Hexan, Benzol oder Toluol, oder auch in Mischungen davon, und, wenn notwendig, unter Kühlen oder Erwärmen, d.h. bei Temperaturen zwischen etwa -80°C bis etwa +100°C, bevorzugt zwischen etwa -20°C bis etwa +20°C, und/oder in einer Inertgasatmosphäre durchgeführt.

Ein Ausgangsmaterial der Formel VIII, worin $X_1$ Halogen ist, kann auch *in situ* hergestellt werden, gegebenenfalls in Gegenwart des

Rhodanwasserstoffsalzes, beispielsweise indem man eine Verbindung der Formel VIII, worin $X_1$ Wasserstoff ist, in einem der genannten Lösungsmittel mit einem Hypohalogenit, z.B. Natrium- oder Kaliumhypobromit, oder mit einem Halogen und einer Alkalimetallbase, wie einem Hydroxid, Carbonat oder Niederalkanolat, z.B. mit Brom und Natriummethylat oder Kaliumcarbonat, behandelt. Die Halogenierung findet bevorzugt unter Kühlen, z.B. bei einer Temperatur von etwa -15°C bis etwa 0°C statt.

Eine Verbindung der Formel VII, worin X Hydroxy ist, kann ebenfalls in situ hergestellt werden, beispielsweise indem man eine Verbindung der Formel VII, worin X Wasserstoff ist, mit einem Oxidationsmittel, wie Wasserstoffperoxid, behandelt.

Die Reaktion eines Ausgangsmaterials der Formel VIII, worin $X_1$ Wasserstoff ist ,mit Rhodan wird vorzugsweise ebenfalls in einem der genannten inerten Lösungsmittel und unter ähnlichen Reaktionsbedingungen durchgeführt. Gegebenenfalls kann auch ein Säureadditionssalz einer Verbindung der Formel VIII eingesetzt werden, dass in situ in die freie Verbindung übergeführt wird, oder das Rhodan kann in situ gebildet werden, z.B. aus einem Rhodanid, z.B. Bleirhodanid, und einem Halogen, z.B. Brom.

Verfahren fa) (Decarbonylierung): In einem Ausgangsmaterial der Formel IX sind die gegebenenfalls vorhandenen funktionellen Gruppen üblicherweise geschützt.

Der Ersatz der Formylgruppe durch Wasserstoff wird in erster Linie durch Behandeln des Ausgangsmaterials mit einem Kohlenmonoxydaufnehmenden Schwermetallkomplex durchgeführt. Solche Schwermetallkomplexe sind insbesondere Platinmetall-Komplexe, wobei man unter einem Platinmetall ausser Platin auch Iridium, Rhodium, Palladium und Osmium versteht.

Vorzugsweise verwendet man Bis-trisubstituierte Phosphin-platinhalogenide, Bis-trisubstituierte Phosphincarbonyliridiumhalogenide oder Tris-trisubstituierte Phosphin-iridiumhalogenide, in erster Linie Tris-trisubstituierte Phosphin-rhodiumhalogenide, worin die Substituenten des Phosphins vorzugsweise Niederalkyl, z.B. n-Butyl, und in erster Linie Phenyl und die Halogenide vorzugsweise Chloride sind. Solche Phosphinplatinmetall-Komplexe, welche Kohlenmonoxyd in kovalenter Bindung aufzunehmen vermögen, sind z.B. Bis-triphenylphosphin-platin-II-chlorid-$[(C_6H_5)_3P]_2PtCl_2$ oder Bis-triphenyl-phosphincarbonyliridium-II-chlorid $[(C_6H_5)_3P]_2$ Ir(CO)Cl, sowie Tris-triphenylphosphin-iridium-I-chlorid $[(C_6H_5)_3P]_3IrCl$, in erster Linie aber Tristriphenyl-phosphin-rhodium-I-chlorid $[(C_6H_5)_3P]_3RhCl$.

Wenn erwünscht oder notwendig, kann die Decarbonylierung mit den obgenannten Schwermetallkomplexen in Gegenwart von geeigneten Katalysatoren oder Aktivierungsmitteln, z.B. Lewissäure, wie Bortrifluorid (das man z.B. zusammen mit dem Bis-triphenylphosphin-platinchlorid verwenden kann), ferner eines Perchlorats, wie Alkalimetallperchlorat, z.B. Natriumperchlorat (das man z.B. zusammen mit Bis-triphenylphosphin-carbonyliridiumchlorid verwenden kann), durchgeführt werden.

Vorzugsweise arbeitet man in Gegenwart von inerten Lösungsmitteln, insbesondere von Kohlenwasserstoffen, wie aliphatischen oder cycloaliphatischen, insbesondere aromatischen Kohlenwasserstoffen, wie entsprechenden aliphatischen oder aromatischen Chlorkohlenwasserstoffen, z.B. Methylenchlorid oder Chlorbenzol, Aethern, wie aliphatischen, cycloaliphatischen oder aromatischen, ferner gemischten Aethern, z.B. Di-n-butyläther, Dioxan, Diphenyläther oder Anisol, Nitrilen, wie aliphatischen oder aromatischen Nitrilen, z.B. Acetonitril oder Benzonitril, oder Ketonen, insbesondere aliphatischen Ketonen, wie Niederalkanonen, z.B. Aceton, Aethylmethylketon oder Isobutylmethylketon, oder Gemischen von solchen Lösungsmitteln. Dabei

wird die Reaktion unter Kühlen, bei Zimmertemperatur oder unter Erwärmen, z.B. in einem Temperaturbereich von etwa 10°C bis etwa 150°C, wie von etwa 40°C bis etwa 120°C, ferner, wenn notwendig, in einem geschlossenen Gefäss und/oder in einer inerten Gasatmosphäre, z.B. unter Stickstoff oder Argon, durchgeführt.

Ausgehend von 2-Cephem-Ausgangsstoffen der Formel IX kann man verfahrensgemäss zu den 3-Cephem-Verbindungen der Formel I gelangen, indem unter den Reaktionsbedingungen der Decarbonylierung die Isomerisierung von einer 2-Cephem- zur erwünschten 3-Cephem-Verbindung erfolgen kann.

Verfahren fb) (Austausch einer Hydroxygruppe oder einer gegebenenfalls substituierten Aminogruppe durch Wasserstoff):
Gegebenenfalls substituiertes Amino $X_2$ in einem Ausgangsmaterial der Formel X kann einen oder zwei einwertige oder einen zweiwertigen Substituenten, wie Niederalkyl, Niederalkylen, Oxaniederalkylen oder N-substituiertes, z.B. N-niederalkyliertes Azaniederalkylen enthalten, und ist z.B. Amino, Methylamino, Dimethylamino, Diäthylamino, Pyrrolidino, Piperidino oder Morpholino.

Der Ersatz der Gruppe $X_2$ in einer Verbindung der Formel X durch Wasserstoff wird vorzugsweise in zwei Stufen durch Reduktion der 3,4-Doppelbindung im 3-Cephemring, gegebenenfalls Ueberführen einer Hydroxygruppe $X_2$ in eine veresterte Hydroxygruppe, und anschliessende Abspaltung der Elemente von Wasser, einer Säure oder eines Amins durchgeführt.

Beim Austausch von $X_2$ gegen Wasserstoff erfolgt die Reduktion der 3,4-Doppelbindung in Ausgangsstoffen der Formel X z.B. mittels geeigneter Hydrid-Reduktionsmittel. Solche sind in erster Linie komplexe Metallhydride, vorzugsweise entsprechende Borhydride, wie Diboran oder Alkalimetallborhydride, z.B. Natriumborhydrid oder

Lithiumborhydrid, ferner Zinkborhydrid, sowie organische Alkalimetallaluminiumhydride, wie Tri-niederalkoxy-alkalimetall-aluminiumhydride, z.B. Tri-(tert.-butyloxy)-lithiumaluminiumhydrid, die man üblicherweise in Gegenwart von Lösungsmitteln, insbesondere von relativ polaren Lösungsmitteln, wie Alkoholen, z.B. Niederalkanolen, wie Methanol oder Aethanol, oder Aethern, wie aliphatischen Aethern, z.B. Glycol- und Polyglycoläthern, wie Aethylenglycoldimethyläther oder Diäthylenglycoldimethyläther, oder cyclischen Aethern, wie Tetrahydrofuran oder Dioxan, oder Lösungsmittelgemischen, auch von wasserhaltigen Lösungsmitteln, anwendet, wobei man bei Temperaturen von etwa -20°C bis etwa 80°C, wenn notwendig, in einem geschlossenen Gefäss und/oder in einer Inertgas-, z.B. Stickstoffatmosphäre arbeitet.

Falls die obige Reduktion in Gegenwart eines geeigneten Wasser- oder Amin-abspaltenden Mittels durchgeführt wird, kann die erwünschte 3-unsubstituierte 3-Cephem-Verbindung der Formel I unmittelbar erhalten werden. Es kann aber auch zunächst eine entsprechende 3-$X_2$-Cepham-Verbindung entstehen, die entweder isoliert oder in situ weiterverarbeitet werden kann.

Wenn erwünscht, kann auf der Stufe der so erhältlichen Cepham-Verbindung eine Hydroxygruppe $X_2$ durch Verestern nach an sich bekannten Methoden (siehe z.B. Verfahren ga),z.B. durch Behandeln mit einem geeigneten Säureanhydrid, wie einem symmetrischen oder gemischten Säureanhydrid, inkl. einem entsprechenden Säurehalogenid, z.B. -chlorid, oder mit einem geeigneten Halogenierungsmittel, wie einer organischen Halogen-Phosphorverbindung, in eine veresterte Hydroxygruppe $X_2$ überführen. Veresterte Hydroxygruppen sind vorzugsweise durch starke organische oder anorganische Säuren, wie starke Mineralsäuren, z.B. Halogenwasserstoffsäuren, wie Chlorwasserstoffsäure, oder starke organische Sulfonsäuren, wie entsprechende niederaliphatische oder aromatische Sulfonsäuren, z.B.

Methansulfonsäure oder p-Toluolsulfonsäure, veresterte Hydroxygruppen und stellen z.B. Halogen, wie Chlor, Niederalkylsulfonyloxy, z.B. Methylsulfonyloxy, oder Aethylsulfonyloxy, oder Arylsulfonyloxy, z.B. p-Toluolsulfonyloxy dar.

In der zweiten Verfahrensstufe erfolgt dann die Abspaltung der Elemente von Wasser, einer Säure oder eines Amins, aus so erhältlichen Cepham-Verbindungen, wobei man diese vorzugsweise mit geeigneten Wasser-, Säure- oder Amin-abspaltenden Mitteln behandelt. Wasser und Amine werden üblicherweise mittels einer starken organischen Carbon-, wie einer Halogen-niederalkancarbonsäure, oder einer starken organischen Sulfonsäure, z.B. p-Toluolsulfonsäure oder Methansulfonsäure, abgespalten. Wasser kann auch mittels eines geeigneten wasserbindenden Säurederivats, wie eines Anhydrids oder insbesondere Säurehalogenids, wie Säurechlorids, einer von Phosphor oder Schwefel abgeleiteten Säure, wie einer entsprechenden anorganischen Säure, z.B. mittels Phosphoroxychlorid oder Thionylchlorid, oder einer organischen Sulfonsäure, wie einer niederaliphatischen oder aromatischen Sulfonsäure, z.B. mittels Tosylchlorid oder Mesylchlorid, abgespalten werden. Man arbeitet dabei vorzugsweise in Gegenwart einer Base, wie eines tertiären Amins, z.B. Triniederalkylamin oder einer heterocyclischen Base, wie Triäthylamin oder Pyridin, oder eines geeigneten Ionenaustauschers, z.B. eines mit Kationen beladenen, sulfonierten Polystyrol-Ionenaustauschers.

Ferner kann man zur Wasserabspaltung auch dehydratisierende Carbodiimidverbindungen, z.B. Dicyclohexylcarbodiimid, oder dehydratisierende, über Stickstoffatome disubstituierte Carbonylverbindungen, z.B. Carbonyldiimidazol, verwenden. Dabei wendet man diese Mittel üblicherweise in Gegenwart eines Lösungsmittels, wie eines gegebenenfalls halogenierten, niederaliphatischen oder aromatischen Kohlenwasserstoffs, z.B. Benzol oder Toluol, oder eines Lösungsmittelgemisches an, wobei geeignete saure Mittel, wie Trifluoressigsäure, gleichzeitig auch als Lösungsmittel verwendet werden können.

Wenn notwendig, kann man ein wasser-adsorbierendes Mittel oder einen Wasserabscheider einsetzen. Man arbeitet unter Kühlen oder Erwärmen und/oder in einer Inertgas-, z.B. Stickstoffatmosphäre.

Verfahren fc) (Ringschluss unter Abspaltung einer Verbindung der Formel $O=P(X_3)(X_4)(X_5)$):

Im Ausgangsmaterial der Formel XI bedeutet jede der Gruppen $X_3$, $X_4$ und $X_5$ in erster Linie gegebenenfalls, z.B. durch veräthertes Hydroxy, wie Niederalkoxy, substituiertes Niederalkyl oder gegebenenfalls, z.B. durch Niederalkyl, Niederalkoxy, Halogen oder Nitro substituiertes Phenyl. Bevorzugt sind Phenylgruppen $X_3$, $X_4$ und/oder $X_5$.

Der Ringschluss erfolgt in einem inerten organischen Lösungsmittel, bei Temperaturen zwischen etwa -20°C und etwa 100°C, üblicherweise in situ nach der oxidativen Bildung der endständigen Oxogruppe aus einer entsprechenden endständigen Hydroxygruppe durch Behandeln mit einem geeigneten Oxidationsmittel, wie einem organischen Sulfoxid, z.B. Dimethylsulfoxid.

Verfahren ga) (Verätherung und Veresterung von 3-Hydroxy):
In einem Ausgangsmaterial der Formel XIII, worin die Gruppe $R_3$ als funktionell abgewandelte Carboxylgruppe vorliegt,und andere, gegebenenfalls vorhandene funktionelle Gruppen vorzugsweise geschützt sind, kann die Hydroxygruppe in an sich bekannter Weise veräthert werden.

Geeignete Verätherungsmittel sind beispielsweise entsprechende Diazoverbindungen, wie gegebenenfalls substituierte Diazoniederalkane, z.B. Diazomethan, Diazoäthan, Diazo-n-butan oder Diphenyldiazomethan. Diese Reagentien werden in Gegenwart eines geeigneten inerten Lösungsmittels,wie eines aliphatischen, cycloaliphatischen oder aromatischen Kohlenwasserstoffs, wie Hexan, Cyclohexan, Benzol oder Toluol, eines halogenierten aliphatischen Kohlenwasserstoffs, z.B.

Methylenchlorid, oder eines Aethers, wie eines Diniederalkyläthers, z.B. Diäthyläther, oder eines cyclischen Aethers, z.B. Tetrahydrofuran oder Dioxan, oder eines Lösungsmittelgemisches, und, je nach Diazoreagens, unter Kühlen, bei Raumtemperatur oder unter leichtem Erwärmen, ferner, wenn notwendig, in einem geschlossenen Gefäss und/oder unter einer Inertgas-, z.B. Stickstoffatmosphäre zur Anwendung gebracht.

Weitere geeignete Verätherungsmittel sind Ester entsprechender Alkohole, in erster Linie solche mit starken anorganischen oder organischen Säuren, wie Mineralsäuren, z.B. Halogenwasserstoffsäuren, wie Chlorwasserstoff-, Bromwasserstoff- oder Jodwasserstoffsäure, ferner Schwefelsäure, oder Halogen-schwefelsäuren, z.B. Fluorschwefelsäure, oder starken organischen Sulfonsäuren, wie gegebenenfalls, z.B. durch Halogen, wie Fluor, substituierten Niederalkansulfonsäuren, oder aromatischen Sulfonsäuren, wie z.B. gegebenenfalls, durch Niederalkyl, wie Methyl, Halogen, wie Brom, und/oder Nitro substituierten Benzolsulfonsäuren, z.B. Methansulfon-, Trifluormethansulfon- oder p-Toluolsulfonsäure. Solche Ester sind u.a. Niederalkylhalogenide, Diniederalkylsulfate, wie Dimethylsulfat, ferner Fluorsulfonsäureester, wie -niederalkylester, z.B. Fluorsulfonsäuremethylester, oder gegebenenfalls Halogen-substituierte Methansulfonsäure-niederalkylester, z.B. Trifluormethansulfonsäuremethylester. Sie werden üblicherweise in Gegenwart eines inerten Lösungsmittels, wie eines gegebenenfalls halogenierten, wie chlorierten, aliphatischen, cycloaliphatischen oder aromatischen Kohlenwasserstoffs, z.B. Methylenchlorid, eines Aethers, wie Dioxan oder Tetrahydrofuran, oder eines Gemisches verwendet. Dabei wendet man vorzugsweise geeignete Kondensationsmittel, wie Alkalimetallcarbonate oder -hydrogencarbonate, z.B. Natrium- oder Kaliumcarbonat oder -hydrogencarbonat (üblicherweise zusammen mit einem Sulfat), oder organische Basen, wie, üblicherweise sterische gehinderte, Triniederalkylamine, z.B. N,N-Diisopropyl-N-äthyl-amin (vorzugsweise zusammen mit Halogensulfonsäure-niederalkylestern oder gegebenenfalls Halogensubstituierten Methansulfonsäure-niederalkylestern) an, wobei unter Kühlen, bei Raumtemperatur oder unter Erwärmen, z.B. bei Temperaturen von etwa -20°C bis etwa 50°C und, wenn notwendig, in einem geschlossenen Gefäss und/oder in einer Inertgas-, z.B. Stickstoffatmosphäre gearbeitet wird.

Durch Phasentransfer-Katalyse (siehe Dehmlow, Angewandte Chemie, Bd. 5, S. 187 (1974) kann die oben beschriebene Verätherungsreaktion wesentlich beschleunigt werden. Als Phasentransfer-Katalysatoren können quartäre Phosphoniumsalze und insbesondere quartäre Ammoniumsalze, wie gegebenenfalls substituierte Tetraalkylammoniumhalogenide, z.B. Tetrabutylammoniumchlorid, -bromid oder -jodid, oder auch Benzyl-triäthylammoniumchlorid, in katalytischen oder bis zu äquimolaren Mengen verwendet werden. Als organische Phase kann irgandeines der mit Wasser nicht mischbaren Lösungsmittel dienen, beispielsweise einer der gegebenenfalls halogenierten, wie chlorierten niederaliphatischen, cycloaliphatischen oder aromatischen Kohlenwasserstoffe, wie Tri- oder Tetrachloräthylen, Tetrachloräthan, Tetrachlorkohlenstoff, Chlorbenzol, Toluol oder Xylol. Die als Kondensationsmittel geeigneten Alkalimetallcarbonate oder -hydrogencarbonate, z.B. Kalium- oder Natriumcarbonat oder -hydrogencarbonat, Alkalimetallphosphate, z.B. Kaliumphosphat, und Alkalimetallhydroxide, z.B. Natriumhydroxid, können bei basenempfindlichen Verbindungen der Reaktonsmischung titrimetrisch, z.B. mittels eines Titrierautomaten, zugesetzt werden, damit der pH-Wert während der Verätherung zwischen etwa 7 und etwa 8.5 bleibt.

Weitere Verätherungsmittel sind geeignete Acetal-Verbindungen, z.B. gem-Di-niederalkoxy-niederalkane, wie 2,2-Dimethoxy-propan, die in Gegenwart von starken organischen Sulfonsäuren, wie p-Toluolsulfonsäure, und eines geeigneten Lösungsmittel, wie eines Diniederalkyl- oder Niederalkylensulfoxyds, z.B. Dimethylsulfoxyd, zur Anwendung gelangen, oder geeignete Orthoester, z.B. Orthoameisensäuretriniederalkylester, z.B. Orthoameisensäure-triäthylester, die in

Gegenwart einer starken Mineralsäure, z.B. Schwefelsäure, oder einer starken organischen Sulfonsäure, wie p-Toluosulfonsäure, und eines geeigneten Lösungsmittels, wie eines Aethers, z.B. Dioxan, verwendet werden.

Weitere Verätherungsmittel sind entsprechende trisubstituierte Oxoniumsalze (sogenannte Meerweinsalze), oder disubstituierte Carbenium- oder Haloniumsalze, worin die Substituenten die veräthernden Reste sind, beispielsweise Triniederalkyloxonium-salze, sowie Diniederalkoxycarbenium- oder Diniederalkylhalonium-salze, insbesondere die entsprechenden Salze mit komplexen, fluorhaltigen Säuren, wie die entsprechenden Tetrafluorborate, Hexafluorphosphate, Hexafluorantimonate oder Hexachlorantimonate. Solche Reagentien sind z.B. Trimethyloxonium- oder Triäthyloxonium-hexafluorantimonat, -hexachlorantimonat, -hexafluorphosphat oder -tetrafluorborat, Dimethoxycarbeniumhexafluorphosphat oder Dimethylbromoniumhexafluorantimonat. Man verwendet diese Verätherungsmittel vorzugsweise in einem inerten Lösungsmittel, wie einem Aether oder einem halogenierten Kohlenwasserstoff, z.B. Diäthyläther, Tetrahydrofuran oder Methylenchlorid, oder in einem Gemisch davon, wenn notwendig, in Gegenwart einer Base, wie einer organischen Base, z.B. eines, vorzugsweise sterisch gehinderten, Triniederalkylamins, z.B. N,N-Diisopropyl-N-äthyl-amin, und unter Kühlen, bei Raumtemperatur oder unter leichtem Erwärmen, z.B. bei etwa -20°C bis etwa 50°C, wenn notwendig, in einem geschlossenen Gefäss und/oder in einer Inertgas-, z.B. Stickstoffatmosphäre.

Weitere Verätherungsmittel sind schliesslich entsprechende 1-substituierte 3-Aryltriazenverbindungen, worin der Substituent den veräthernden Rest, und Aryl vorzugsweise gegebenenfalls substituiertes Phenyl, z.B. Niederalkylphenyl, wie 4-Methylphenyl, bedeutet. Solche Triazenverbindungen sind 3-Aryl-1-niederalkyl-triazene, z.B. 3-(4-Methylphenyl)-1-methyl-triazen, 3-(4-Methyl-phenyl)-1-äthyl-triazen oder 3-(4-methylphenyl)-1-isopropyl-tria-

zen. Diese Reagentien werden üblicherweise in Gegenwart von inerten Lösungsmitteln, wie gegebenenfalls halogenierten Kohlenwasserstoffen oder Aethern, z.B. Benzol, oder Lösungsmittelgemischen, und unter Kühlen, bei Raumtemperatur und vorzugsweise bei erhöhter Temperatur, z.B. bei etwa 20°C bis etwa 100°C, wenn notwendig, in einem geschlossenen Gefäss und/oder in einer Inertgas-, z.B. Stickstoffatmosphäre verwendet.

In einem Ausgangsmaterial der Formel XIII, worin die Gruppe $R_3$ als funktionell abgewandeltes Carboxyl vorliegt, und andere, gegebenenfalls vorhandene funktionelle Gruppen vorzugsweise geschützt sind, kann die Hydroxygruppe in an sich bekannter Weise verestert werden. So kann man die Hydroxygruppe durch Behandeln des Ausgangsmaterials der Formel XIII mit einem, den gewünschten Acylrest einer organischen Carbonsäure einführenden Acylierungsmittel in eine Acyloxygruppe umwandeln. Dabei verwendet man die entsprechende Carbonsäure oder ein reaktionsfähiges Derivat davon, insbesondere ein Anhydrid, inkl. ein gemischtes oder inneres Anhydrid einer solchen Säure. Gemischte Anhydride sind z.B. diejenigen mit Halogenwasserstoffsäuren, d.h. die entsprechenden Säurehalogenide, insbesondere -chloride, ferner mit Cyanwasserstoffsäure, oder dann diejenigen mit geeigneten Kohlensäurehalbderivaten, wie entsprechenden -halbestern (wie die z.B. mit einem Halogen-ameisensäure-niederalkyl-, wie Chlorameisensäure-äthylester oder -isobutylester, gebildeten gemischten Anhydride) oder mit gegebenenfalls substituierten, z.B. Halogen, wie Chlor, enthaltenden Niederalkancarbonsäuren (wie die mit Pivalinsäurechlorid oder Trichloressigsäurechlorid gebildeten gemischten Anhydride). Innere Anhydride sind z.B. diejenigen von organischen Carbonsäuren, d.h. Ketene, wie Keten oder Diketen, oder diejenigen von Carbamin- oder Thiocarbaminsäuren, d.h. Isocyanate oder Isothiocyanate. Weitere reaktonsfähige, als Acylierungsmittel verwendbare Derivate von organischen Carbonsäuren sind aktivierte Ester, wie geeignet substituierte Niederalkyl-, z.B. Cyanmethylester, oder geeignet substituierte Phenyl-, z.B. Pentachlorphenyl- oder 4-Nitrophenylester. Die Veresterung kann, wenn notwendig, in Gegenwart von geeigneten Kondensationsmitteln, bei Verwendung von freien Carbonsäuren z.B. in Gegenwart von Carbodiimidverbindungen, wie Dicyclohexylcarbodiimid, oder Carbonylverbindungen, wie Diimidazolylcarbonyl, und bei Verwendung von reaktonsfähigen Säurederivaten z.B. in Gegenwart von basischen Mitteln, wie Triniederalkylaminen, z.B. Triäthylamin, oder heterocyclischen Basen, z.B. Pyridin, durchgeführt werden. Die Acylierungsreaktion kann in Abwesenheit oder in Gegenwart eines Lösungsmittels oder Lösungsmittelgemisches, unter Kühlen, bei Raumtemperatur oder unter Erwärmen, und, wenn notwendig, in einem geschlossenen Gefäss und/oder in einer Inertgas-, z.B. Stickstoffatmosphäre durchgeführt werden. Geeignete Lösungsmittel sind z.B. gegebenenfalls substituierte, insbesondere gegebenenfalls chlorierte, aliphatische, cycloaliphatische oder aromatische Kohlenwasserstoffe, wie Benzol oder Toluol, wobei man geeignete Veresterungsreagentien, wie Essigsäureanhydrid, auch als Verdünnungsmittel verwenden kann.

Eine durch eine organische Sulfonsäure, z.B. Niederalkansulfonsäure, wie Methansulfonsäure, oder eine aromatische Sulfonsäure, wie p-Toluolsulfonsäure, veresterte Hydroxygruppe kann man vorzugsweise durch Behandeln des Ausgangsmaterials der Formel XIII mit einem reaktionsfähigen Sulfonsäurederivat, wie einem entsprechenden Halogenid, z.B. Chlorid, wenn notwendig, in Gegenwart eines Säure-neutralisierenden basischen Mittels, z.B. einer anorganischen oder organischen Base, z.B. in analoger Weise wie die entsprechenden Ester mit organischen Carbonsäuren, bilden.

Die Ueberführung der freien Hydroxygruppe in einem Ausgangsmaterial der Formel XIII in eine, durch eine Halogenwasserstoffsäure veresterte Hydroxygruppe, d.h. in ein Halogenatom, kann in verschiedenartiger Weise, üblicherweise durch Behandeln mit einem halogenierenden, insbesondere chlorierenden Mittel durchgeführt werden.

Geeignete Halogenierungsmittel sind beispielsweise Halogen-Phosphor-Reagentien, die enolische Hydroxylgruppen durch Halogen ersetzen können, wie Di-halogen-triorganyl-phosphorane, Trihalogendiorganyl-phosphorane oder ein Gemisch bestehend aus einem Triorganylphosphin und einem Tetrahalogenmethan, worin der organische Rest z.B. gegebenenfalls substituiertes, wie Diniederalkylamino enthaltendes Niederalkyl oder vorzugsweise Phenyl sein kann.

Repräsentative Vertreter der genannten Phosphorane sind z.B. Difluor-triphenyl-, Trifluor-diphenyl-, Dichlor-triphenyl-, Trichlordiphenyl, Dibrom-triphenyl- und Tribrom-diphenylphosphoran, worin eine der Phenylgruppen durch ein geeignetes Polymeres, wie einem mit Divinylbenzol vernetzten Polystyrol, oder dann durch Niederalkyl oder Diniederalkylaminoniederalkyl, wie Dimethylaminomethyl, ersetzt sein kann.

Repräsentative Vertreter der genannten Phosphine sind Triäthyl-, Methyl-propyl-phenyl-, Bis-(3-dimethylaminopropyl)-phenyl- und insbesondere Triphenylphosphin, worin eine der Phenylgruppen durch ein Polymeres, wie einem mit Divinylbenzol vernetzten Polystyrol, ersetzt sein kann.

Tetrahalogenmethane sind z.B. Tetrabrom- und insbesondere Tetrachlorkohlenstoff.

Die Reaktion mit den halogenierenden Phosphorreagentien findet in an sich bekannter Weise, z.B. in einem inerten, aprotischen, bevorzugt polaren Lösungsmittel, wie einem chlorierten Kohlenwasserstoff, z.B. Methylenchlorid, Chloroform, Tetrachlorkohlenstoff oder 1,2-Dichloräthan, einem Nitril, wie Acetonitril oder Benzonitril, oder einem N,N-disubstituierten Carbonsäureamid, wie Dimethylformamid oder N,N-Dimethylacetamid, oder Mischungen davon, je nach Reaktivität des eingesetzten Reagens unter Kühlen oder Erwärmen, d.h. bei Temperaturen zwischen etwa -60°C bis zur Rückflusstemperatur des

verwendeten Lösungsmittels, gegebenenfalls in einer Inertgas-, wie Stickstoffatmosphäre, statt. Bei Verwendung von Triniederalkyl- oder Tris-(diniederalkylamino)-phosphinen und Tetrachlorkohlenstoff oder Tetrabromkohlenstoff wird gewöhnlich in einem Temperaturbereich von -60°C bis -20°C gearbeitet. Bei der Umsetzung mit Phosphoranen kann dem Reaktionsmedium zum Abfangen entstehenden Halogenwasserstoffs eine schwache Base, wie Pyridin oder ein N,N-Diniederalkylanilin, z.B. N,N-Dimethylanilin, zugesetzt werden.

Weitere geeignete Halogenierungsmittel sind entsprechende N,N-disubstituierte Halogen-iminiumhalogenid-Verbindungen, insbesondere der Formel $(Alkyl)_2N^{\oplus}=C(Hal)(H \text{ oder } Alkyl)\ Hal^{\ominus}$, worin Hal Chlor bzw. Brom ist. Diese Verbindungen werden üblicherweise in situ hergestellt, indem man ein geeignetes N,N-disubstituiertes Amid, wie Dimethylformamid oder N,N-Dimethylacetamid, mit einem Chlorierungs- bzw. Bromierungsmittel, wie z.B. Phosgen, Carbonyldibromid, Oxalylchlorid, Oxalylbromid, Thionylchlorid, Thionylbromid, Phosphortrichlorid, Phosphoroxychlorid, Phosphortribromid, Phosphoroxybromid oder Phosphorpentachlorid, insbesondere Phosphortrichlorid oder Phosphortribromid, behandelt.

Die obige Reaktion wird üblicherweise in Gegenwart eines Lösungs- oder Verdünnungsmittels durchgeführt, wobei man neben dem normalerweise im Ueberschuss vorliegenden und auch als Lösungsmittel verwendbaren Amid z.B. auch ätherartige Lösungsmittel, z.B. Tetrahydrofuran oder Dioxan, Halogenkohlenwasserstoffe, z.B. Methylenchlorid, oder Sulfoxide, z.B. Dimethylsulfoxid, verwenden kann.

Die Chlorierungs- und Bromierungsmittel werden üblicherweise in Mengen verwendet, die zwei Aequivalenten des Ausgangsmaterials der Formel XIII entsprechen. Die Reaktion kann man z.B. so durchführen, dass man das Chlorierungs- bzw. Bromierungsmittel unter Kühlen zu einer Lösung des Ausgangsmaterials in Dimethylformamid zugibt, worauf man das Reaktionsgemisch während einigen Stunden bei Raumtemperatur stehen lässt.

Die Chlorierung oder Bromierung kann auch so durchgeführt werden, dass man zunächst das Chlorierungs- bzw. Bromierungsmittel mit dem Amid, insbesondere dem Dimethylformamid mischt, so das Halogeniminiumhalogenid-Reagens bildet,und darauf die Lösung des Ausgangsmaterials im Amid, z.B. Dimethylformamid, dem noch ein zusätzliches Lösungsmittel zugegeben werden kann, oder in einem anderen Lösungsmittel, z.B. Tetrahydrofuran, zufügt. Falls notwendig, werden diese Reaktionen in einer Inertgasatmosphäre durchgeführt.

Die Umwandlung der freien Hydroxygruppe in einem Ausgangsmaterial der Formel XIII in Fluor kann z.B. durch dessen Behandlung mit einem Reagens der Formel $F_3S$-Am erfolgen, worin Am eine disubstituierte Aminogruppe darstellt; solche Reagentien sind u.a. von Markovskij et al., Synthesis, Bd. 1973, S. 787,beschrieben worden. Die Gruppe Am steht in erster Linie für Diniederalkylamino, z.B. Dimethylamino, Diäthylamino, Aethyl-methyl-amino, Methyl-propylamino, Di-n-propyl-amino oder Diisopropylamino, Niederalkyl-phenyl-amino,z.B. Methyl-phenyl-amino oder Aethyl-phenyl-amino, Niederalkylenamino, z.B. Pyrrolidino oder Piperidino, Oxaniederalkylenamino, z.B. Morpholino, oder gegebenenfalls Aza-Niederalkyl-substituiertes Azaniederalkylenamino, z.B. 4-Methyl-piperazino.

Die Reaktion wird vorzugsweise in einem geeigneten inerten Lösungsmittel, wie einem gegebenenfalls substituierten Kohlenwasserstoff, z.B. Cyclopentan, Cyclohexan, Benzol, Toluol, Xylol, Chlorbenzol, Dichlorbenzol, Pentan, Hexan, Chloroform, und vor allem Methylenchlorid,oder einem Aether, wie Diäthyläther, Tetrahydrofuran oder vor allem Dioxan, falls notwendig, unter Kühlen oder Erwärmen, z.B. in einem Temperaturbereich von etwa -20°C bis etwa 80°C, vorzugsweise von etwa 0°C bis etwa 30°C, und/oder unter einer Inertgasatmosphäre durchgeführt.

Verfahren gb) (Ringschluss unter Entfernung der Gruppen $X_6$ und $X_7$)
In einem Ausgangsmaterial der Formel XIV liegen die Gruppe $R_3$ als funktionell abgewandelte Carboxylgruppe und andere, gegebenenfalls vorhandene funktionelle Gruppen vorzugsweise in geschützter Form vor.

In einem Ausgangsmaterial der Formel XIV ist $X_7$ Wasserstoff oder Halogen, wie Chlor, Brom oder Jod, und $X_6$ beispielsweise eine Gruppe der Formel $-S-X_6^a$ oder eine Gruppe der Formeln $-SO_2-X_6^b$ oder $-S-SO_2-X_6^b$ .

In der Gruppe der Formel $-S-X_6^a$ ist $X_6^a$ ein gegebenenfalls substituierter aromatischer heterocyclischer Rest mit bis zu 15, vorzugsweise mit bis zu 9 Kohlenstoffatomen, und mindestens einem Ringstickstoffatom und gegebenenfalls einem weiteren Ringheteroatom, wie Sauerstoff- oder Schwefelatom; dieser Reste ist mit einem seiner Ringkohlenstoffatome, das mit einem Ringstickstoffatom durch eine Doppelbindung verbunden ist, an die Thiogruppe -S- gebunden.

Solche Reste $X_6^a$ sind z.B. monocyclische, fünfgliedrige, thiadiaza-, thiatriaza-, oxadiaza- oder oxatriazacyclische Reste aromatischen Charakters, insbesondere aber monocyclische, fünfgliedrige diaza-, oxaza- oder thiazacyclische Reste aromatischen Charakters, oder in erster Linie die entsprechenden benzdiazacyclischen, benzazacyclischen oder benzthiazacyclischen Reste, worin der heterocyclische Teil fünfgliedrig ist und aromatischen Charakter hat, wobei in Resten $X_6^a$ ein substituierbares Ringstickstoffatom z.B. durch Niederalkyl substituiert sein kann. Repräsentativ für solche Gruppen $X_6^a$ sind 1-Methyl-2-imidazolyl, 1,3-Thiazol-2-yl, 1,3,4-Thiadiazol-2-yl, 1,3,4,5-Thiatriazol-2-yl, 2-Oxazolyl, 1,3,4-Oxadiazol-2-yl, 1,3,-4,5-Oxatriazol-2-yl, 2-Chinolinyl, 1-Methyl-2-benzimidazolyl, 2-Benzoazolyl und insbesondere 2-Benthiazolyl.

Die Gruppe $X_6^a$ in einem Rest der Formel $-S-X_6^a$ kann auch den Acylrest einer organischen Carbon- oder Thiocarbonsäure, wie einer gegebenenfalls substituierten, aliphatischen, cycloaliphatischen, araliphatischen oder aromatischen Carbon- oder Thiocarbonsäure mit bis zu 18, vorzugsweise mit bis zu 10, Kohlenstoffatomen darstellen.

Representative Acyl- oder Thioacylreste $X_6^a$ sind beispielsweise Niederalkanoyl, wie Formyl, Acetyl oder Propionyl, Niederthioalkanoyl, z.B. Thioacetyl oder Thiopropionyl, Cycloalkylcarbonyl, z.B. Cyclohexylcarbonyl, Cycloalkylthiocarbonyl, z.B. Cyclohexylthiocarbonyl, Aroyl oder Thioaroyl, z.B. Benzoyl, Thiobenzoyl, Naphthylcarbonyl oder Naphthylthiocarbonyl, heterocyclisch substituiertes Carbonyl oder Thiocarbonyl, wie Pyridylcarbonyl, z.B. 2-, 3- oder 4-Pyridylcarbonyl, Thenoyl, z.B. 2- oder 3-Thenoyl, Furoyl, z.B. 2- oder 3-Furoyl, Pyridylthiocarbonyl, z.B. 2-, 3- oder 4-Pyridylthiocarbonvl, Thiothenoyl, z.B. 2- oder 3-Thiothenoyl, oder Thiofuroyl, z.B. 2- oder 3-Thiofuroyl, oder entsprechende substituierte, wie Niederalkyl, z.B. Methyl, Halogen, z.B. Fluor oder Chlor, Niederalkoxy, z.B. Methoxy, Aryl, z.B. Phenyl, oder Aryloxy, z.B. Phenyloxy, enthaltende Acyl- oder Thioacylgruppen.

In den Gruppen $-SO_2-X_6^b$ und $-S-SO_2-X_6^b$ ist $X_6^b$ ein gegebenenfalls substituierter, insbesondere aliphatischer, cycloaliphatischer, araliphatischer oder aromatischer, Kohlenwasserstoffrest mit bis zu 18, vorzugsweise mit bis zu 10, Kohlenstoffatomen. Geeignete Gruppen $X_6^b$ sind beispielsweise gegebenenfalls substituiertes, wie durch Niederalkoxy, z.B. Methoxy, Halogen, z.B. Fluor, Chlor oder Brom, Aryl, z.B. Phenyl, oder Aryloxy, wie Phenyloxy, mono- oder polysubstituiertes Niederalkyl, wie Methyl, Aethyl oder Butyl, Niederalkenyl-, wie Allyl oder Butenyl, oder Cycloalkyl, wie Cyclopentyl oder Cyclohexyl, oder gegebenenfalls, wie durch Niederalkyl, z.B. Methyl, Niederalkoxy, z.B. Methoxy, Halogen, z.B. Fluor, Chlor oder Brom, Aryl, z.B. Phenyl, Aryloxy, z.B. Phenyloxy, oder Nitro, mono- oder polysubstituiertes

Naphthyl oder insbesondere Phenyl, beispielsweise Phenyl, 2-, 3- oder bevorzugt 4-Tolyl, 2-, 3- oder bevorzugt 4-Methoxyphenyl, 2-, 3- oder 4-Chlorphenyl, 4-Biphenylyl, 4-Phenoxyphenyl, 4-Nitrophenyl oder 1- oder 2-Naphthyl.

In einer Verbindung der Formel XIV kann die Gruppe $R_1$ in trans-(Crotonsäurekonfiguration) oder in cis-Stellung (Isocrotonsäurekonfiguration) zur funktonell abgewandelten Carboxylgruppe $R_3$ stehen.

Der Ringschluss unter Abspaltung von $X_6$ und $X_7$ wird durch Behandeln des Ausgangsmaterials der Formel XIV mit einer geeigneten Base, insbesondere einer starken organischen oder anorganischen Base bewirkt. Hervorzuheben als solche Basen sind bicyclische Amidine, wie Diazabicycloalkene, z.B. 1,5-Diazabicyclo[4.3.0]non-5-en oder 1,5-Diazabicyclo[5.4.0]undec-5-en, substituierte, z.B. durch Niederalkyl mehrfach substituierte Guanidine, wie Tetramethylguanidin, ferner Metallbasen, wie Hydride, Amide oder Alkanolate von Alkalimetallen, insbesondere von Lithium, Natrium oder Kalium, beispielsweise Natriumhydrid, Lithiumdiniederalkylamide, wie Lithiumdiisopropylamid, oder Kalium-niederalkanolate, wie Kalium-tert.-butylat, sowie tertiäre organische Stickstoffbasen, z.B. Triniederalkylamine, wie Triäthylamin.

Die Reaktion wird üblicherweise in einem geeigneten inerten Lösungsmittel durchgeführt, z.B. in einem aliphatischen, cycloaliphatischen oder aromatischen Kohlenwasserstoff, wie Hexan, Cyclohexan, Benzol oder Toluol, einem halogenierten Kohlenwasserstoff, wie Methylenchlorid, einem Aether, wie einem Diniederalkyläther, z.B. Diäthyläther, einem Diniederalkoxyniederalkan, wie Dimethoxyäthan, einen cyclischen Aether, wie Dioxan oder Tetrahydrofuran, oder auch in einem Niederalkanol, z.B. Methanol, Aethanol oder tert.-Butanol, oder in einem Gemisch davon, bei Raumtemperatur oder unter leichtem Erwärmen, z.B. einem Temperaturbereich von etwa 20°C bis etwa 50°C, gewünschtenfalls in einer Inertgas-, wie Stickstoffatmosphäre.

Verfahren h) (Einführung einer Ammoniogruppe): In einem Ausgangsmaterial der Formel XV stellt die Gruppe $X_8$ vorzugsweise eine veresterte Hydroxygruppe dar, insbesondere eine durch eine organische Säure, wie Niederalkancarbonsäure, z.B. Ameisen- oder Essigsäure, ferner eine anorganische Säure, wie Halogenwasserstoffsäure, z.B. Brom- oder Jodwasserstoffsäure,veresterte Hydroxygruppe.

Die Reaktion mit einer tertiären organischen Base, insbesondere einem gegebenenfalls substituierten Pyridin, wird unter neutralen oder schwach sauren Bedingungen, vorzugsweise bei einem pH-Wert von etwa 6,5, in Gegenwart von Wasser und gegebenenfalls in einem mit Wasser mischbaren organischen Lösungsmittel vorgenommen. Die schwachsauren Bedingungen können durch Zugabe einer geeigneten organischen oder anorganischen Säure, beispielsweise Essigsäure, Chlorwasserstoffsäure, Phsophorsäure oder auch Schwefelsäure eingestellt werden. Als organische Lösungsmittel können beispielsweise mit Wasser mischbare Lösungsmittel, wie Niederalkanole, z.B. Methanol oder Aethanol, Niederalkanone, z.B. Aceton, Niederalkancarbonsäureamide, z.B. Dimethylformamid, oder Niederalkancarbonsäurenitrile, z.B. Acetonitril, verwendet werden. Ferner können, z.B. zur Erhöhung der Ausbeuten, dem Reaktionsgemisch geeignete Salze, wie Alkalimetall-, z.B. Natrium- und insbesondere Kaliumsalze, von anorganischen Säuren, wie Halogenwasserstoffsäuren, z.B. Chlorwasserstoff- und insbesondere Jodwasserstoffsäuren, sowie der Thiocyansäure, oder von organischen Säuren, wie Niederalkancarbonsäuren, z.B. Essigsäure, insbesondere Kaliumjodid und Kaliumthiocyanat, zugesetzt werden. Auch Salze von geeigneten Anionenaustauschern mit Säuren, z.B. Essigsäure, z.B. flüssige Ionenaustauscher in Salzform, z.B. Amberlite LA-1 (flüssige sekundäre Amine mit einem Molekulargewicht von 351-393; Oel-löslich und Wasser-unlöslich; mAeq./g = 2,5-2,7, z.B. in Acetatform), können für diesen Zweck verwendet werden.

Ammoniogruppen $R_2$ können vorteilhafterweise unter Verwendung eines Zwischenprodukts der Formel XV, in welchem $X_8$ für eine substituierte, insbesondere für eine aromatische substituierte Carbonylthiogruppe und in erster Linie für die Benzoylthiogruppe steht, eingeführt werden. Ein solches Zwischenprodukt kann man z.B. durch Umsetzen eines Ausgangsmaterials der Formel XV , worin $X_8$ eine veresterte Hydroxygruppe, insbesondere Niederalkanoyloxy, z.B. Acetyloxy, bedeutet, mit einem geeigneten Salz, wie einem Alkalimetall-, z.B. Natriumsalz, einer Thiocarbonsäure, wie einer aromatischen Thiocarbonsäure, z.B. Thiobenzoesäure, erhalten. Das Zwischenprodukt wird dann mit der tertiären Aminbase, insbesondere einer entsprechenden heterocyclischen Base, wie einem gegebenenfalls substituierten Pyridin, umgesetzt, wobei man die gewünschte Ammonioverbindung erhält. Die Reaktion wird üblicherweise in Gegenwart eines geeigneten Entschwefelungsmittels, insbesondere eines Quecksilbersalzes, z.B. Quecksilber-II-perchlorat, und eines geeigneten Lösungs- oder Verdünnungsmittels oder eines Gemisches, wenn notwendig, unter Kühlen oder Erwärmen, in einen geschlossenen Gefäss und/oder in einer Inertgas-, z.B. Stickstoffatmosphäre, durchgeführt.

Verfahren i) (Einführung von Acylmethyl oder gegebenenfalls funktionell abgewandeltem Carboxymethyl):
Das 3-Hydroxy-3-cephem-Ausgangsmaterial der Formel XIII liegt mit der dazu tautomeren 3-Oxo-cepham-Verbindung im Gleichgewicht, wobei letztere durch Reaktion mit dem Ylid laufend aus dem Gleichgewicht entfernt wird.

Im Ylid der Formel XVI ist die Gruppe $X_9^{\oplus}$ eine der bei sog. Wittig-Kondensationsreaktionen gebräuchlichen drei- bzw. zweifach substituierten Phosphonium- oder Phosphonogruppen, insbesondere eine Triaryl-, wie Triphenyl-, oder Triniederalkyl-, wie Tributylphosphoniumgruppe, oder eine Di-niederalkyl-, wie Diäthylphosphonogruppe, wobei Triphenylphosphonium und Diäthylphosphono bevorzugt sind. Für den Fall der disubstituierten Phosphonogruppe umfasst das Symbol $X_9^{\oplus}$ noch das Kation einer starken Base.

In Phosphoniumverbindungen der Formel XVI, die in der isomeren Ylenform auch als Phosphorane, z.B. als Niederalkoxycarbonylmethylen-triphenylphosphorane,vorliegend können, wird die negative Ladung durch die positive geladene Phosphoniumgruppe neutralisiert. Phosphonoverbindungen der Formel XVI, die man in ihrer isomeren Form auch als Phosphonate bezeichnen kann, werden von einem Kation einer starken Base neutralisiert, das je nach Herstellungsweise, beispielsweise ein Alkalimetall-, wie Kalium-, Natrium- oder Lithiumion sein kann. Die Phosphonate werden in dieser Form, d.h. als Salze, in die Reaktion eingesetzt.

Die obige Kondensationsreaktion wird in einem geeigneten inerten Lösungsmittel durchgeführt, beispielsweise in einem gegebenenfalls halogenierten niederaliphatischen, cycloaliphatischen oder aromatischen Kohlenwasserstoff, wie Hexan, Cyclohexan, Benzol, Toluol oder Methylenchlorid, einem Aether, wie einem Diniederalkyläther, z.B. Diäthyläther, einem Diniederalkoxyniederalkan, wie Dimethoxyäthan, einem cyclischen Aether, wie Dioxan oder Tetrahydrofuran, einem N,N-Diniederalkylamid, wie Dimethylformamid, einem Diniederalkylsulfoxid, wie Dimethylsulfoxid, oder auch einem Niederalkanol, z.B. Methanol oder Aethanol,oder in einem Gemisch davon, üblicherweise bei erhöhter Temperatur, wie bei etwa 50°C bis 150°C, bevorzugt bei etwa 60°C bis 100°C, gewünschtenfalls in einer Inertgas-, wie Stickstoffatmosphäre.

Die obige Wittig-Reaktion kann, je nach Ausgangsmaterial und Reaktionsbedingungen, einheitliche Verbindungen der Formel I oder deren einheitliche Isomere, worin $R_1$ einen Exomethylenrest der Teilformel $=CH-R_2$ darstellt, oder Gemische der beiden isomeren Formen liefern. Erhaltene Gemische können in an sich bekannter Weise, z.B. mit Hilfe von geeigneten Trennmethoden, z.B. durch Adsoprtion und fraktionierte Elution, inkl. Chromatographie (Säulen-, Papier- oder

Plattenchromatographie) unter Verwendung von geeigneten Adsorptionsmitteln, wie Silikagel oder Aluminiumoxyd, und Elutionsmitteln, ferner durch fraktioniertes Kristallisieren, Lösungsmittelverteilung, etc. aufgetrennt werden. Entsprechende Verfahrensweisen sind beispielsweise in der DE-OS 2.637.176 beschrieben.

Verfahren j) (Austausch von Y gegen Amine AmH):
In einer Verbindung der Formel XVII ist Y Brom, Jod oder bevorzugt Chlor.

Die Amine Am-H werden in freier Form, d.h. als Ammoniak, Niederalkylamin, wie Methyl-, Aethyl-, Propyl- oder Butylamin, oder Diniederalkylamin, wie Dimethyl-, Diäthyl-, Dipropyl-, Dibutyl-, Methyl-äthyl-, Aethyl-propyl-, Methyl-butyl-, Propyl-butylamin, oder dergleichen, oder in Form von Metallamiden davon, beispielsweise als Alkalimetallamide, wie Lithium-, Natrium- oder Kaliumamide, eingesetzt.

Die Austauschreaktion findet bevorzugt in einem inerten organischen Lösungsmittel, wie Benzol, Toluol, Dimethylformamid, Tetrahydrofuran, oder dergleichen, oder, beim Austausch von Y gegen $NH_2$ auch in flüssigem Ammoniak statt, wobei gegebenenfalls die Säure H-Y abfangende Basen, wie tertiäre Amine, z.B. Aethyl-diisopropylamin, Pyridin, oder dergleichen, zugefügt werden können.

Die Reaktion erfolgt, je nach Reaktionsfähigkeit, bei Zimmertemperatur oder unter Abkühlen oder Erwärmen, zwischen etwa -70° und etwa +100°, bevorzugt zwischen etwa -70° und etwa +50°.

Die erfindungsgemäss erhältlichen Verbindungen der Formel I können in an sich bekannter Weise in andere Verbindungen der Formel I übergeführt werden.

Substituierung einer Hydroxyiminogruppe: In einer Verbindung der Formel I, worin $R_4$ für Wasserstoff steht, und andere gegebenenfalls vorhandene funktionelle Gruppen, insbesondere diejenigen, die in analoger Weise wie die Hydroxyiminogruppe substituiert werden können, vorzugsweise geschützt sind, kann die Hydroxygruppe des unsubstituierten Hydroxyiminorestes in an sich bekannter Weise veräthert werden.

Geeignete Verätherungsmittel sind z.B. die unter Verfahren ga) erwähnten Reagentien, wie Diazoniederalkane, z.B. Diazomethan, Ester von Alkoholen der Formel $R_4$-OH, worin $R_4$ gegebenenfalls substituiertes Niederalkyl oder Cycloalkyl darstellt, mit starken anorganischen oder organischen Säuren, wie Niederalkyl- oder Cycloalkylhalogenide, z.B. -chloride, -bromide oder -iodide, oder Diniederalkylsulfate, ferner Fluorsulfonsäure-niederalkylester, oder gegebenenfalls Halogen-substituierte Methansulfonsäure-niederalkylester, sowie geeignete Acetale, z.B. gem-Di-niederalkoxy-niederalkane, Tri-$R_4$-oxoniumsalze (sogenannte Meerweinsalze), Di-$R_4$-O-Carbeniumsalze oder Di-$R_4$-Haloniumsalze, worin $R_4$ gegebenenfalls substituiertes Niederalkyl oder Cycloalkyl darstellt, wie Triniederalkyloxoniumsalze, sowie Diniederalkoxycarbenium- oder Diniederalkylhaloniumsalze, insbesondere die entsprechenden Salze mit komplexen, fluorhaltigen Säuren, oder 1-$R_4$-3-Aryl-triazenverbindungen, worin $R_4$ gegebenenfalls substituiertes Niederalkyl oder Cycloalkyl und Aryl vorzugsweise einen gegebenenfalls substituierten Phenylrest bedeuten. Diese Verätherungsreagenzien werden in an sich bekannter Weise, z.B. wie oben beschrieben, verwendet, üblicherweise in einem geeigneten Lösungsmittel oder Lösungsmittelgemisch, wenn notwendig, in Gegenwart eines Kondensationsmittels, wie einer Base, unter Kühlen oder Erwärmen, in einem geschlossenen Gefäss unter Druck und/oder in einer Intertgas-, z.B. Stickstoffatmosphäre.

Die Einführung einer Carbonylgruppe -C(=O)-NHR anstelle des Wasserstoffatoms geschieht auf an sich bekannte Weise durch Carbamoylierung. Zu diesem Zwecke wird eine Verbindung der Formel (If), worin alle reaktionsfähigen Gruppen, die nicht carbamoyliert werden sollen, oder die die Reagentien verbrauchen und zerstören würden, in geschützter Form vorliegen, gegebenenfalls stufenweise mit einem Carbamoylierungsmittel behandelt. Geeignete Carbamoylierungsmittel sind reaktionsfähige Derivate der Carbamidsäuren R-NH-C(=O)OH, insbesondere die Isocyanate R-N=C=O oder die gemischten Anhydride, z.B. die Carbamidsäurechloride R-NH-C(=O)Cl, die auch in situ aus Phosgen und einem Amin $R-NH_2$ hergestellt werden können.

Der Umsatz mit den Isocyanaten erfolgt in einem der üblichen inerten Lösungsmittel, beispielsweise einem gegebenenfalls halogenierten Kohlenwasserstoff, z.B. Benzol, Xylol oder Methylenchlorid, einem Aether, z.B. Diäthyläther, Dioxan oder Tetrahydrofuran, oder einem Ester, z.B. Essigsäureäthylester, gegebenenfalls in Gegenwart eines basischen Katalysators, beispielsweise eines tertiären Amins, z.B. Triäthylamin oder Pyridin, bei Raumtemperatur oder etwas erniedrigter oder erhöhter Temperatur, etwa zwischen -20° und +50°.

Zum Umsatz mit einem gemischten Anhydrid der Carbamidsäure wird die Alkoholgruppe mit Vorteil zunächst metallisiert, beispielsweise mit einem Alkalimetallhydrid, z.B. Natrium- oder Lithiumhydrid. Die an der Hydroxygruppe metallierte Verbindung der Formel If) kann auch zunächst mit Phosgen und anschliessend mit dem Amin $R-NH_2$ behandelt werden und auf diese Art stufenweise carbamoyliert werden. Diese Reaktionen finden ebenfalls in einem inerten Lösungsmittel und bei den oben angegebenen Temperaturen statt.

Substituierung einer Aminogruppe: Eine gegebenenfalls monosubstituierte Aminogruppe Am in einer erfindungsgemäss erhältlichen Verbindung der Formel I, worin andere gegebenenfalls vorhandene funktionelle Gruppen, insbesondere diejenigen, die analog wie eine Aminogruppe substituiert werden können, vorzugsweise geschützt sind, kann in an sich bekannter Weise, z.B. durch Behandeln mit einem reaktionsfähigen Ester eines Niederalkanols, wie einem Niederalkylhalogenid, z.B. -chlorid, -bromid oder -jodid, vorzugsweise in Gegenwart eines Lösungsmittels, oder, unter Einführung von Methyl, mit Formaldehyd in Gegenwart von Ameisensäure substituiert werden. Ferner kann man eine gegebenenfalls monosubstituierte Aminogruppe Am mit einem geeigneten Derivat einer Niederalkancarbonsäure, wie einem entsprechenden Halogenid, z.B. Chlorid, acylieren,und in einer so erhältlichen Niederalkanoylamino-Verbindung die Carbonylgruppe, z.B. durch Behandeln mit einem geeigneten Hydridreduktionsreagens, wie Diboran,zu einer Methylengruppe reduzieren. Die Substitutionsreaktion wird in an sich bekannter Weise, bevorzugt in Gegenwart eines Lösungsmittels, und wenn notwendig, unter Kühlen oder Erwärmen und/oder in einer Inertgasatmosphäre durchgeführt.

Ersatz einer veresterten Hydroxygruppe $R_1$ durch Wasserstoff: Eine gegen Wasserstoff austauschbare veresterte Hydroxygruppe $R_1$ in einer Verbindung der Formel I ist vorzugsweise durch eine starke organische oder anorganische Säure, wie eine starke Mineralsäure, z.B. Halogenwasserstoffsäure, wie Chlorwasserstoffsäure, oder eine starke organische Sulfonsäure, wie eine entsprechende niederaliphatische oder aromatische Sulfonsäure, z.B. Methansulfonsäure oder p-Toluolsulfonsäure, veresterte Hydroxygruppe. So stellt $R_1$ z.B. Halogen, wie Chlor, Niederalkylsulfonyloxy, z.B. Methylsulfonyloxy oder Aethylsulfonyloxy, oder Arylsulfonyloxy, z.B. p-Toluolsulfonyloxy, dar.

Der Ersatz von verestertem Hydroxy $R_1$ in einer Verbindung der Formel I durch Wasserstoff kann entweder in zwei Stufen durch Reduktion der 3,4-Doppelbindung im 3-Cephemring und anschliessende Abspaltung einer Säure der Formel $R_1$-H, oder einstufig durch unmittelbare reduktive Abspaltung von $R_1$ erfolgen.

Beim zweistufigen Austausch von $R_1$ gegen Wasserstoff erfolgt die Reduktion der 3,4-Doppelbindung in Verbindungen der Formel I z.B. durch Behandeln mit geeigneten Hydrid-Reduktionsmitteln. Solche sind in erster Linie komplexe Metallhydride, vorzugsweise entsprechende Borhydride, wie Diboran oder Alkalimetallborhydride, z.B. Natriumborhydrid oder Lithiumborhydrid, ferner Zinkborhydrid, sowie organische Alkalimetallaluminiumhydride, wie Tri-niederalkoxy-alkalimetall-aluminiumhydride, z.B. Tri-(tert.-butyloxy)-lithiumaluminiumhydrid, die man in an sich bekannter Weise, z.B. wie oben unter Verfahren fb) beschrieben, verwendet.

Falls die obige Reduktion in Gegenwart geeigneter Säure-abspaltender Mittel durchgeführt wird, kann die erwünschte 3-unsubstituierte 3-Cephem-Verbindung der Formel I unmittelbar erhalten werden. Es kann aber auch zunächst eine 3-$R_1$-Cepham-Verbindung entstehen, die entweder isoliert oder in situ weiterverarbeitet werden kann.

In der zweiten Verfahrensstufe erfolgt dann die Abspaltung der Elemente einer Säure der Formel $R_1$-H aus der erhaltenen 3-$R_1$-Cepham-Verbindung, wobei man diese vorzugsweise mit geeigneten Säure-abspaltenden Mitteln behandelt.

Die einstufige reduktive Abspaltung einer veresterten Hydroxygruppe $R_1$ aus einer Verbindung der Formel I gelingt am besten, wenn $R_1$ Halogen, wie Chlor, oder eine durch eine organische Sulfonsäure, wie eine niederaliphatische oder aromatische Sulfonsäure, z.B. Methansulfonsäure oder p-Toluolsulfonsäure, veresterte Hydroxygruppe ist. In diesem Fall reduziert man vorzugsweise durch Behandeln mit metallischen Reduktionsmitteln ("nascierendem Wasserstoff"), z.B. mit

Aluminium oder insbesondere Zink, oder mit reduzierenden Metallverbindungen, z.B. entsprechenden Metalllegierungen, Amalgamen wie Aluminiumamalgam oder Salzen. Diese wendet man üblicherweise in Gegenwart von schwachen Protonendonatoren, wie Wasser, Alkohol (z.B. Methanol oder Aethanol) oder einer niederaliphatischen Carbonsäure, z.B. Essigsäure oder Ameisensäure an. Mit Zink reduziert man beispielsweise in Gegenwart von Essigsäure oder Ameisensäure, mit einem Amalgam z.B. in Gegenwart eines wasserhaltigen, inerten, organischen Lösungsmittel, wie eines Aethers.

Gewisse veresterte Hydroxygruppen $R_1$, insbesondere organisches Sulfonyloxy, z.B. Methylsulfonyloxygruppen, lassen sich in Form einer Säure der Formel $R_1$-H auch durch Adsorption, z.B. an Silikagel, Aluminiumoxyd, etc., und Elution (Chromatographie), abspalten.

Bevorzugt wird in einer Verbindung der Formel I als veresterte Hydroxygruppe $R_1$ Halogen, z.B. Chlor oder Brom, oder organisches Sulfonyloxy, z.B. p-Toluolsulfonyloxy oder Methansulfonyloxy, die z.B. durch Behandeln mit Zink in Gegenwart einer Säure, wie Eisessig oder Ameisensäure, abgespalten werden.

Die Abspaltung von einer Säure der Formel $R_1$-H wird üblicherweise in Gegenwart eines Lösungsmittels durchgeführt. Bei Anwendung von anorganischen Basen kann man dabei in Wasser, einem wasserlöslichen organischen Lösungsmittel, wie Aceton oder Dioxan, oder wässrigen Gemischen davon und bei einem pH von höchstens 9, bei Verwendung von Aminen vorzugsweise auch in einem gegebenenfalls halogenierten, wie chlorierten, niederaliphatische, cycloaliphatischen oder aromatischen Kohlenwasserstoff, wie Methylenchlorid, einem Niederalkanon, z.B. Aceton, oder Aether, z.B. Tetrahydrofuran oder Dioxan, oder einem entsprechenden Lösungsmittelgemisch, inkl. einem wässrigen Gemisch, wenn notwendig unter Kühlen oder Erwärmen und/oder in einer Inertgas-, z.B. Stickstoffatmosphäre, arbeiten.

Austausch von Sulfonyloxy durch Halogen: Eine Sulfonyloxygruppe $R_1$ in einer Verbindung der Formel I, in erster Linie Niederalkylsulfonyloxy, insbesondere Methylsulfonyloxy, ferner Arylsulfonyloxy, worin Aryl vorzugsweise gegebenenfalls, z.B. durch Niederalkyl, wie Methyl, Halogen, z.B. Brom, oder Nitro substituiertes Phenyl, wie 4-Methylphenylsulfonyloxy, kann in ein Halogen-, insbesondere Fluoratom $R_1$ übergeführt werden. So kann man den Austausch einer organischen Sulfonyloxygruppe durch Fluor mittels Behandeln mit einem anorganischen Fluorid in Gegenwart eines Kronenäthers vornehmen.

Ein anorganisches Fluorid ist in erster Linie ein Metallfluorid, insbesondere ein Alkalimetallfluorid, z.B. Natriumfluorid, oder ein Schwermetallfluorid, z.B. Silberfluorid.

Die zusammen mit dem anorganischen Fluorid verwendeten Kronenäther sind gegebenenfalls substituierte 18-Kronen-6-äther, wie der Dicyclohexyl-18-kronen-6-äther.

Die Reaktion wird in Gegenwart eines inerten Lösungsmittels, insbesondere eines Nitrils, z.B. Acetonitril oder Propionitril, oder eines Nitroniederalkans, z.B. Nitromethan oder Nitroäthan, unter im wesentlichen wasserfreien Bedingungen und, falls notwendig, unter Kühlen, z.B. in einem Temperaturbereich von etwa -20°C bis etwa 25°C, vorzugsweise bei etwa Raumtemperatur, und/oder in einer Inertgasatmosphäre durchgeführt.

Ueberführen von verestertem oder veräthertem Hydroxy in veräthertes Mercapto:

In einer erhaltenen Verbindung der Formel I, worin gegebenenfalls vorhandene funktionelle Gruppen vorzugsweise geschützt sind, kann eine durch veräthertes Mercapto ersetzbare veresterte oder verätherte Hydroxygruppe $R_1$ durch Behandeln mit einem Thiol in eine solche übergeführt werden.

Eine durch ein Thiol substituierbare veresterte Hydroxygruppe $R_1$ ist durch eine geeignete anorganische oder organische Säure, wie durch eine Halogen-, z.B. Chlorwasserstoffsäure, eine Schwefel- oder Phosphor- enthaltende Säure, wie eine Schwefel- oder Sulfonsäure, eine Phosphor-, Phosphon- oder Phosphinsäure, oder eine Carbonsäure verestertes Hydroxy. Eine reaktionsfähige verätherte Hydroxygruppe $R_1$ ist durch einen gegebenenfalls substituierten Kohlenwasserstoffrest veräthert, beispielsweise durch einen aliphatischen, cycloaliphatischen oder araliphatischen Kohlenwasserstoffrest mit z.B. bis zu 15 C-Atomen, wie Niederalkyl, z.B. Methyl oder Aethyl, oder Phenylniederalkyl, wie Benzyl. Vorzugsweise ist der gegen veräthertes Mercapto austauschbare Rest $R_1$ Chlor, Brom, Niederalkansulfonyloxy, wie Methan- oder Aethansulfonyloxy, oder Arylsulfonyloxy, wie Benzol-, p-Toluol-, p-Chlorbenzol- oder p-Nitrobenzolsulfonyloxy.

Die Substitution einer solchen Gruppe $R_1$ durch die verätherte Mercaptogruppe findet in an sich bekannter Weise, d.h. unter Kühlen, bei Raumtemperatur oder unter Erwärmen, z.B. in einem Temperaturbereich zwischen etwa 0°C und etwa 40°C, in einem geeigneten inerten Lösungsmittel und vorzugsweise in Gegenwart einer Base statt. Geeignete Basen sind insbesondere sterisch gehinderte Amine, wie entsprechende Triniederalkylamine, z.B. N,N-Diisopropyl-N-äthylamin, bicylische Amidine, wie Diazabicycloalkane, z.B. 1,5-Diazabicyclo-[4.3.0]non-5-en oder 1,5-Diazabicyclo[5.4.0]undec-5-en, sowie Alkalimetallhydride, -amide oder -niederalkanolate, wie Natriumhydrid, Natrium- oder Lithiumamid, Natriumäthylat oder Kalium-tert.-butylat. Die Reaktion kann, wenn notwendig, in einer Inertgas-, wie Stickstoffatmosphäre, und/oder in einem geschlossenen Gefäss unter Druck durchgeführt werden.

Falls $R_1$ in einer Verbindung der Formel I eine verätherte Hydroxygruppe ist, findet unter den basischen Bedingungen zunächst eine Addition des Thiols an die Doppelbindung statt, worauf der

Alkohol $H-R_1$ danach abgespalten werden muss. Die Abspaltung des Alkohols $H-R_1$ findet in Gegenwart einer Säure, in An- oder Abwesenheit eines geeigneten, inerten Lösungsmittels und unter Kühlen, bei Raumtemperatur oder unter Erwärmen, d.h. bei Temperaturen zwischen etwa -70°C und etwa +100°C, bevorzugt zwischen etwa 5°C und etwa 40° C, statt. Zur Abspaltung des Alkohols $H-R_1$ geeignete Säuren sind starke organische Protonensäure, insbesondere Mineralsäuren, wie Salzsäure, ferner organische Sulfonsäure, wie Niederalkansulfonsäuren, z.B. Methansulfonsäure, oder aromatische Sulfonsäuren, z.B. Benzol- oder Toluolsulfonsäure, ferner halogenierte Niederalkancarbonsäuren, wie Trifluor- oder Trichloressigsäure, oder auch Ameisensäure.

Veresterung einer freien Carboxylgruppe: Die Umwandlung von freiem Carboxyl, in erster Linie einer entsprechenden Gruppe $R_3$, in einer Verbindung der Formel I in verestertes Carboxyl, insbesondere in eine veresterte Carboxylgruppe, die unter physiologischen Bedingungen spaltbar ist, erfolgt nach an sich bekannten Veresterungsmethoden, beispielsweise indem man eine Verbindung der Formel I, worin andere, gegebenenfalls vorhandene funktionelle Gruppen gegebenenfalls in geschützter Form vorliegen, oder ein reaktionsfähiges funktionelles Carboxyderivat, inkl. ein Salz davon, mit einem entsprechenden Alkohol oder einem reaktionsfähigen funktionellen Derivat davon, umsetzt.

Die Veresterung von freiem Carboxyl mit dem gewünschten Alkohol wird in Gegenwart eines geeigneten Kondensationsmittels durchgeführt. Uebliche Kondensationsmittel sind z.B. Carbodiimide, beispielsweise N,N'-Diäthyl-, N,N'-Dipropyl-, N,N'-Dicyclohexyl- oder N-Aethyl-N'-(3-dimethylaminopropyl)-carbodiimid, geeignete Carbonylverbindungen, beispielsweise Carbonyldiimidazol, oder 1,2-Oxazoliumverbindungen, z.B 2-Aethyl-5-phenyl-1,2-oxazolium-3'-sulfonat und 2-tert.-Butyl-5-methyl-isoxazolium-perchlorat, oder eine geeignete Acylaminoverbindung, z.B. 2-Aethoxy-1-äthoxycarbonyl-1,2-dihydrochinolin. Die

Kondensationsreaktion wird vorzugsweise in einem wasserfreien Reaktionsmedium, vorzugsweise in Gegenwart eines Lösungs- oder Verdünnungsmittels, z.B. Methylenchlorid, Dimethylformamid, Acetonitril oder Tetrahydrofuran und, wenn notwendig, unter Kühlen oder Erwärmen und/oder in einer Inertgasatmosphäre durchgeführt.

Geeignete reaktionsfähige funktionelle Derivate der zu veresternden Carboxylverbindungen der Formel I sind z.B. Anhydride, insbesondere gemischte Anhydride, und aktivierte Ester.

Gemischte Anhydride sind z.B. diejenigen mit anorganischen Säuren, wie Halogenwasserstoffsäuren, d.h. die entsprechenden Säurehalogenide, z.B. -chloride oder -bromide, ferner Stickstoffwasserstoffsäure, d.h. die entsprechenden Säureazide, sowie phosphorhaltigen Säuren, z.B. Phosphorsäure, Diäthylphosphorsäure oder phosphoriger Säure, oder schwefelhaltigen Säuren, z.B. Schwefelsäure, oder Cyanwasserstoffsäure. Gemischte Anhydride sind weiter z.B. diejenigen mit organischen Carbonsäuren, wie mit gegebenenfalls, z.B. durch Halogen, wie Fluro oder Chlor, substituierten Niederalkancarbonsäuren, z.B. Pivalinsäure oder Trichloressigsäure, oder mit Halbestern, insbesondere Niederalkylhalbestern der Kohlensäure, wie Aethyl- oder Isobutylhalbestern der Kohlensäure, oder mit organischen, insbesondere aliphatischen oder aromatischen, Sulfonsäure, z.B. p-Toluolsulfonsäure.

Zur Reaktion mit dem Alkohol geeignete aktivierte Ester sind z.B. Ester mit vinylogen Alkoholen (d.h. Enolen), wie vinylogen Niederalkenolen, oder Iminomethylesterhalogeniden, wie Dimethyliminomethylesterchlorid (hergestellt aus der Carbonsäure und Dimethylchlormethyliden-iminium-chlorid der Formel $[(CH_3)_2N{=}CHCl]^{\oplus}Cl^{\ominus}$), oder Arylester, wie Pentachlorphenyl-, 4-Nitrophenyl- oder 2,3-Dinitrophenylester, heteroaromatische Ester, wie Benztriazol-, c.B. 1-Benztriazolester, oder Diacyliminoester, wie Succinylimino- oder Phthaliminoester.

Die Acylierung mit einem solchen Säurederivat, wie einem Anhydrid, insbesondere mit einem Säurehalogenid wird bevorzugt in Anwesenheit eines säurebindenden Mittels, beispielsweise einer organischen Base, wie eines organischen Amins, z.B. eines tertiären Amins, wie Triniederalkylamin, z.B. Trimethylamin, Triäthylamin oder Aethyl-diisopropylamin, oder N,N-Diniederalkyl-anilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder eines Oxirans, beispielsweise eines niederen 1,2-Alkylenoxids, wie Aethylenoxid oder Propylenoxid, durchgeführt.

Ein reaktionsfähiges funktionelles Derivat des veresternden Alkohols ist in erster Linie ein entsprechender Ester, vorzugsweise mit einer starken anorganischen oder organischen Säure und stellt insbesondere ein entsprechendes Halogenid, z.B. Chlorid, Bromid oder Jodid, oder eine entsprechende Niederalkyl- oder Aryl-, wie Methyl- oder 4-Methylphenylsulfonyloxyverbindung dar.

Ein solcher reaktionsfähiger Ester eines Alkohols kann mit der freien Carboxylverbindung der Formel I oder einem Salz, wie einen Alkalimetall- oder Ammoniumsalz davon umgesetzt werden, wobei man bei Verwendung der freien Säure vorzugsweise in Gegenwart eines säurebindenden Mittels arbeitet.

Die obigen Veresterungsreaktionen werden in einem inerten, üblicherweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essigsäureäthylester, oder

einem Nitril, z.B. Acetonitril, oder Mischungen davon, wenn notwendig, unter Kühlen oder Erwärmen, z.B. in einem Temperaturbereich von etwa bei -40°C bis etwa +100°C, vorzugsweise bei etwa -10°C bis etwa +40°C, und/oder in einer Inertgas-, z.B. Stickstoffatmosphäre.

Ferner kann das Säurederivat, wenn erwünscht, in situ gebildet werden. So erhält man z.B. ein gemischtes Anhydrid durch Behandeln der Carbonsäureverbindung mit entsprechend geschützten funktionellen Gruppen oder eines geeigneten Salzes davon, wie eines Ammoniumsalzes, z.B. mit einem organischem Amin, wie Piperidin oder 4-Methylmorpholin, oder eines Metall-, z.B. Alkalimetallsalzes mit einem geeigneten Säurederivat, wie dem entsprechenden Säurehalogenid einer gegebenenfalls substituierten Niederalkancarbonsäure, z.B. Trichloracetylchlorid, mit einem Halbester eines Kohlensäure-halbhalogenids, z.B. Chlorameisensäureäthylester oder -isobutylester, oder mit einem Halogenid einer Diniederalkylphosphorsäure, z.B. Diäthylphosphorbromidat, und verwendet das so erhältliche gemischte Anhydrid ohne Isolierung.

Bildung des 1-Oxids: Eine Verbindung der Formel I, worin n für 0 steht, kann durch Oxidation, z.B. nach einer der unter Verfahren b) beschriebenen Oxidationsmethoden, in eine Verbindung der Formel I, worin n für 1 steht, übergeführt werden.

Reduktion des 1-Oxids: Eine Verbindung der Formel I, worin n für 1 steht, kann durch Reduktion, z.B. nach einer der unter Verfahren b) beschriebenen Reduktionsmethoden, in eine Verbindung der Formel I, worin n für 0 steht, übergeführt werden.

Abspaltung von Schutzgruppen: In einer erhaltenen Verbindung der Formel I, worin eine oder mehrere funktionelle Gruppe geschützt sind, können diese, z.B. geschützte Carboxyl-, Amino-, Hydroxy- und/oder Sulfogruppen, in an sich bekannter Weise, mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt werden.

So kann man tert.-Niederalkoxycarbonyl oder in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B. durch Behandeln mit einer geeigneten Säure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, freigesetzt werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlor-mandelsäure oder Weinsäure, oder eines Alkohols oder Thiols, wobei man vorzugsweise Wasser zugibt, in freies Carboxyl überführen. Durch Behandeln mit einem reduzierenden Metall oder Metallsalz, wie oben beschrieben, kann man auch 2-Halogen-niederalkoxycarbonyl (gegebenenfalls nach Umwandlung einer 2-Brom-niederalkoxycarbonylgruppe in eine entsprechende 2-Jod-niederalkoxycarbonylgruppe) oder Aroylmethoxycarbonyl in freies Carboxyl umwandeln, wobei Aroylmethoxycarbonyl ebenfalls durch Behandeln mit einem nucleophilen, vorzugsweise salzbildenden Reagens, wie Natriumthiophenolat oder Natriumjodid, gespalten werden kann. Substituiertes 2-Silyläthoxycarbonyl kann auch durch Behandeln mit einem, das Fluoridanion liefernden Salz der Fluorwasserstoffsäure, wie einem Alkalimetallfluorid, z.B. Natrium- oder Kaliumfluorid, in Anwesenheit eines macrocyclischen Polyäthers ("Kronenäther"), oder

mit einem Fluorid einer organischen quaternären Base, wie Tetra-niederalkylammoniumfluorid oder Triniederalkyl-aryl-ammoniumfluorid, z.B. Tetraäthylammoniumfluorid oder Tetrabutylammoniumfluorid, in Gegenwart eines aprotischen polaren Lösungsmittels, wie Dimethylsulfoxid oder N,N-Dimethylacetamid, in freies Carboxyl übergeführt werden. Mit einer organischen Silyl- oder Stannylgruppe, wie Triniederalkylsilyl oder -stannyl, z.B. Trimethylsilyl, verestertes Carboxyl kann in üblicherweise solvolytisch, z.B. durch Behandeln mit Wasser, einem Alkohol oder Säure freigesetzt werden.

Eine geschützte Aminogruppe, z.B. Am, setzt man in an sich bekannter und je nach Art der Schutzgruppen in verschiedenartiger Weise, vorzugsweise mittels Solvolyse oder Reduktion, frei. 2-Halogen-niederalkoxycarbonylamino (gegebenenfalls nach Umwandlung einer 2-Brom-niederalkoxycarbonylaminogruppe in eine 2-Jod-niederalkoxycarbonylaminogruppe), Aroylmethoxycarbonylamino oder 4-Nitrobenzyloxycarbonylamino kann z.B. durch Behandeln mit einem geeigneten chemischen Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagens, wie Natriumthiophenolat, und 4-Nitro-benzyloxycarbonylamino auch durch Behandeln mit einem Alkalimetall-, z.B. Natriumdithionit, gespalten werden. Gegebenenfalls substituiertes Diphenylmethoxycarbonylamino, tert.-Niederalkoxycarbonylamino oder 2-trisubstituiertes Silyläthoxycarbonylamino kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino, Formylamino oder 2-Acyl-niederalk-1-en-1-yl-amino, z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, und

eine mit einer organischen Silyl- oder Stannylgruppe geschützte Aminogruppe z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat, des Thioharnstoffs und anschliessende Solvolyse, wie Alkoholyse oder Hydrolyse, des entstandenen Kondensationsprodukts freigesetzt werden. Eine durch 2-substituiertes Silyläthoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxylgruppe angegeben, in die freie Aminogruppe übergeführt werden. Eine Phosphor-, Phosphon- oder Phosphin-amidogruppe kann z.B. durch Behandeln mit einer Phosphorhaltigen Säure, wie einer Phosphor-,Phosphon- oder Phosphinsäure, z.B. Orthophosphorsäure oder Polyphosphorsäure, einem sauren Ester, z.B. Monomethyl-, Monoäthyl-, Dimethyl- oder Diäthylphosphat oder Monomethylphosphonsäure, oder einem Anhydrid davon, wie Phosphorpentoxid, in die freie Aminogruppe übergeführt werden.

Ein in Form einer Azidogruppe geschütztes Amino wird z.B. durch Reduktion in freies Amino übergeführt, beispielsweise durch katalytische Hydrierung mit Wasserstoff in Gegenwart eines Hydrierkatalysators, wie Platinoxid, Palladium oder Raney-Nickel, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20°C bis 25°C, oder auch unter Kühlen oder Erwärmen, durchgeführt.

Eine durch eine geeignete Acylgruppe, eine organische Silyl- oder Stannylgruppe oder durch gegebenenfalls substituiertes 1-Phenylniederalkyl geschützte Hydroxygruppe wird wie eine entsprechend geschützte Aminogruppe freigesetzt. Eine durch 2,2-Dichloracetyl geschützte Hydroxygruppe wird z.B. durch basische Hydrolyse, während eine durch tert.-Niederalkyl oder durch einen 2-oxa- oder 2-thia-aliphatischen oder -cycloaliphatischen Kohlenwasserstoffrest verätherte Hydroxygruppe durch Acidolyse, z.B. durch Behandeln mit einer Mineralsäure oder einer starken Carbonsäure, z.B. Trifluoressigsäure, freigesetzt.

Eine geschützte, insbesondere veresterte Sulfogruppe wird analog einer geschützten Carboxylgruppe freigesetzt.

Die beschriebenen Spaltungsreaktionen werden unter an sich bekannten Bedingungen durchgeführt, wenn notwendig unter Kühlen oder Erwärmen, in einem geschlossenen Gefäss und/oder in einer Inertgas-, z.B. Stickstoffatmosphäre.

Bevorzugt werden beim Vorhandensein von mehreren geschützten funktionellen Gruppen die Schutzgruppen so gewählt, dass gleichzeitig mehr als eine solche Gruppe abgespalten werden kann, beispielsweise acidolytisch, wie durch Behandeln mit Trifluoressigsäure oder Ameisensäure, oder reduktiv, wie durch Behandeln mit Zink und Essigsäure, oder mit Wasserstoff und einem Hydrierkatalysator, wie einem Palladium/Kohle/Katalysator.

<u>Salzbildung:</u> Salze von Verbindungen der Formel I mit salzbildenden Gruppen können in an sich bekannter Weise hergestellt werden. So kann man Salze von Verbindungen der Formel I mit sauren Gruppen z.B. durch Behandeln mit Metallverbindungen, wie Alkalimetallsalzen von geeigneten organischen Carbonsäuren, z.B. dem Natriumsalz der α-Aethyl-capronsäure, oder mit anorganischen Alkali- oder Erdalkalimetallsalzen, z.B. Natriumhydrogencarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man

in üblicher Weise, z.B. durch Behandeln mit einer Säure oder einem geeigneten Anionenaustauschreagens. Innere Salze von Verbindungen der Formel I, welche z.B. eine freie Carboxylgruppe enthalten, können z.B. durch Neutralisieren von Salzen, wie Säureadditionssalzen, auf den isoelektrischen Punkt, z.B. mit schwachen Basen, oder durch Behandeln mit flüssigen Ionenaustauschern gebildet werden.

Salze können in üblicher Weise in die freien Verbindungen übergeführt werden, Metall- und Ammoniumsalze z.B. durch Behandeln mit geeigneten Säuren, und Säureadditionssalze z.B. durch Behandeln mit einem geeigneten basischen Mittel.

Gemische von Isomeren können in an sich bekannter Weise, z.B. durch fraktionierte Kristallisation, Chromatographie, etc. in die einzelnen Isomeren aufgetrennt werden.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder während der Reaktion gebildet werden.

Vorzugsweise werden solche Ausgangsstoffe verwendet und die Reaktionsbedingungen so gewählt, dass man zu den vorstehend als besonders bevorzugt aufgeführten Verbindungen gelangt.

Ausgangsstoffe: Die im Verfahren zur Herstellung der Verbindungen der vorliegenden Erfindung verwendeten Ausgangsstoffe sind bekannt oder, falls neu, können in an sich bekannter Weise hergestellt werden.

Die Ausgangsverbindungen vom Typ der Formel II, sowie entsprechende Verbindungen mit geschützten funktionellen Gruppen sind bekannt.

Die zur Einführung eines Acylrests einer Säure der Formel III verwendeten Ausgangsstoffe können in an sich bekannter Weise hergestellt werden. Die Herstellungsweise wird anhand des folgenden Reaktionsschemas zur Herstellung einer 2-(5-Amino-1,2,4-thiadiazol-3-yl)-2-methoximino-essigsäureverbindung illustriert:

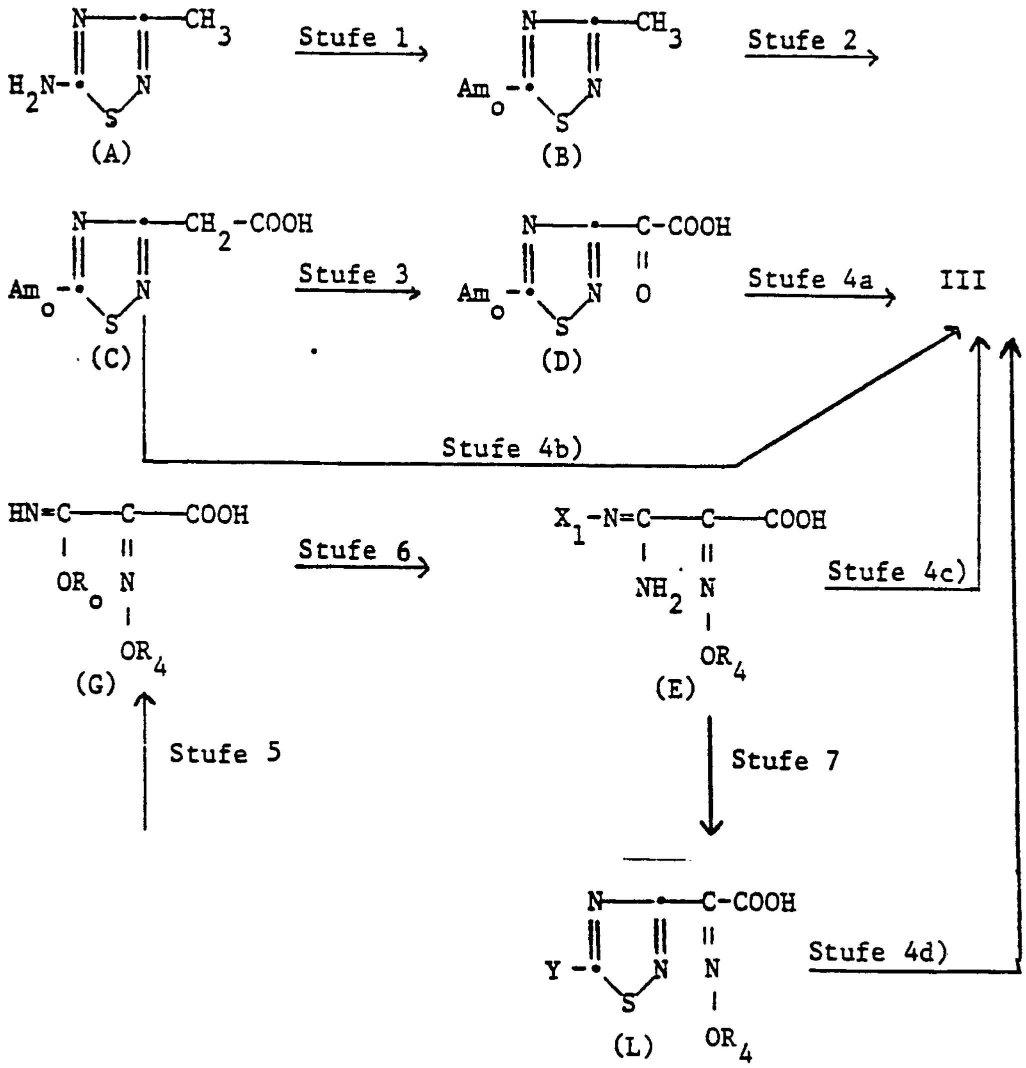


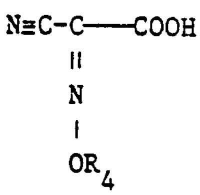

(F)

($Am_o$ = geschützte Aminogruppe; $R_o$ = Niederalkyl; $X_1$ = Wasserstoff oder Halogen)

Stufe 1: In einer Verbindung der Formel A wird die Aminogruppe nach einem der üblichen Verfahren, z.B. Acylieren, Silylieren, etc., in eine geschützte oder durch Alkylierung in eine tertiäre Aminogruppe $Am_o$ übergeführt. Eine geschützte Aminogruppe $Am_o$ ist z.B. eine der unter Am angeführten geschützten Aminogruppen, insbesondere eine der genannten Acylaminogruppen, wie z.B. tert.-Butoxycarbonylamino oder N-Methyl-tert.-butoxycarbonylamino. Eine tertiäre Aminogruppe $Am_o$ ist z.B. eine der genannten Diniederalkylaminogruppen. Bei der Alkylierung kann die Alkylgruppe gegebenenfalls zunächst an das Ringstickstoffatom in 4-Stellung gehen, worauf durch Erhitzen auf etwa 100° eine Umalkylierung in die exo-Aminogruppe auftritt. Eine solche Alkylierung und Umlagerung tritt beispielsweise auch beim Methylieren von 5-Amino-3-methyl-1,2,4-thiadiazol auf.

Stufe 2: Eine Verbindung der Formel C wird erhalten, indem man eine Verbindung der Formel B carboxyliert. Die Carboxylierung erfolgt in an sich bekannter Weise, indem man eine Verbindung der Formel B metalliert und mit Kohlendioxid behandelt. Als Metallierungsmittel kommen insbesondere Organolithiumverbindungen, wie Niederalkyllithium, z.B. Methyl-, Aethyl- oder Butyllithium, Aryllithium, z.B. Phenyllithium, Lithiumamide, wie Lithiumdiniederalkylamide, z.B. Lithiumdiisopropylamid, in Frage. Die Metallierung wird in einem inerten Lösungsmittel, wie einem Aether, z.B. Diäthyläther, Dioxan oder Tetrahydrofuran, oder einem Kohlenwasserstoff, z.B. Hexan oder Benzol, oder einem tertiären Amin oder Diamin, z.B. Tetramethyläthylendiamin , bei erniedrigten Temperaturen, z.B. bei etwa 0°C bis etwa -80°C, durchgeführt. Anschliessend wird bei der gleichen Temperatur Kohlendioxid eingeleitet oder das Metallierungsgemisch wird auf Trockeneis gegossen. Wenn man statt Kohlendioxid einen Halogenameisensäureester benutzt, erhält man einen Ester einer Säure der Formel C.

Einen Ester kann man auch erhalten, gegebenenfalls nur als Nebenprodukt, wenn man Verbindungen der Formel B carboxyliert, worin $Am_o$ eine durch verestertes Carboxyl geschützte sekundäre Aminogruppe ist. Dabei kann die Acylschutzgruppe von der Aminogruppe auf das Methylcarbanion in 3-Stellung wandern, so dass nach Aufarbeitung eine Verbindung der Formel C resultiert, worin $Am_o$ eine freie sekundäre Aminogruppe ist und worin die Carboxylgruppe in veresterter Form vorliegt. Beispielsweise ergibt 5-(N-Methyl-N-tert.-butoxycarbonylamino)-3-methyl-1,2,4-thiadiazol bei der Behandlung mit Lithiumdiisopropylamid den 2-(5-Methylamino-1,2,4-thiadiazol-2-yl)essigsäure-tert.-butylester. Die Aufarbeitung der Reaktionsmischung erfolgt durch Behandeln mit Wasser und Säure oder Base und Extraktion auf übliche Weise.

Stufe 3: Die α-Ketoverbindung der Formel D oder ein Ester davon wird z.B. erhalten, indem man eine Verbindung der Formel C oder einen Ester davon mit einem geeigneten Oxidationsmittel, z.B. Selendioxid behandelt. Die Reaktion wird in einem inerten Lösungsmittel, wie einem Aether, z.B. Dioxan oder Tetrahydrofuran, einem Amid, z.B. Dimethylformamid, oder einem tertiären Amin, z.B. Pyridin, bei erniedrigter oder erhöhter Temperatur, z.B. bei etwa 0°C bis etwa 100°C, bevorzugt bei Zimmertemperatur bis zu etwa 80°C, durchführt.

Stufe 4a: Eine Verbindung der Formel III oder ein Ester davon, worin Am eine geschützte oder tertiäre Aminogruppe $Am_o$ bedeutet, kann erhalten werden, indem man eine Verbindung der Formel D mit einem Hydroxylamin der Formel $H_2N-O-R_4$ (VI) behandelt. Die Reaktion kann z.B. wie unter Verfahren c) beschrieben durchgeführt werden.

Stufe 4b: Eine Verbindung der Formel III oder ein Ester davon, worin Am eine geschützte oder tertiäre Aminogruppe $Am_o$ bedeutet, kann auch erhalten werden, indem man eine Verbindung der Formel C nitrosiert und gewünschtenfalls eine erhaltene Verbindung der Formel III, worin $R_4$ Wasserstoff bedeutet, in eine Verbindung der Formel III überführt,

worin $R_4$ für einen von Wasserstoff verschiedenen Rest $R_4$ steht. Die Nitrosierung kann z.B. wie unter Verfahren d) und die Substituierung der Hydroxygruppe im Hydroxyiminorest wie unter "Substituierung einer Hydroximinogruppe" beschrieben durchgeführt werden.

Stufe 4c: Eine Verbindung der Formel III oder ein Ester davon, worin Am eine freie primäre oder sekundäre Aminogruppe bedeutet, kann auch erhalten werden, indem man eine Verbindung der Formel E, worin $X_1$ Halogen bedeutet, und $R_4$ die oben gegebene Bedeutung hat, mit einem Salz der Rhodanwasserstoffsäure bzw. einem Isorhodanwasserstoffsäureester, oder eine Verbindung der Formel E, worin $X_1$ Wasserstoff ist, mit Rhodan behandelt. Die Ringschlussreaktion mit dem Salz der Rhodanwasserstoffsäure bzw. Rhodan kann wie unter Verfahren e) beschrieben durchgeführt werden.

Stufe 4d: Eine Verbindung der Formel III oder ein Ester davon, worin Am eine primäre, sekundäre oder tertiäre Aminogruppe bedeutet, kann auch erhalten werden, indem man eine Verbindung der Formel L oder einen Ester davon, worin Y Halogen ist und $R_4$ die oben angegebene Bedeutung hat, mit Ammoniak oder einem primären oder sekundären Amin Am-H, oder einem Metallamid davon, umsetzt. Der Austausch von Halogen Y, z.B. von Chlor, gegen die Aminogruppe Am erfolgt wie unter Verfahren j) beschrieben.

In einer Verbindung der Formel III kann eine geschützte Aminogruppe $Am_o$ in eine freie Aminogruppe oder in eine andere geschützte Aminogruppe übergeführt werden, oder, falls $R_4$ Wasserstoff ist, kann dieses entsprechend durch einen Rest $R_4$ substituiert werden. Erhaltene Ester von Verbindungen der Formel III werden auf an sich bekannte Weise in die freien Carbonsäuren übergeführt.

Reaktionsfähige Derivate von Verbindungen der Formel III, die zur Acylierung geeignet sind, werden in an sich bekannter Weise, gegebenenfalls in situ, gebildet.

Verbindungen der Formel E können, ausgehend von Verbindungen der Formel F, über Verbindungen der Formel G gemäss den Stufen 5 und 6 erhalten werden:

Stufe 5: Verbindungen der Formel G, worin $R_o$ Niederalkyl ist, und worin die Carboxylgruppe gegebenenfalls geschützt ist, können erhalten werden, indem man an eine Verbindung der Formel F, worin die funktionelle Carboxylgruppe gegebenenfalls geschützt ist, den Alkohol der Formel $R_o$-OH an die Cyangruppe anlagert. Die Reaktion erfolgt in an sich bekannter Weise, z.B. in Gegenwart einer Säure, wie einer Halogenwasserstoffsäure, z.B. Salzsäure, bei erniedrigter oder erhöhter Temperatur, bevorzugt zwischen etwa -30°C und etwa +40°C, insbesondere bei etwa 0°C, und in einem geeigneten Lösungsmittel, z.B. im Alkohol der Formel $R_o$-OH selbst. Man erhält so ein Säureadditionssalz des Iminoesters der Formel G oder eines geschützten Derivates davon.

Stufe 6: Eine Verbindung der Formel G, worin $R_o$ Niederalkyl bedeutet, oder ein Derivat davon, worin funktionelle Gruppen geschützt sind, oder ein Säureadditionssalz einer solchen Verbindung wird durch Behandeln mit Ammoniak in eine Verbindung der Formel E übergeführt, worin X Wasserstoff ist,und worin funktionelle Gruppen gegebenenfalls geschützt sind. Statt Ammoniak kann z.B. auch Ammoniumhydroxid oder Ammoniumchlorid eingesetzt werden. Die Umsetzung mit Ammoniak erfolgt in einem geeigneten, gegebenenfalls wässrigen Lösungsmittel und bei Temperaturen zwischen etwa -30°C und etwa +40°C. In einer Verbindung der Formel E, worin X Wasserstoff ist, kann dieser, z.B. wie im Verfahren fb) beschrieben wird, durch Halogen substituiert werden.

Stufe 7: Verbindungen der Formel L, worin Y Halogen darstellt und $R_4$ die oben gegebene Bedeutung hat, Ester oder Salze davon werden erhalten, indem man eine Verbindung der Formel E, worin $X_1$ Wasserstoff ist, oder einen Ester oder ein Salz davon, mit einem Perhalogenmethylmercaptan der Formel $Y_3C-S-Y$, worin Y die obige Bedeutung hat, umsetzt.

Im Ausgangsmaterial und im Produkt der Formel L ist Y bevorzugt Chlor. Die Reaktion wird in Gegenwart einer den entstehenden Halogenwasserstoff H-Y neutralisierenden Base in einem organischen oder organisch-wässrigen Lösungsmittel oder Lösungsmittelgemisch durchgeführt. Als Basen können organische Basen, wie tertiäre Amine, z.B. Triniederalkylamine, wie Trimethylamin, Aethyldiisopropylamin, oder aromatische Amine, wie Pyridin oder anorganische Basen, z.B. Alkalimetallhydroxide, wie Lithium-, Natrium- oder Kaliumhydroxid, oder dergleichen, bevorzugt in stöchiometrischen Mengen, eingesetzt werden. Als Lösungsmittel dienen Wasser, organische Lösungsmittel, wie Alkohol, z.B. Methanol oder Aethanol, Halogenkohlenwasserstoffe, wie Methylenchlorid oder Chloroform, Amide, wie Dimethylformamid, Sulfoxide, wie Dimethylsulfoxid, Aether, wie Dioxan, Tetrahydrofuran, oder dergleichen, oder gegebenenfalls Mischungen oder wässrige Emulsionen davon, denen zur besseren Emulgierung gegebenenfalls ein Emulgator, wie Natriumdodecylsulfat, zugefügt werden kann. Die Reaktion wird bei Zimmertemperatur, unter Abkühlen oder leichtem Erwärmen, je nach Reaktionsfähigkeit, d.h. bei etwa -15°C bis etwa +50°C, bevorzugt bei etwa -10°C bis etwa +20°C, durchgeführt.

Ausgangsstoffe der Formel IV können u.a. als Nebenprodukt bei der Herstellung von Verbindungen vom Typ der Formel I erhalten werden, z.B. wenn diese unter basischen Bedingungen gebildet werden. Sie brauchen nicht in reiner Form vorzuliegen, sondern können z.B. auch im Gemisch mit entsprechenden Verbindungen der Formel I eingesetzt werden.

Verbindungen der Formel V können z.B. durch Acylieren von Verbindungen der Formel II mit Verbindungen der Formel D oder einem reaktionsfähigen funktionellen Derivat davon, z.B. nach einer der unter Verfahren a) beschriebenen Methoden, erhalten werden.

Verbindungen der Formel VII können z.B. durch Acylieren von Verbindungen der Formel II mit einem, den Acylrest einer Säure der Formel C einführenden Acylierungsmittel, z.B. nach einer der unter Verfahren a) beschriebenen Methoden, erhalten werden.

Verbindungen der Formel VIII können z.B. durch Acylieren von Verbindungen der Formel II mit einem, den Acylrest einer Säure der Formel E einführenden Acylierungsmittel, z.B. nach einer der unter Verfahren a) beschriebenen Methoden erhalten werden. Ferner kann man, analog den Stufen 6 und 7 im Reaktionsschema zur Herstellung von Ausgangsstoffen der Formel III, ausgehend von Verbindungen der Formel

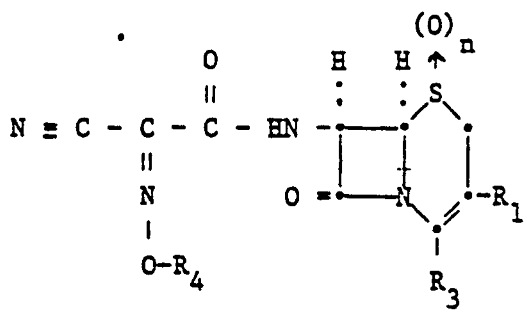

(H)

über Verbindungen der Formel

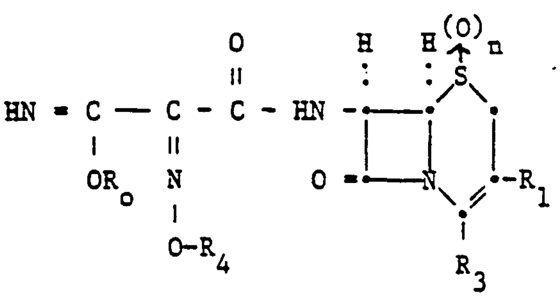

(J),

zu den gewünschten Ausgangsstoffen der Formel VIII gelangen. Die Verbindungen der Formeln H und J können ihrerseits durch Acylieren von

Verbindungen der Formel II mit einem, den Acylrest einer Säure der Formel F bzw. G einführenden Acylierungsmittel, z.B. nach einer der unter Verfahren a) beschriebenen Methoden erhalten werden.

Verbindungen der Formel IX können z.B. durch Acylieren der Aminogruppe in einer 7β-Amino-3-formyl-3-cephem-4-carbonsäure-Verbindung mit einem, den Acylrest einer Säure der Formel III einführenden Acylierungsmittel, z.B. nach einer der unter Verfahren a) beschriebenen Methoden, gebildet werden.

Ausgangsstoffe der Formel X, sowie der Formel XIII, kann man z.B. erhalten, indem man die Aminogruppe in einer 7β-Amino-3-methylen-cepham-4ξ-carbonsäure-Verbindung mit geschützter Carboxylgruppe in 4-Stellung durch Behandeln mit einem, den Acylrest einer Säure der Formel III einführenden Acylierungsmittel, z.B. nach einer unter Verfahren a) beschriebenen Methoden, acyliert, und die Methylengruppe in der so erhältlichen Verbindung durch geeignete Oxidation, z.B. mit Ozon, oxidativ abspaltet; man kann so die Ausgangsstoffe der Formel XIII bzw. der Formel X, worin $X_2$ für Hydroxy steht, erhalten. Analoge Verbindungen, worin $X_2$ gegebenenfalls substituiertes Amino bedeutet, kann man z.B. nach dem in der deutschen Offenlegungsschrift 2 606 196 beschriebenen Verfahren erhalten, indem man von einer 6-Acylamino-2,2-dimethyl-penam-3-carbonsäure-1-oxid-Verbindung ausgeht, in welcher Acyl den entsprechenden Rest einer Säure der Formel III darstellt, worin gegebenenfalls vorhandene funktionelle Gruppen vorzugsweise in geschützter Form vorliegen, und den man z.B. nach einer der unter Verfahren a) beschriebenen Methoden einführt.

Verbindungen der Formel XI kann man z.B. nach dem in der deutschen Offenlegungsschrift 2 151 567 beschriebenen Verfahren erhalten, indem man von einem Bis-(cis-3β-amino-2-oxo-4β-azetidinyl)-disulfid, in welchem die Aminogruppen frei oder mit Niederalkyliden, wie Isopropyliden oder 1-Isobutyliden, substituiert sind, ausgeht, in diesem die Aminogruppen durch Umsetzten mit einem geeigneten, den Acylrest einer Aminosäure der Formel III, worin gegebenenfalls

vorhandene funktionelle Gruppen vorzugsweise in geschützter Form vorliegen, Acylierungsmittel, z.B. nach einer der unter Verfahren a) beschriebenen Methoden, acyliert, und dann die restlichen Verfahrensschritte bis zur Herstellung des Ausgangmaterials der Formel XI durchführt, wobei dieses üblicherweise in situ gebildet wird.

Ausgangsstoffe der Formel XIV kann man z.B. nach dem in der deutschen Offenlegungsschrift 2 506 330 beschriebenen Verfahren herstellen, indem man, wie z.B. im Zusammenhang mit einem Ausgangsmaterial der Formel X, worin $X_2$ für gegebenenfalls substituiertes Amino steht, von einer 6-Acylamino-2,2-dimethyl-penam-3-carbonsäure-1-oxid-Verbindung ausgeht, in welcher Acyl den entsprechenden Rest einer Säure der Formel III darstellt, worin gegebenenfalls vorhandene funktionelle Gruppen vorzugsweise in geschützter Form vorliegen. Dabei kann man auf der Stufe von Verbindungen vom Typ der Formel

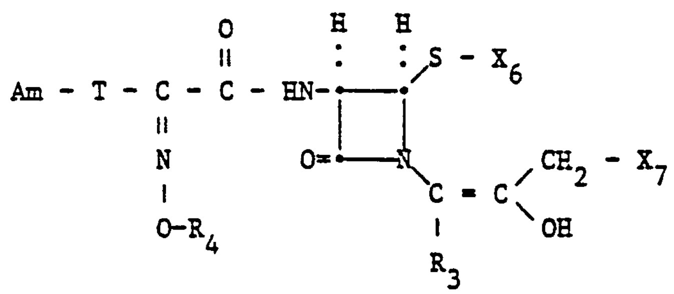

(K)

die Hydroxygruppe, anstatt sie zu veräthern, z.B. nach einem der unter Verfahren fb) beschriebenen Methoden, gegen Halogen, z.B. Chlor, austauschen.

Ausgangsstoffe der Formel XV kann man z.B. erhalten, indem man von den entsprechenden 7β-Amino-3-($X_8$-$CH_2$)-3-cephem-4-carbonsäure-Verbindungen ausgeht, und die Aminogruppe z.B. durch Behandeln mit einem, den Acylrest einer Säure der Formel III einführenden Acylierungsmittel, z.B. nach einer der unter Verfahren a) beschriebenen Methoden, acyliert.

Verbindungen der Formel XVII können auf verschiedene Weise hergestellt werden, beispielsweise analog den Verfahren a) oder b) oder der Stufe 7, insbesondere indem man, analog Verfahren a), in einer Verbindung der Formel II, worin n, $R_1$ und $R_3$ die angegebenen Bedeutungen haben, und worin die 7β-Aminogruppe gegebenenfalls durch eine die Acylierung erlaubende Gruppe substituiert ist, die 7β-Aminogruppe durch Behandeln mit einem den Acylrest einer Carbonsäure der Formel L einführenden Acylierungsmittel acyliert, oder indem man, analog Stufe 7, eine Verbindung der Formel VIII, worin $X_1$ Wasserstoff bedeutet und n, $R_1$, $R_3$ und $R_4$ die angegebenen Bedeutungen haben, oder ein Salz davon, mit einem Perhalogenmethylmercaptan der Formel $Y_3C-S-Y$ umsetzt, und gewünschtenfalls die bei der Herstellung der Verbindungen der Formel I genannten nachträglichen Operationen durchführt.

Neue Ausgangsverbindungen, sowie Zwischenprodukte und Verfahren zu ihrer Herstellung sind ebenfalls Gegenstand der vorliegenden Erfindung.

Die pharmakologisch verwendbaren Verbindungen der vorliegenden Erfindung können z.B. zur Herstellung von pharmazeutischen Präparaten verwendet werden, welche eine wirksame Menge der Aktivsubstanz zusammen oder im Gemisch mit anorganischen oder organischen, festen oder flüssigen, pharmazeutisch verwendbaren Trägerstoffen enthalten, die sich vorzugsweise zur parenteralen Verabreichung eignen.

Vorzugsweise verwendet man die pharmakologisch wirksamen Verbindungen der vorliegenden Erfindung in Form von parenteral, z.B. intramuskulär oder intravenös, verabreichbaren Präparaten oder von Infusionslösungen. Solche Lösungen sind vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfstoffe, z.B. Konservier-, Stabilisier-, Netz

und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Lösungs- oder Lyophilisierungsverfahren, hergestellt und enthalten von etwa 0,1% bis 100%, insbesondere von etwa 1% bis etwa 50%, Lyophilisate bis zu 100% des Aktivstoffes.

Verbindungen der Formel I, insbesondere diejenigen, in welchen $R_3$ eine physiologisch spaltbare, veresterte Carboxylgruppe, z.B. Pivaloyloxymethoxycarbonyl, darstellt, können auch oral, z.B. in Form von Kapseln, verabreicht werden. Diese enthalten den Wirkstoff, gegebenenfalls zusammen mit geeigneten Trägerstoffen, in Form eines Granulats und zwar in Dosen von etwa 0,2 g bis etwa 0,5 g pro Dosiseinheit.

Je nach Art der Infektion und Zustand des infizierten Organismus verwendet man tägliche Dosen von etwa 0,5 g bis etwa 5 g s.c. zur Behandlung von Warmblütern von etwa 70 kg Gewicht.

Die folgenden Beispiele dienen zur Illustration der Erfindung; Temperaturen werden in Celsiusgraden angegeben.

In den Beispielen werden folgende Abkürzungen verwendet:

BOC: tert.-Butyloxycarbonyl
F. : Schmelzpunkt

Dünnschichtchromatographie: Opti-$UPC_{12}$-Platten der Fa. Antec sind mit Silicagel beschichtete Glasplatten, wobei das Silicagel mit Dodecyltrichlorsilan behandelt wurde (zur Chromatographie mit umgekehrter Phase).

Beispiel 1: 7β-[2-(5-tert.-Butyloxycarbonylamino-1,2,4-thiadiazol-3-yl)-2-Z-methoxyimino-acetylamino]-3-cephem-4-carbonsäure-diphenylmethylester

Eine Lösung von 3.3 g (11.0 mMol) 2-(5-tert.-Butyloxycarbonylamino-1,2,4-thiadiazol-3-yl)-2-Z-methoxyimino-essigsäure in 50 ml Methylenchlorid, enthaltend 0.93 ml Pyridin, wird bei -15° und unter einer Stickstoffatmosphäre mit 1.9 ml (12.9 mMol) Diäthylphosphorbromidat versetzt und 30 Minuten bei dieser Temperatur gerührt. Nach Zugabe von 3.5 g (9.5 mMol) 7β-Amino-3-cephem-4-carbonsäure-diphenylmethylester bei -15°, wird das Reaktionsgemisch bei Raumtemperatur während 2-1/2 Stunden gerührt, dann mit 50 ml Methylenchlorid verdünnt, und mit verdünnter wässriger Schwefelsäure, Wasser und wässriger Natriumhydrogencarbonatlösung gewaschen. Die organische Phase wird über Magnesiumsulfat getrocknet und unter vermindertem Druck eingedampft. Der Rückstand wird durch Chromatographie an Silikagel gereinigt, wobei man das Produkt in Toluol aufzieht und mit Toluol, enthaltend steigende Anteile Essigsäureäthylester eluiert. Man erhält so den 7β-[2-(5-tert.-Butyloxycarbonylamino-1,2,4-thiadiazol-3-yl)-2-Z-methoxyimino-acetylamino]-3-cephem-4-carbonsäure-diphenylmethylester, Dünnschichtchromatogramm (Silikagel): Rf = 0.50 (System: Essigsäureäthylester); Infratorabsorptionsspektrum (in Mineralöl): charakteristische Banden bei 3.10, 5.65, 5.80 und 6.45 μ.

Das Ausgangsmaterial kann z.B. wie folgt hergestellt werden:

Eine Suspension von 20,0 g (0,174 Mol) 5-Amino-3-methyl-1,2,4-thiadiazol (deutsches Patent 955.684) in 100 ml des Anhydrids des Kohlensäure-tert.-butylhalbesters wird bei 100° (Badtemperatur) 12 Stunden gerührt. Das Reaktionsgemisch wird abgekühlt, die flüchtigen Anteile werden unter vermindertem Druck entfernt, und der teilweise kristalline Rückstand wird durch Umkristallisieren aus Isopropanol gereinigt. Man erhält so 18,2 g 5-tert.-Butyloxycarbonylamino-3-methyl-1,2,4-thiadiazol, F. 136-137°; Dünnschichtchromatogramm (Silikagel): Rf = 0.65 (System: Essigsäureäthylester); Ultraviolettabsorptionsspektrum (in Aethanol): $\lambda_{max.}$ = 222 nm (ε= 4000) und 245 nm (ε=8250); Infrarotabsorptionsspektrum (in Mineralöl): charakteristische Banden bei 5.90, 6.45 und 8.75 μ.

Eine frisch zubereitete Lösung von Lithiumdiisopropylamid in 100 ml absolutem Tetrahydrofuran [aus 26 ml (0,184 Mol) Diisopropylamin und 104 ml (0,208 Mol) einer etwa 2-n. Lösung von Butyllithium in n-Hexan] wird bei -78° unter Stickstoff mit 10,0 g (0,046 Mol) 5-tert.-Butyloxycarbonylamino-3-methyl-1,2,4-thiadiazol in 50 ml absolutem Tetrahydrofuran versetzt und 30 Minuten bei dieser Temperatur gerührt. Nach Erwärmen des Reaktionsgemisches auf -30° wird während 30 Min. Kohlendioxid eingeleitet. Die Lösung wird mit 100 ml Wasser versetzt und mit 50 ml Diäthyläther verdünnt. Die organische Phase wird abgetrennt; die wässrige Phase wird mit 2-n. Salzsäure auf pH 2,0 gestellt und mit Essigsäureäthylester extrahiert. Die organische Phase wird abgetrennt, mit wässriger Natriumchloridlösung gewaschen, über Magnesiumsulfat getrocknet und unter vermindertem Druck eingeengt. Der Rückstand wird mit Diäthyläther trituriert und ergibt die 2-(5-tert.-Butyloxycarbonylamino-1,2,4-thiadiazol-3-yl)-essigsäure, F. 163-165°; Dünnschichtchromatogramm (Silikagel): Rf = 0.40 (System: Chloroform/Methanol/Eisessig 85:12:3); Ultraviolettabsorptionsspektrum (in Methanol): $\lambda_{max}$ = 215 nm ($\varepsilon$= 5250) und 245 nm ($\varepsilon$= 8250); Infrarotabsorptionsspektrum (in Mineralöl): charakteristische Banden bei 2.85, 2.90, 3.15 und 6.45 $\mu$.

Eine Suspension von 5,0 g (19,25 mMol) 2-(5-tert.-Butyloxycarbonylamino-1,2,4-thiadiazol-3-yl)-essigsäure und 4,2 g Selendioxid in 100 ml absolutem Dioxan wird 3 Stunden bei 80° gerührt. Nach Filtrieren des Reaktionsgemisches wird das Filtrat unter vermindertem Druck eingeengt. Der Rückstand wird in 150 ml Essigsäureäthylester aufgenommen und mit wässriger Natriumhydrogencarbonatlösung extrahiert.

Die organische Phase wird abgetrennt, mit wässriger Natriumchloridlösung gewaschen, über Magnesiumsulfat getrocknet und unter vermindertem Druck eingeengt. Man erhält so das einheitliche 5-tert.-Butyloxycarbonylamino-3-formyl-1,2,4-thiadiazol, Dünnschichtchromatogramm (Silikagel): Rf = 0.65 (System: Chloroform/Methanol/Eisessig 85:12:3); Infrarotabsorptionsspektrum (in Methylenchlorid): charak-

teristische Banden bei 2.90, 5.80, 6.15, 6.50 und 8.70 µ.

Die wässrige Natriumhydrogencarbonatphase wird mit 2-n. Salzsäure auf pH 1,0 gestellt und mit Essigsäureäthylester extrahiert. Die organische Phase wird abgetrennt, mit wässriger Natriumchloridlösung gewaschen, über Magnesiumsulfat getrocknet und eingeengt. Man erhält so die einheitliche 2-(5-tert.-Butyloxycarbonylamino-1,2,4-thiadiazol-3-yl)-2-oxo-essigsäure in der Form eines Schaums, Dünnschichtchromatogramm (Silikagel): Rf = 0.30 (System: Chloroform/Methanol/Eisessig 85:12:3); Ultraviolettabsorptionsspektrum (in Methanol): $\lambda_{max}$ = 219 nm ($\varepsilon$=7150) und 243 nm ($\varepsilon$= 7900); Infrarotabsorptionsspektrum (in Mineralöl): charakteristische Banden bei 3.10, 5.80, 6.15, 6.45 und 8.60 µ.

Eine Suspension von 3,4 g (12,4 mMol) 2-(5-tert.-Butyloxycarbonylamino-1,2,4-thiadiazol-3-yl)-2-oxo-essigsäure, 1,11 g (13,35 mMol) Methoxyaminhydrochlorid und 1,1 ml Pyridin in 70 ml 95%-igem Aethanol wird 3 Stunden bei Raumtemperatur gerührt. Das Reaktionsgemisch wird unter vermindertem Druck eingeengt. Der Rückstand wird mit 100 ml Essigsäureäthylester und 100 ml Wasser verdünnt; die wässrige Phase wird mit 2-n. Salzsäure auf pH 2 gestellt und anschliessend mit Essigsäureäthylester extrahiert. Die organische Phase wird abgetrennt, mit wässriger Natriumchloridlösung gewaschen, über Magnesiumsulfat getrocknet und eingeengt. Der Rückstand besteht aus einem etwa 9:1 Gemisch der Z- und E-Formen der 2-(5-tert.-Butyloxycarbonylamino-1,2,4-thiadiazol-3-yl)-2-methoxyimino-essigsäure. Die reine Z-Form erhält man durch fraktionierte Kristallisation aus Methylenchlorid, F. 137-139° (mit Zersetzen); Dünnschichtchromatogramm (Silikagel): Rf = 0.30 (System: sek.-Butanol/Eisessig/Wasser 67:10:23); Infrarotabsorptionsspektrum (in Mineralöl): charakteristische Banden bei 2.75, 2.90, 5.85, 6.18, 6.45 und 9.60 µ; Ultraviolettabsorptionsspektrum (in Methanol): $\lambda_{max.}$ = 234 nm ($\varepsilon$= 22250).

Beispiel 2: 7β-[2-(5-Amino-1,2,4-thiadiazol-3-yl)-2-Z-methoxyimino-acetylamino]-3-cephem-4-carbonsäure-natriumsalz

Eine Lösung von 3,7 g (5,7 mMol) 7β-[2-(5-tert.-Butyloxycarbonylamino-1,2,4-thiadiazol-5-yl)-2-Z-methoxyimino-acetylamino]-3-cephem-4-carbonsäure-diphenylmethylester, in 11 ml Methylenchlorid und 3,1 ml Anisol wird bei Raumtemperatur mit 44 ml Trifluoressigsäure versetzt und während 30 Minuten gerührt.

Man giesst in 750 ml eines 1:2-Gemisches von Diäthyläther und n-Hexan, wobei man dieses rührt. Der Niederschlag wird abfiltriert und in einem Gemisch von 500 ml Wasser und 10 ml Methanol gelöst. Die Lösung wird mit wässriger Natriumhydrogencarbonatlösung auf pH 6.8 gestellt und dann lyophilisiert. Den Rückstand chromatographiert man an Silikagel (Opti $UPC_{12}$ der Firme Antec), wobei man mit Wasser eluiert, und die Elution der gewünschten Verbindung mittels eines Ultraviolettabsorptionsdetektors verfolgt. Man erhält so das Natriumsalz der 7β-[2-(5-Amino-1,2,4-thiadiazol-3-yl)-2-Z-methoxyimino-acetylamino]-3-cephem-4-carbonsäure, F. 190° (mit Zersetzen); Dünnschichtchromatogramm (Silikagel): Rf = 0.60 (System: Wasser/Acetonitril 9:1); Ultraviolettabsorptionsspektrum (in Methanol): $\lambda_{max.}$ = 234 nm ($\varepsilon$ = 19900); Infrarotabsorptionsspektrum (in Mineralöl): charakteristische Banden bei 3.00, 5.67, 6.00 und 6.25 μ.

Beispiel 3: 7β-[2-(5-Amino-1,2,4-thiadiazol-3-yl)-2-Z-(2-carboxyprop-2-yloxyimino)-acetamido]-3-(4-carbamoylpyridiniomethyl)-3-cephem-4-carboxylat

a) Zu einer auf 80° vorgewärmten Lösung von 750 mg Natriumiodid (5 mM) und 63 mg (0,51 mM) Isonicotinsäureamid in 1,0 ml Wasser gibt man eine getrennt davon bereitete Lösung von 0,25 g (0,43 mMol) 7β-[2-(5-Amino-1,2,4-thiadiazol-3-yl)-2-Z-(2-carboxyprop-2-yloxyimino)-acetamido]-3-acetoxymethyl-3-cephem-4-carbonsäure-dinatriumsalz in 1 ml Wasser. Nach Zugabe von 0,02 ml Essigsäure wird 2 Stunden bei

80° unter Stickstoffatmosphäre gerührt. Die Reaktionslösung wird unter reduziertem Druck auf etwa das halbe Volumen eingeengt und unter Kühlung auf -20° in 40 ml Aceton eingetragen. Der gebildete Niederschlag wird abfiltriert, mit Aceton gewaschen und getrocknet. Man erhält die Titelverbindung vom Rf-Wert 0,18 ($UPC_{12}$-Platten, Wasser/Acetonitril 95:5); UV-Spektrum (Wasser): $\lambda_{max}$ = 237 nm ($\varepsilon$ = 18'800).

Das Ausgangsmaterial kann z.B. wie folgt hergestellt werden:

b) 2-(5-tert.-Butoxycarbonylamino-1,2,4-thiadiazol-3-yl)-2-Z-(2-tert.-butoxycarbonylprop-2-yloxyimino)essigsäuremethylester

Eine Lösung von 1,4 g (6,27mMol) 2-Bromisobuttersäure-tert.-butylester und 1,94 g (14,03mMol) fein pulverisiertes Kaliumcarbonat in 12,50 ml Dimethylsulfoxid wird bei Zimmertemperatur unter Stickstoff mit einer Lösung von 1,70 g (5,62 mMol) 2-(5-tert.-Butoxycarbonylamino-1,2,4-thiadiazol-3-yl)-2-hydroxyiminoessigsäure-methylester in 7,5 ml Dimethylsulfoxid versetzt und 14 Stunden bei dieser Temperatur gerührt. Das Reaktionsgemisch wird bei 30° im Hochvakuum zur Trockne eingedampft. Der Rückstand wird in 200 ml Essigsäureäthylester aufgenommen und nacheinander mit Wasser, verdünnter Salzsäure (1 N), Wasser und gesättigter Natriumchloridlösung gewaschen, über Magnesiumsulfat getrocknet und im Vakuum zur Trockne eingedampft. Der Rückstand wird aus ca. 5 ml Aether kristallisiert und ergibt die Titelverbindung vom Schmelzpunkt 142-144°.

c) 2-(5-tert.-Butoxycarbonylamino-1,2,4-thiadiazol-3-yl)-2-Z-(2-tert.-butoxycarbonylprop-2-yloxyimino)essigsäure

Eine Lösung von 11 g (22,55 mMol) 2-(5-tert.-Butoxycarbonylamino-1,2,4-thiadiazol-3-yl)-2-Z-(2-tert.-butoxycarbonylprop-2-yloxyimino)essigsäure-methylester in 290 ml Methanol und 96 ml 2 N Natronlauge wird bei einer Temperatur von 50° 6 1/2 Stunden rühren gelassen. Das Reaktionsgemisch wird bei 35° am Hochvakuum zur Trockne

eingedampft. Der Rückstand wird in 200 ml Wasser (destilliert) aufgenommen und einmal mit 200 ml Essigsäureäthylester extrahiert. Die wässrige Phase wird mit 200 ml Essigsäureäthylester überschichtet und mit konz. Salzsäure sauer gestellt. Die Essigsäureäthylesterphase wird nacheinander mit Wasser und gesättigter Natriumchloridlösung gewaschen, über Magnesiumsulfat getrocknet und im Vakuum eingeengt, wobei die Titelverbindung kristallin anfällt. DC-Rf-Wert 0,2 (Silicagel, Chloroform/Methanol 4:1); IR-Spektrum (Dioxan): charakteristische Absorptionsbanden bei 5,81; 5.74; 3,3 μ. F.: 180-183°.

d) <u>7β-[2-(5-tert.-Butoxycarbonylamino-1,2,4-thiadiazol-3-yl)-2-Z-(2-tert.-butoxycarbonylprop-2-yloxyimino)-acetamido]-3-acetoxymethyl-3-cephem-4-carbonsäure</u>

Eine Suspension von 15 ml Methylenchlorid abs., 0,3 ml Dimethylformamid und 0,33 ml (7,84 mMol) Oxalsäuredichlorid wird bei -10° 30 Minuten unter Stickstoff gerührt. Dann wird 1,3 g (3,01 mMol) 7β-[2-(5-tert.-Butoxycarbonylamino-1,2,4-thiadiazol-3-yl)-2-Z-(2-tert.-butoxycarbonylprop-2-yloxyimino)essigsäure zur Suspension gegeben. Die entstehende klare Lösung wird 30 Minuten bei 0-5° unter Stickstoff gerührt. Zu dieser Lösung tropft man eine frisch hergestellte Lösung von 0,87 g (3,19 mMol) 7β-Aminocephalosporansäure in 18 ml Methylenchlorid abs. und 3 ml N,O-Bistrimethylsilylacetamid und rührt bei einer Temperatur von 0-5° unter Stickstoff 16 Stunden. Die Reaktionslösung wird nacheinander mit Wasser und gesättigter Natriumchloridlösung gewaschen, über Magnesiumsulfat getrocknet und am Vakuum zum trockenen Schaum eingedampft. Man erhält die Titelverbindung vom Rf-Wert 0,43 (Silicagel, Essigester/Essigsäure 9:1). IR-Spektrum (Nujol): charakteristische Absorptionsbanden bei 5,6; 5,82; 6,04 μ.

e) <u>7β-[2-(5-Amino-1,2,4-thiadiazol-3-yl)-2-Z-(2-carboxyprop-2-yloxyimino)acetamido]-3-acetoxymethyl-3-cephem-4-carbonsäure Dinatriumsalz</u>

Eine Lösung von 2,3 g (3,35 mMol) 7β-[2-(5-tert.-Butoxy-

carbonylamino-1,2,4-thiadiazol-3-yl)-2-Z-(2-tert.-butoxycarbonylprop-2-yloxyimino)acetamido]-3-acetoxymethyl-3-cephem-4-carbonsäure in 12,54 ml (16,38 mMol) Trifluoressigsäure und 2,10 ml (19,28 mMol) Anisol wird bei Zimmertemperatur 1 Stunde gerührt. Das Reaktionsgemisch wird bei 30° Badtemperatur am Vakuum zur Trockne eingedampft, der Rückstand mit Aether verrührt und das Produkt abfiltriert. Dann wird das Produkt in 28 ml Methanol gelöst und mit Natrium-äthylhexanoat versetzt. Man rührt die Lösung in 50 ml Essigsäureäthylester ein. Das ausgefallene Produkt wird abfiltriert und am Hochvakuum getrocknet. Man erhält die Titelverbindung mit dem Rf-Wert 0,78 ($UPC_{12}$-Platten, Wasser/Acetonitril 4:1). IR-Spektrum (Nujol): charakteristische Absorptionsbanden bei 3,04; 5,68; 6,24; 6,57 µ.

Beispiel 4: 7β-[2-(5-Amino-1,2,4-thiadiazol-3-yl)-2-Z-methoxyimino-acetamido]-3-pyridiniomethyl)-3-cephem-4-carboxylat

a) Aus 778 mg (1,63 mMol) 7β-[2-(5-Amino-1,2,4-thiadiazol-3-yl)-2-Z-methoxyiminoacetamido]-3-acetoxymethyl-3-cephem-4-carbonsäure-Natriumsalz, 270 mg Isonicotinsäureamid, 2,44 g Natriumjodid und 267 mg Trichloressigsäure in 1,63 ml Wasser erhält man analog Beispiel 3a), die Titelverbindung vom Rf-Wert 0,25 (Silicagel $UPC_{12}$, Wasser/Acetonitril 6:1), IR-Spektrum (Nujol): charakteristische Absorptionsbanden bei 3,0; 5,65; 6,40 µ.

Das Ausgangsmaterial kann z.B. wie folgt hergestellt werden:

b) 7β-[2-(5-tert.-Butoxycarbonylamino-1,2,4-thiadiazol-3-yl)-2-Z-methoxyiminoacetamido]-3-acetoxymethyl-3-cephem-4-carbonsäurediphenylmethylester

Eine Lösung von 1,5 g (4,96 mMol) 2-(5-tert.-Butoxycarbonylamino-1,2,4-thiadiazol-3-yl)-2-methoxyiminoessigsäure in 20 ml Methylenchlorid und 0,44 ml Pyridin wird bei -15° unter Stickstoff mit 0,88 ml (6,0 mMol) Diäthylphosphorbromidat versetzt und 30 Minuten bei dieser Temperatur gerührt. Nach Zugabe von 2,1 g (4,70 mMol)

7β-Amino-3-acetoxymethyl-3-cephem-4-carbonsäure-diphenylmethylester bei -15° wird das Reaktionsgemisch bei Raumtemperatur während 2 1/2 Stunden gerührt, dann mit 250 ml Methylenchlorid verdünnt, nacheinander mit verdünnter Schwefelsäure, Wasser, wässriger Natriumbicarbonatlösung und wässriger Natriumchloridlösung gewaschen, über Magnesiumsulfat getrocknet und im Vakuum eingeengt. Der Rückstand wird durch präparative Schichtchromatographie an Silicagel mit Chloroform gereinigt. Man erhält die einheitliche Titelverbindung vom Rf-Wert 0,40 (Silicagel, Toluol/Aethylacetat 1:1); IR-Spektrum ($CH_2Cl_2$): charakteristische Absorptionsbanden bei 3,10; 5,65; 5,80; 5,95; 6,40; 8,20 μ; UV-Spektrum (Methanol): $\lambda_{max}$ ($\varepsilon$) 232 (26280) nm.

c) <u>7β-[2-(5-Amino-1,2,4-thiadiazol-3-yl)-2-Z-methoxyimino-acetamido]-3-acetoxymethyl-3-cephem-4-carbonsäure Natriumsalz</u>

Eine Lösung von 3,8 g (5,23 mMol) 7β-[2-(5-tert.-Butoxycarbonylamino-1,2,4-thiadiazol-3-yl)-2-methoxyiminoacetamido]-3-acetoxymethyl-3-cephem-4-carbonsäure-diphenylmethylester in 8,5 ml Methylenchlorid und 2,8 ml Anisol wird bei Raumtemperatur mit 39 ml Trifluoressigsäure versetzt und während 25 Minuten gerührt, dann in 900 ml Diäthyläther/Hexan 1:2 eingerührt. Der Niederschlag, bestehend aus dem Trifluoracetat der 7β-[2-(5-Amino-1,2,4-thiadiazol-3-yl)-2-Z-methoxyiminoacetamido]-3-acetoxymethyl-3-cephem-4-carbonsäure, wird durch Filtration isoliert, in Methanol-haltigem Wasser (10 ml Methanol/500 ml Wasser) gelöst und die wässrige Phase mit wässriger Natriumbicarbonatlösung auf pH 6,8 gestellt. Die Lösung wird lyophilisiert, und die erhaltenen Natriumsalze werden mittels Chromatographie an Silicagel "Opti $UPC_{12}$" (Fa. Antec) mit Wasser/Acetonitril 6:1 gereinigt. Man erhält die einheitliche Titelverbindung vom Rf-Wert 0,30 ($UPC_{12}$-Platten, Wasser/Acetonitril 6:1), F. ab 150° (Zers.); UV-Spektrum (Methanol): $\lambda_{max}$ ($\varepsilon$): 234 (12240) nm; IR-Spektrum (Nujol): charakteristische Absorptionsbanden bei 3,00; 5,65; 5,95; 6,20; 6,52; 8,10; 9,60 μ.

Beispiel 5: 7β-[2-(5-Amino-1,2,4-thiadiazol-3-yl)-2-Z-methoxyimino-acetylamino]-3-(4-carbamoyl-pyridiniomethyl)-3-cephem-4-carboxylat

Eine Lösung von 0,478 g (1,0 mMol) 7β-[2-(5-Amino-1,2,4-thiadiazol-3-yl)-2-Z-methoxyimino-acetylamino]-3-acetoxymethyl-3-cephem-4-carbonsäure Natriumsalz in 1 ml Wasser wird zu einer 80° warmen Lösung von 0,17 g Isonicotinsäureamid und 1,71 g Natriumjodid in 0,4 ml Wasser gegeben und während 3 Stunden bei 70° gerührt. Nach Abkühlen auf Raumtemperatur wird das Reaktionsgemisch auf 100 ml Aceton gegossen, der gebildete Niederschlag filtriert und getrocknet. Nach dem Reinigen des Rohproduktes an einer XAD-2-Säule mit Wasser/Methanol Gemischen erhält man die einheitliche Titelverbindung vom Rf-Wert 0,35 ($UPC_{12}$-Platten, Wasser/Acetonitril 6:1); IR-Spektrum (Nujol): charakteristische Absorptionsbanden bei 3,05; 5,65; 6,20 μ.

Beispiel 6: 7β-[2-(5-tert.-Butyloxycarbonylamino-1,2,4-thiadiazol-3-yl)-2-methoxyimino-acetylamino]-3-methoxy-3-cephem-4-carbonsäure-diphenylmethylester

Aus 1,0 g (3,31 mMol) 2-(5-tert.-Butyloxycarbonylamino-1,2,4-thiadiazol-3-yl)-2-methoxyiminoessigsäure, 1,3 g (3,0 mMol) 7β-Ammonium-3-methoxy-3-cephem-4-carbonsäure-diphenylmethylester-chlorid, 0,80 ml Pyridin und 0,53 ml Diäthylphosphorbromidat erhält man analog Beispiel 1 die einheitliche Titelverbindung vom Rf-Wert 0,45 (Silicagel, Aethylacetat). IR-Spektrum (Nujol): charakteristische Absorptionsbanden bei 3,10; 5,65; 5,82; 5,90; 6,37 μ.

Beispiel 7: 7β-[2-(5-Amino-1,2,4-thiadiazol-3-yl)-2-Z-methoxyimino-acetylamino]-3-methoxy-3-cephem-4-carbonsäure Natriumsalz

Aus 0,60 g (0,9 mMol) 7β-[2-(5-tert.-Butyloxycarbonyl-amino-1,2,4-thiadiazol-3-yl)-2-methoxyimino-acetylamino]-3-methoxy-3-cephem-4-carbonsäure-diphenylmethylester in 1,7 ml Methylenchlorid, 0,5 ml Anisol und 6,9 ml Trifluoressigsäure erhält man analog Beispiel 2 die Titelverbindung vom Rf-Wert 0,45 ($UPC_{12}$-Platten, Wasser/Acetonitril 9:1), UV-Spektrum (Methanol): $\lambda_{max}$ ($\varepsilon$) 233 (16850) nm.

Beispiel 8: 7β-[2-(5-Amino-1,2,4-thiadiazol-3-yl)-2-Z-methoxyimino-acetylamino]-3-methoxy-4-carbonsäure-pivaloyloxymethylester

Eine Mischung von 1,2 g (3 mMol) 7β-[2-(5-Amino-1,2,4-thiadiazol-3-yl)-2-Z-methoxyimino-acetylamino]-3-methoxy-3-cephem-4-carbonsäure Natriumsalz in 17 ml N,N-Dimethylformamid wird bei 0° mit 0,68 ml Jodmethylpivalat versetzt und während 50 Minuten bei dieser Temperatur gerührt, dann in 100 ml Phosphatpuffer pH 7,0 gegossen und mit Aethylacetat/Aceton 4:1 extrahiert. Die organischen Extrakte werden mit wässriger Natriumchloridlösung gewaschen, über Magnesiumsulfat getrocknet und im Vakuum eingeengt. Der Rückstand wird mit Diäthyläther/Hexan 1:1 gerieben. Man erhält die Titelverbindung vom Rf-Wert 0,85 (Silicagel, Aceton/Eisessig/Wasser/Ammoniak 20:8:5:1).

Beispiel 9: 7β-[2-(5-tert.-Butyloxycarbonylamino-1,2,4-thiadiazol-3-yl)-2-N-methylcarbamoyloxyimino-acetylamino]-3-cephem-4-carbonsäure-diphenylmethylester

a) 1,01 g (2,95 mMol) 2-(5-tert.-Butyloxycarbonylamino-1,2,4-thiadiazol-3-yl)-2-methylcarbamoyloxyiminoessigsäure und 0,4 ml N-Methylmorpholin werden bei -5° zu einer gerührten Suspension eines Vilsmeier-Reagenses (dargestellt aus 0,3 ml (3,6 mMol) Oxalylchlorid und 0,28 ml N,N-Dimethylformamid in 19 ml trockenem Aethylacetat) gegeben. Das Gemisch wird bei 0° während 45 Minuten gerührt und anschliessend auf -5° abgekühlt. Nach Zugabe von 1,01 g (3,0 mMol) 7β-Amino-3-cephem-4-carbonsäure-diphenylmethylester wird während 2 Stunden bei 0° gerührt, dann mit Aethylacetat und Wasser verdünnt. Die organische Phase wird abgetrennt, nacheinander mit Phosphatpufferlösung pH 8,0 und wässriger Natriumchloridlösung gewaschen, über Magnesiumsulfat getrocknet und im Vakuum eingeengt. Der Rückstand wird mit Aether verrieben. Es entsteht die Titelverbindung vom Rf-Wert 0,80 (Silicagel, Aethylacetat). IR-Spektrum (Methylenchlorid): charakteristische Absorptionsbanden bei 2,90; 3,10; 5,65; 5,80; 5,90; 6,45 μ.

Das Ausgangsmaterial kann z.B. wie folgt hergestellt werden:

b) 2-(5-tert.-Butoxycarbonylamino-1,2,4-thiadiazol-3-yl)-essigsäuremethylester

Eine Mischung von 30,0 g (0,115 Mol) 2-(5-tert.-Butoxycarbonylamino-1,2,4-thiadiazol-3-yl)essigsäure in 350 ml Methylenchlorid wird nacheinander mit 26,16 g N,N-Dicyclohexylcarbodiimid, 5,13 ml abs. Methanol und 1,71 g 4-Pyrrolidinopyridin versetzt und während 3 Stunden bei Raumtemperatur gerührt. Nach Zugabe von weiteren 0,5 ml abs. Methanol und 2,6 g N,N-Dicyclohexylcarbodiimid wird für weitere 60 Minuten gerührt. Der Niederschlag wird abgenutscht, das Filtrat nacheinander mit wässriger Natriumbicarbonatlösung, Wasser und wässriger Natriumchloridlösung gewaschen, über Magnesiumsulfat getrocknet und im Vakuum eingeengt. Der Rückstand wird mit Diäthyläther verrieben. Man erhält die einheitliche Titelverbindung vom Rf-Wert 0,50 (Silicagel, Toluol:Aethylacetat 1:1). IR-Spektrum ($CH_2Cl_2$): charakteristische Absorptionsbanden bei 2,90; 5,75; 5,85; 6,50; 8,70 μ.

c) 2-(5-tert.-Butoxycarbonylamino-1,2,4-thiadiazol-3-yl)-2-oxoessigsäure-methylester

Eine Suspension von 20,0 g (73.18 mMol) 2-(5-tert.-Butoxycarbonylamino-1,2,4-thiadiazol-3-yl)essigsäure-methylester und 22,0 g Selendioxid in 330 ml abs. Dioxan wird 3 Stunden bei 95° gerührt. Nach Filtrieren des Reaktionsgemisches wird das Filtrat im Vakuum eingeengt. Der Rückstand wird mit Aethylacetat verdünnt, nacheinander mit Wasser, wässriger Natriumbicarbonatlösung und wässriger Natriumchloridlösung gewaschen, über Magnesiumsulfat getrocknet und eingeengt. Man erhält die Titelverbindung in Form eines Schaumes vom Rf-Wert 0,35 (Silicagel, Toluol:Aethylacetat 1:1). IR-Spektrum ($CH_2Cl_2$): charakteristische Absorptionsbanden bei 2,95; 5,75; 5,85; 6,50 μ.

d) 2-(5-tert.-Butoxycarbonylamino-1,2,4-thiadiazol-3-yl)-2-hydroxyiminoessigsäure-methylester

Eine Suspension von 21,8 g (75,88 mMol) 2-(5-tert.-Butoxycarbonylamino-1,2,4-thiadiazol-3-yl)-2-oxoessigsäuremethylester, 7,48 g Hydroxylaminhydrochlorid und 9,8 ml Pyridin in 300 ml 95% Aethanol wird 3 Stunden bei Raumtemperatur gerührt. Das Reaktionsgemisch wird im Vakuum eingeengt. Der Rückstand wird in Aethylacetat gelöst und die Lösung nacheinander mit verdünnter Salzsäure, Wasser und wässriger Natriumchloridlösung extrahiert, über Magnesiumsulfat getrocknet und im Vakuum eingeengt. Der Rückstand wird aus Diäthyläther kristallisiert. Man erhält die Titelverbindung vom Rf-Wert 0,30 (Silicagel, Chloroform:Methanol 95:5). F. 131-132°. IR-Spektrum ($CH_2Cl_2$): charakteristische Absorptionsbanden bei 2,85; 5,75; 5,85; 6,50; 8,70; 8,90 µ.

e) 2-(5-tert.-Butoxycarbonylamino-1,2,4-thiadiazol-3-yl)-2-hydroxyiminoessigsäure

Eine Lösung von 8,0 g (26,46 mMol) 2-(5-tert.-Butoxycarbonylamino-1,2,4-thiadiazol-3-yl)-2-hydroxyiminoessigsäuremethylester in 175 ml 95% Aethanol wird mit 8,6 g Kaliumhydroxyd und 70 ml Wasser versetzt und während 5 Stunden bei 40° gerührt. Das Reaktionsgemisch wird im Vakuum eingeengt, der Rückstand in Wasser gelöst, die Lösung mit verdünnter Salzsäure auf pH 8,5 gestellt und mit Aethylacetat extrahiert. Die wässrige Phase wird abgetrennt, mit 2 N HCl auf pH 2,0 gestellt und mit Aethylacetat extrahiert. Die organische Phase wird abgetrennt, mit wässriger Natriumchloridlösung gewaschen, über Magnesiumsulfat getrocknet und eingeengt. Der Rückstand wird aus Diäthyläther kristallisiert. Man erhält die einheitliche Titelverbindung vom Rf-Wert 0,60 (Silicagel, s-Butanol/Eisessig/Wasser 67:10:23). F. 158-160° (Zers.); IR-Spektrum (Nujol): charakteristische Absorptionsbanden bei 3,10; 5,85; 8,65 µ.

f) 2-(5-tert.-Butoxycarbonylamino-1,2,4-thiadiazol-3-yl)-2-Z-methylcarbamoyloxyiminoessigsäure

Eine Lösung von 2,85 g (8,95 mMol) 2-(5-tert.-Butoxycarbonylamino-1,2,4-thiadiazol-3-yl)-2-hydroxyiminoessigsäure in 20 ml Tetrahydrofuran wird bei 0-5° mit 10 ml Methylisocyanat versetzt und bei dieser Temperatur während 2 Stunden gerührt. Das Reaktionsgemisch wird mit n-Hexan versetzt und der entstandene Niederschlag abgenutscht. Nach Umkristallisation des Niederschlages aus Methylenchlorid erhält man die einheitliche Titelverbindung vom Rf-Wert 0,25 ($UPC_{12}$-Platten, Wasser/Acetonitril 6:1). F. 124-127°. IR-Spektrum (Nujol): charakteristische Absorptionsbanden bei 5,75; 5,85; 6,40 µ.

Beispiel 10: 7β-[2-(5-Amino-1,2,4-thiadiazol-3-yl)-2-methylcarbamoyloxyimino-acetylamino]-3-cephem-4-carbonsäure Natriumsalz (1) und 7β-[2-(5-Amino-1,2,4-thiadiazol-3-yl)-2-hydroxyimino-acetylamino]-3-cephem-4-carbonsäure Natriumsalz (2)

Aus 3,1 g (4,5 mMol) 7β-[2-(5-tert.-Butyloxycarbonylamino-1,2,4-thiadiazol-3-yl)-2-methylcarbamoyloxyimino-acetylamino]-3-cephem-4-carbonsäure-diphenylmethylester in 7 ml Methylenchlorid, 2,4 ml Anisol und 33 ml Trifluoressigsäure erhält man analog Beispiel 2 die Titelverbindung (1) vom Rf-Wert 0,35 ($UPC_{12}$-Platten, Wasser/Acetonitril 6:1); IR-Spektrum (Nujol): charakteristische Absorptionsbanden bei 2,97; 5,67; 6,25; 7,95 µ,

sowie die Verbindung (2) vom Rf-Wert 0,65 ($UPC_{12}$-Platten, Wasser/Acetonitril 6:1). IR-Spektrum (Nujol): charakteristische Absorptionsbanden bei 3,0; 5,67; 6,25; 9,82 µ.

Verbindung 2 wird auch durch Erwärmen der Verbindung 1 mit Phosphatpufferlösung pH 7,4 auf 45° während 24 Stunden erhalten.

Beispiel 11: 7β-[2-(5-Amino-1,2,4-thiadiazol-3-yl)-2-hydroxyimino-acetylamino]-3-cephem-4-carbonsäure-pivaloyloxymethylester

Aus 1,0 g (2,55 mMol) 7β-[2-(5-Amino-1,2,4-thiadiazol-3-yl)-2-hydroxyimino-acetylamino-3-cephem-4-carbonsäure-Natriumsalz und 0,8 ml (3,42 mMol) Jodmethylpivalat in 20 ml N,N-Dimethylformamid erhält man analog Beispiel 8 die Titelverbindung vom Rf-Wert 0,55 (Silicagel, Aceton/Eisessig/Wasser/Ammoniak 20:8:5:1).

Beispiel 12: 7β-[2-(5-Amino-1,2,4-thiadiazol-3-yl)-2-methylcarbamoyl-oxyimino-acetylamino]-3-(4-carbamoyl-pyridiniomethyl)-3-cephem-4-carboxylat (1) und

7β-[2-(5-Amino-1,2,4-thiadiazol-3-yl)-2-hydroxyimino-acetylamino]-3-(4-carbamoyl-pyridiniomethyl)-3-cephem-4-carboxylat (2)

a) Aus 0,40 g (0,77 mMol) 7β-[2-(5-Amino-1,2,4-thiadiazol-3-yl)-2-methylcarbamoyloxyimino-acetylamino]-3-acetoxymethyl-3-cephem-4-carbonsäure-Natriumsalz, 0,13 g Isonicotinamid und 1,33 g Natriumjodid in 1,3 ml Wasser erhält man analog Beispiel 5 die Titelverbindung (1) vom Rf-Wert 0,45 ($UPC_{12}$-Platten, Wasser/Acetonitril 6:1) und die Titelverbindung (2) vom Rf-Wert 0,65 ($UPC_{12}$-Platten, Wasser/Acetonitril 6:1).

Das Ausgangsmaterial kann wie folgt hergestellt werden:

b) 7β-[2-(5-tert.-Butoxycarbonylamino-1,2,4-thiadiazol-3-yl)-2-Z-methylcarbamoyloxyiminoacetamido]-3-acetoxymethyl-3-cephem-4-carbonsäure-diphenylmethylester

1,05 g (3,70 mMol) 2-(5-tert.-Butoxycarbonylamino-1,2,4-thiadiazol-3-yl)-2-Z-methylcarbamoyloxyiminoessigsäure und 0,4 ml N-Methylmorpholin werden bei -5° zu einer gerührten Suspension eines Vilsmeier-Reagenses (dargestellt aus 0,031 ml Oxalylchlorid und 0,29 ml N,N-Dimethylformamid in 10 ml Aethylacetat) gegeben. Das Gemisch wird während 30 Minuten bei 0° gerührt und anschliessend auf -10° abgekühlt. Nach Zugabe von 1,357 g (3,1 mMol) 7β-Amino-3-acetoxymethyl-3-cephem-4-carbonsäure-diphenylmethylester wird während

2 1/2 Stunden bei 0° gerührt, dann mit Aethylacetat verdünnt und nacheinander mit Wasser, Phosphatpuffer pH 8,0 und wässriger Natriumchloridlösung gewaschen, über Magnesiumsulfat getrocknet und im Vakuum eingeengt. Ein Teil des Rohproduktes wird durch präparative Schichtchromatographie mit Aethylacetat gereinigt. Man erhält die einheitliche Titelverbindung vom Rf-Wert 0,60 (Silicagel, Aethylacetat). IR-Spektrum ($CH_2Cl_2$): charakteristische Absorptionsbanden bei 2,95; 3,10; 5,65; 5,80; 5,95; 8,20 µ.

c) 7β-[2-(5-Amino-1,2,4-thiadiazol-3-yl)-2-Z-methylcarbamoyloxyiminoacetamido]-3-acetoxymethyl-3-cephem-4-carbonsäure-Natriumsalz

Aus 519 g (0,68 mMol) 7β-[2-(5-tert.-Butoxycarbonylamino-1,2,4-thiadiazol-3-yl)-2-Z-methylcarbamoyloxyimino-acetamido]-3-acetoxymethyl-3-cephem-4-carbonsäurediphenylmethylester in 1,1 ml Methylenchlorid, 0,37 ml Anisol und 5 ml Trifluoressigsäure erhält man analog Beispiel 2 die Titelverbindung vom Rf-Wert 0,40 ($UPC_{12}$-Platten, Wasser/Acetonitril 6:1). IR-Spektrum (Nujol): charakteristische Absorptionsbanden bei 3,0; 5,67; 6,22; 8,10 µ.

Beispiel 13: 7β-[2-(5-N-Methylamino-1,2,4-thiadiazol-3-yl)-2-Z-methoxyimino-acetylamino]-3-cephem-4-carbonsäure-Natriumsalz

a) Eine Suspension von 0,432 g (2,0 mMol) 2-(5-N-Methylamino-1,2,4-thiadiazol-3-yl)-2-methoxyiminoessigsäure in 5 ml Methylenchlorid wird bei 0° mit HCl-Gas gesättigt, dann zu einer -5° kalten Suspension eines Vilsmeier-Reagenses (dargestellt aus 0,2 ml Oxalylchlorid und 0,18 ml N,N-Dimethylformamid in 9 ml Methylenchlorid) gegeben und während 30 Minuten bei dieser Temperatur gerührt. Nach der Zugabe einer Lösung von 0,40 g (2,0 mMol) 7β-Amino-3-cephem-4-carbonsäure und 2,2 ml N,O-Bis-(trimethylsilyl)acetamid in 10 ml Methylenchlorid wird während 2 Stunden bei 0° gerührt, dann mit Wasser verdünnt. Die organische Phase wird abgetrennt, über Magnesiumsulfat getrocknet und im Vakuum eingeengt. Nach Chromatographie des Rückstandes (Silicagel "Opti $UPC_{12}$", Wasser/Acetonitril 1:1) erhält man die Titelverbindung vom Rf-Wert 0,45 ($UPC_{12}$-Platten, Wasser Acetonitril 6:1). IP-Spektrum (Nujol): charakteristische Absorptionsbanden bei 3,0; 5,67; 6,25 μ.

Das Ausgangsmaterial kann z.B. wie folgt erhalten werden:

b) <u>5-(N-Methyl-N-tert.-Butoxycarbonylamino)-3-methyl-1,2,4-thiadiazol</u>

Eine Suspension von 15 g (116,3 mMol) 5-N-Methylamino-3-methyl-1,2,4-thiadiazol in 55 ml BOC-Anhydrid wird bei 100° (Badtemperatur) 4 Stunden gerührt. Das Reaktionsgemisch wird abgekühlt, die flüchtigen Anteile werden im Vakuum entfernt und der Rückstand wird chromatographiert (Silicagel, Toluol/steigende Anteile Aethylacetat). Man erhält so 22,3 g der Titelverbindung vom Rf-Wert 0,65 (Silicagel, Toluol/Aethylacetat 1:1). F. 47-48°. IR-Spektrum ($CH_2Cl_2$): charakteristische Absorptionsbanden bei 5,90; 6,55; 8,80 μ.

c) <u>2-(5-N-Methylamino-1,2,4-thiadiazol-3-yl) essigsäure-tert.-butylester</u>

Eine frisch zubereitete Lösung von Lithiumdiisopropylamid in 30 ml abs. Tetrahydrofuran [aus 7,8 ml (55,2 mMol) Diisopropylamin und 30 ml einer ca. 2 N Lösung von Butyllithium in Hexan] wird bei -78° unter Stickstoff mit 3,15 g (13,8 mMol) 5-(N-Methyl-N-tert.-butyloxycarbonylamino)-3-methyl-1,2,4-thiadiazol in 15 ml abs. Tetrahydrofuran versetzt und 50 Minuten bei dieser Temperatur gerührt. Das Reaktionsgemisch wird unter Rühren auf festes $CO_2$ gegossen und während 30 Minuten gerührt. Die Lösung wird mit 60 ml Wasser versetzt und mit Diäthyläther extrahiert. Die wässrige Phase wird mit 2 N Salzsäure auf pH 2,0 eingestellt und mit Aethylacetat reextrahiert. Die vereinigten organischen Phasen werden mit wässriger Natriumchloridlösung gewaschen, über Magnesiumsulfat getrocknet und eingeengt.

Nach Chromatographie des Rückstandes an Silicagel mit Toluol/steigende Anteile Aethylacetat erhält man die einheitliche Titelverbindung mit Rf-Wert 0,50 (Silicagel, Aethylacetat) F. 91-92°. IR-Spektrum ($CH_2Cl_2$): charakteristische Absorptionsbanden bei 2,90; 5,80; 8,65 μ.

d) <u>2-(5-N-Methylamino-1,2,4-thiadiazol-3-yl)-2-oxo-essigsäure-tert.-butylester</u>

Eine Suspension von 2,3 g (10,0 mMol) 2-(5-N-Methylamino-1,2,4-thiadiazol-3-yl)-essigsäure-tert.-butylester und 3,0 g Selendioxid in 50 ml abs. Dioxan wird 3 Stunden bei 90° gerührt. Nach Filtrieren des Reaktionsgemisches wird das Filtrat im Vakuum eingeengt. Der Rückstand wird mit Diäthyläther verrieben. Man erhält so die Titelverbindung vom Rf-Wert 0,55 (Silicagel, Chloroform/Methanol 9:1). IR-Spektrum ($CH_2Cl_2$): charakteristische Absorptionsbanden bei 2,90; 3,10; 5,80; 5,90; 6,25; 9,35: 9,70 μ.

e) <u>2-(5-N-Methylamino-1,2,4-thiadiazol-3-yl)-2-Z-methoxy-imino-essigsäure-tert.-butylester</u>

Eine Suspension von 2,0 g (8,0 mMol) 2-(5-Methylamino-1,2,4-thiadiazol-3-yl)-2-oxoessigsäure-tert.-butylester, 0,736 g (8,64 mMol) Methoxyaminhydrochlorid und 0,72 ml Pyridin in 50 ml 95% Aethanol wird 3 Stunden bei Raumtemperatur gerührt, dann im Vakuum eingeengt. Der Rückstand, der aus den beiden Z- und E-Isomeren der Titelverbindung besteht, wird aus einem Gemisch von Methylenchlorid/Aethylacetat fraktioniert kristallisiert. Man erhält das einheitliche Z-Isomere vom Rf-Wert 0,75 (Silicagel, Chloroform/Methanol 9:1). F. 153-155°. IR-Spektrum ($CH_2Cl_2$): charakteristische Absorptionsbanden bei 2,90; 3,10; 5,80; 6,25; 9,50 u. Durch präaparative Schichtchromatographie der Mutterlauge (Silicagel, Chloroform/Methanol 95:5)

erhält man das E-Isomere vom Rf-Wert 0,70 (Silicagel, Chloroform/Methanol 9:1).

f) 2-(5-N-Methylamino-1,2,4-thiadiazol-3-yl)-2-Z-methoxyiminoessigsäure

Ein Gemisch von 1,5 g (6,24 mMol) 2-(5-N-Methylamino-1,2,4-thiadiazol-3-yl)-2-Z-methoxyiminoessigsäure-tert.-butylester in 15 ml Methylenchlorid wird bei Raumtemperatur mit 15 ml Trifluoressigsäure versetzt und während 40 Minuten gerührt, dann in 200 ml Diäthyläther/Hexan 1:2 eingerührt. Der Niederschlag wird durch Filtration isoliert, in Wasser gelöst und die wässrige Phase mit wässriger Natriumbicarbonatlösung auf pH 5,3 gestellt. Die wässrige Lösung wird zum Teil eingeengt und anschliessend mit abs. Aethanol versetzt. Der ausgefallene Niederschlag wird durch Filtration isoliert und mit Diäthyläther gewaschen. Man erhält die Titelverbindung vom Rf-Wert 0,35 (Silicagel), s-Butanol/Eisessig/Wasser 67:10:23). IR-Spektrum (Nujol): charakteristische Absorptionsbanden bei 2,80; 3,05; 5,85; 6,20; 6,40; 9,50 μ.

Die Verbindung vom Beispiel 13f) kann auch gemäss den folgenden Beispielen 13g) bis 13j) hergestellt werden:

g) 2-(5-N-Methyl-N-tert.-butoxycarbonylamino-1,2,4-thiadiazol-3-yl)-essigsäure-tert.-butylester und 2-(5-N-Methylamino-1,2,4-thiadiazol-3-yl)-essigsäure-tert.-butylester

Eine frisch zubereitete Lösung von Lithiumdiisopropylamid in 200 ml abs. Tetrahydrofuran [aus 26 ml (0,184 Mol) Diisopropylamin und 110 ml einer ca. 2 N Lösung von Butyllithium in Hexan] wird bei -78° unter Stickstoff mit 21,0 g (92,0 mMol) 5-(N-Methyl-N-tert.-butoxycarbonylamino)-3-methyl-1,2,4-thiadiazol in 100 ml abs. Tetrahydrofuran versetzt und 1 Stunde bei dieser Temperatur gerührt, dann auf -20° erwärmt und mit 200 ml Wasser versetzt. Das Gemisch wird mit

Aethylacetat verdünnt, mit Wasser gewaschen, über Magnesiumsulfat getrocknet und im Vakuum eingeengt. Der Rückstand wird chromatographiert (Silicagel, Toluol/steigende Anteile Aethylacetat).

Man erhält den einheitlichen 2-(5-N-Methyl-N-tert.-butoxycarbonylamino-1,2,4-thiadiazol-3-yl)-essigsäure-tert.-butylester vom Rf-Wert 0,80 (Silicagel, Aethylacetat). IR-Spektrum ($CH_2Cl_2$): charakteristische Absorptionsbanden bei 5,78; 5,85; 6,60 μ, sowie einheitlichen 2-(5-N-Methylamino-1,2,4-thiadiazol-3-yl)-essigsäure-tert.-butylester vom Rf-Wert 0,50 (Silicagel, Aethylacetat).

h) <u>2-(5-N-Methyl-N-tert.-butoxycarbonylamino-1,2,4-thiadiazol-3-yl)-2-oxo-essigsäure-tert.-butylester</u>

Aus 5,0 g (15,2 mMol) 2-(5-N-Methyl-N-tert.-butoxycarbonylamino-1,2,4-thiadiazol-3-yl)-essigsäure-tert.-butylester und 4,2 g Selendioxid in 75 ml Dioxan erhält man analog Beispiel 13d) die Titelverbindung vom Rf-Wert 0,70 (Silicagel, Chloroform/Methanol 95:5). F. 96-98°. IR-Spektrum ($CH_2Cl_2$): charakteristische Absorptionsbanden bei 5,80; 5,90; 6,60 μ.

i) <u>2-(5-N-Methyl-N-tert.-butoxycarbonylamino-1,2,4-thiadiazol-3-yl)-2-Z-methoxyimino-essigsäure-tert.-butylester</u>

Aus 6,3 g (18,3 mMol) 2-(5-N-Methyl-N-tert.-butoxycarbonylamino-1,2,4-thiadiazol-3-yl)-2-oxo-essigsäure-tert.-butylester, 1,65 g Methoxyaminhydrochlorid und 1,6 ml Pyridin in 40 ml 95% Aethanol erhält man analog Beispiel 13e) die Titelverbindung vom Rf-Wert 0,80 (Silicagel, Chloroform/Methanol 99:1). F. 125-127°; IR-Spektrum ($CH_2Cl_2$): charakteristische Absorptionsbanden bei 5,78; 5,90; 6,62; 8,90 μ.

j) <u>2-(5-N-Methylamino-1,2,4-thiadiazol-3-yl)-2-Z-methoxyiminoessigsäure</u>

Die Titelverbindung kann auch analog Beispiel 13f) aus

0,5 g (1,34 mMol) 2-(5-N-Methyl-N-tert.-butoxycarbonylamino-1,2,4-thiadiazol-3-yl)-2-Z-methoxyiminoessigsäure-tert.-butylester und 5 ml Trifluoressigsäure erhalten werden.

Beispiel 14: 7β-[2-(5-N-Methylamino-1,2,4-thiadiazol-3-yl)-2-Z-methoxyimino-acetylamino]-3-cephem-4-carbonsäure-pivaloyloxymethylester

Aus 1,05 g (2,5 mMol) 7β-[2-(5-N-Methylamino-1,2,4-thiadiazol-3-yl)-2-Z-methoxyimino-acetylamino]-3-cephem-4-carbonsäure-Natriumsalz und 0,57 ml Jodmethylpivalat in 12 ml N,N-Dimethylformamid erhält man analog Beispiel 8 die Titelverbindung vom Rf-Wert 0,85 (Silicagel, Aceton/Eisessig/Wasser/Ammoniak 20:8:5:1).

Beispiel 15: 7β-[2-(5-tert.-Butyloxycarbonylamino-1,2,4-thiadiazol-3-yl)-2-methoxyimino-acetylamino]-3-methoxy-2- und -3-cephem-4-carbonsäure-diphenylmethylester

1,5 g (5,0 mMol) 2-(5-tert.-Butyloxycarbonylamino-1,2,4-thiadiazol-3-yl)-2-methoxyiminoessigsäure und 0,67 ml N-Methylmorpholin werden bei -10° zu einer gerührten Suspension eines Vilsmeier-Reagenses (dargestellt aus 0,45 ml Oxalylchlorid und 0,47 ml N,N-Dimethylformamid in 21 ml trockenem Aethylacetat) gegeben. Das Gemisch wird bei -10° während 45 Minuten gerührt, anschliessend mit einer Suspension von 2,25 g (5,2 mMol) 7β-Ammonium-3-methoxy-3-cephem-4-carbonsäure-diphenylmethylester-chlorid und 0,58 ml N-Methylmorpholin in 10 ml Aethylacetat versetzt, während 3 1/2 Stunden bei 0° gerührt und dann mit Aethylacetat und Wasser verdünnt. Die organische Phase wird abgetrennt und nacheinander mit Phosphatpufferlösung pH 8,0 und wässriger Natriumchloridlösung gewaschen, über Magnesiumsulfat getrocknet und im Vakuum eingeengt. Man erhält das $\Delta^2$-$\Delta^3$-Isomerengemisch der Titelverbindung. Rf-Wert der $\Delta^2$-Verbindung: 0,70 (Silicagel, Chloroform/Methanol/Eisessig 100:100:0.5). Rf-Wert der $\Delta^3$-Verbindung: 0,40 (Silicagel, Chloroform/Methanol/Eisessig 100:100:0,5).

Beispiel 16: 7β-[2-(5-tert.-Butyloxycarbonylamino-1,2,4-thiadiazol-3-yl)-2-methoxyimino-acetylamino]-3-methoxy-3-cephem-4-carbonsäure-diphenylmethylester-1-oxid

Eine Lösung von 8,0 g (12 mMol) des gemäss Beispiel 15 erhaltenen Isomerengemisches in 130 ml Methylenchlorid wird auf 0° gekühlt, bei dieser Temperatur mit 2,48 g m-Chlorperbenzoesäure (ca. 90%-ig) versetzt und 20 Minuten gerührt. Hierauf wird überschüssige Persäure durch Zugabe von 0,1 N Natriumthiosulfatlösung zerstört. Die Reaktionslösung wird nacheinander mit wässriger Natriumbicarbonatlösung und wässriger Natriumchloridlösung extrahiert, über Magnesiumsulfat getrocknet und eingeengt. Das Rohprodukt wird mit Diäthyläther verrieben. Man erhält die Titelverbindung vom Rf-Wert 0,35 (Silicagel, Chloroform/Methanol 95:5).

Beispiel 17: 7β-[2-(5-Amino-1,2,4-thiadiazol-3-yl)-2-methoxyimino-acetylamino]-3-methoxy-3-cephem-4-carbonsäure-Natriumsalz

Eine Lösung von 2,7 g (3,96 mMol) 7β-[2-(5-tert-Butyloxycarbonylamino-1,2,4-thiadiazol-3-yl)-2-methoxyimino-acetylamino]-3-methoxy-3-cephem-4-carbonsäure-diphenylmethylester-1-oxid in 14 ml Methylenchlorid wird mit 0,65 ml N,N-Dimethylformamid versetzt, auf 0° abgekühlt und mit 0,71 ml Phosphor(III)chlorid versetzt. Nach 25 Minuten Rühren bei 0° wird die Lösung auf -10° abgekühlt, mit 3,35 ml Trifluoressigsäure versetzt und bei 0° während 30 Minuten gerührt. Das Reaktionsgemisch wird mit Aethylacetat verdünnt, nacheinander mit wässriger Kaliumhydrogenphosphatlösung, Wasser und wässriger Natriumchloridlösung gewaschen, über Magnesiumsulfat getrocknet und im Vakuum eingeengt. Der Rückstand wird in 7,5 ml Methylenchlorid suspendiert, mit 32,6 ml Trifluoressigsäure versetzt und während 30 Minuten bei Raumtemperatur gerührt. Die Lösung wird auf 750 ml Hexan/Diäthyläther 2:1 gegossen, der Niederschlag wird abfiltriert, in Methanol gelöst und mit einer methanolischen ca. 3 N Natrium-2-äthylhexanoatlösung auf pH 7,0 gestellt. Nach Zugabe von Diäthyläther wird der gebildete Niederschlag abgenutscht und chromatographiert (Silicagel "Opti $UPC_{12}$",

Wasser). Man erhält die einheitliche Titelverbindung vom Rf-Wert 0,45 ($UPC_{12}$-Platten, Wasser/Acetonitril 9:1).

Beispiel 18: 7β-[2-(5-tert.-Butyloxycarbonylamino-1,2,4-thiadiazol-3-yl)-2-methoxyimino-acetylamino]-3-methoxy-3-cephem-4-carbonsäure

3,0 g (10 mMol) 2-(5-tert.-Butyloxycarbonylamino-1,2,4-thiadiazol-3-yl)-2-methoxyimino-essigsäure und 1,35 ml N-Methylmorpholin werden bei -10° zu einer gerührten Suspension eines Vilsmeier-Reagenses (dargestellt aus 0,9 ml Oxalylchlorid und 0,93 ml N,N-Dimethylformamid in 42 ml trockenem Methylenchlorid) gegeben. Das Gemisch wird bei 0° während 45 Minuten gerührt (Mischung 1). Andererseits wird eine Mischung von 2,40 g (10,4 mMol) 7β-Amino-3-methoxy-3-cephem-4-carbonsäure, 5,8 ml N,O-Bis(trimethylsilyl)acetamid und 30 ml Methylenchlorid während 10 Minuten auf 30° erwärmt und die entstandene Lösung anschliessend auf 0° abgekühlt (Mischung 2). Mischung 2 wird zu der -10° kalten Mischung 1 gegeben. Das Gemisch wird während 4 Stunden bei 0° gerührt, dann mit Wasser und Methylenchlorid verdünnt. Die organische Phase wird abgetrennt, mit wässriger Natriumchloridlösung gewaschen, über Magnesiumsulfat getrocknet und im Vakuum eingeengt. Man erhält die Titelverbindung vom Rf-Wert 0,55 (Silicagel, s-Butanol/Eisessig/Wasser 67:10:23).

Beispiel 19: 7β-[2-(5-Amino-1,2,4-thiadiazol-3-yl)-2-Z-methoxyimino-acetylamino]-3-methoxy-3-cephem-4-carbonsäure-natriumsalz

Aus 4,48 g (8,7 mMol) 7β-[2-(5-tert.-Butyloxycarbonylamino-1,2,4-thiadiazol-3-yl)-2-methoxyimino-acetylamino]-3-methoxy-3-cephem-4-carbonsäure in 14 ml Methylenchlorid und 63 ml Trifluoressigsäure erhält man analog Beispiel 2 die einheitliche Titelverbindung vom Rf-Wert 0,45 ($UPC_{12}$-Platten, Wasser/Acetonitril 9:1).

Beispiel 20: Das Natriumsalz der 7β-[2-(5-Amino-1,2,4-thiadiazol-3-yl)-2-Z-methoxyimino-acetylamino]-3-cephem-4-carbonsäure ergibt durch Umsetzen mit dem Pivalinsäurechlormethylester den 7ß-[2-(5-Amino-1,2,4-thiadiazol-3-yl)-2-Z-methoxyimino-acetylamino]-3-cephem-4-carbonsäure-pivaloyloxymethylester.

Beispiel 21: In analoger Weise kann man unter Verwendung der geeigneten Ausgangsstoffe die folgenden Verbindungen erhalten:

7β-[2-(5-Amino-1,2,4-thiadiazol-3-yl)-2-Z-methoxyimino-acetylamino]-3-chlor-3-cephem-4-carbonsäure oder ein Salz davon;
7β-[2-(5-Amino-1,2,4-thiadiazol-3-yl)-2-Z-methoxyimino-acetylamino]-3-(1-methyl-1H-5-tetrazolyl-thio)-3-cephem-4-carbonsäure oder ein Salz davon;
7β-[2-(5-Amino-1,2,4-thiadiazol-3-yl)-2-hydroxyimino-acetylamino]-3-methoxy-3-cephem-4-carbonsäure oder ein Salz davon;
7β-[2-(5-Amino-1,2,4-thiadiazol-3-yl)-2-Z-methylcarbamoyloxyimino-acetylamino]-3-methoxy-3-cephem-4-carbonsäure oder ein Salz davon;
7β-[2-(5-Amino-1,2,4-thiadiazol-3-yl)-2-Z-carbamoyloxyimino-acetylamino]-3-cephem-4-carbonsäure oder ein Salz davon;
7β-[2-(5-Methylamino-1,2,4-thiadiazol-3-yl)-2-hydroxyimino-acetylamino]-3-cephem-4-carbonsäure-pivaloyloxymethylester oder ein Salz davon; und
7β-[2-(5-Amino-1,2,4-thiadiazol-3-yl)-2-Z-(2-aminoäthyl)oxyimino-acetylamino]-3-(4-carbamoylpyridiniomethyl)-3-cephem-4-carboxylat in der Form des inneren Salzes oder als Säureadditionssalz.

Beispiel 22: Trockenampullen oder Vials, enthaltend 0,5 g Wirksubstanz, z.B. Natriumsalz der 7ß-[2-(5-Amino-1,2,4-thiadiazol-3-yl)-2-Z-methoxyimino-acetylamino]-3-cephem-4-carbonsäure, können z.B. wie folgt hergestellt werden:

Zusammensetzung (für 1 Ampulle oder Vial)

| | |
|---|---|
| Wirksubstanz | 0,5 g |
| Mannit | 0,05 g |

Eine sterile, wässrige Lösung der Wirksubstanz und des Mannits wird unter aseptischen Bedingungen in 5 ml.-Ampullen oder 5 ml.-Vials eingefüllt, und diese verschlossen und geprüft.

Patentansprüche: (für alle benannten Länder ausser Oesterreich)

1. 7β-Aminothiadiazolylacetylamino-3-cephem-4-carbonsäureverbindungen der Formel

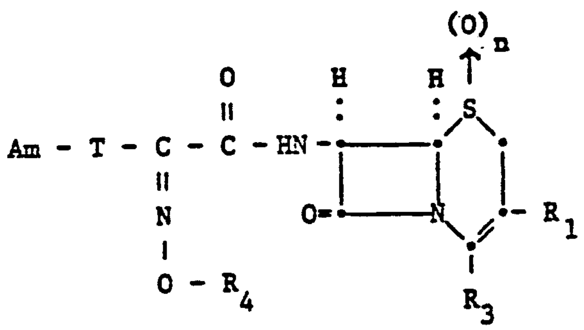

(I),

worin n für 0 oder 1 steht, $R_1$ für Wasserstoff, veräthertes oder verestertes Hydroxy, veräthertes Mercapto oder den Rest der Formel $-CH_2-R_2$ steht, worin $R_2$ Acyl, gegebenenfalls funktionell abgewandeltes Carboxy, eine Ammoniogruppe oder Azido darstellt, $R_3$ Carboxy oder in physiologisch spaltbarer oder geschützter Form vorliegendes funktionell abgewandeltes Carboxy bedeutet, Am für gegebenenfalls substituiertes Amino steht, T einen zweiwertigen, über die Ringkohlenstoffatome mit Am und dem Kohlenstoffatom der Gruppe der Formel $-C(=N-OR_4)-$ verbundenen Thiadiazolrest bedeutet, und $R_4$ Wasserstoff, gegebenenfalls substituiertes Niederalkyl oder Cycloalkyl oder gegebenenfalls N-substituiertes Carbamoyl darstellt, und Salze von solchen Verbindungen, die eine salzbildende Gruppierung aufweisen.

2. Verbindungen der Formel I gemäss Anspruch 1, worin n für 0 oder 1 steht, $R_1$ für Wasserstoff, veräthertes oder verestertes Hydroxy, veräthertes Mercapto oder den Rest der Formel $-CH_2-R_2$ steht, worin $R_2$ Acyl, gegebenenfalls funktionell abgewandeltes Carboxy oder eine Ammoniogruppe darstellt, $R_3$ Carboxy oder in physiologisch spaltbarer oder geschützter Form vorliegendes funktionell abgewandeltes Carboxy bedeutet, Am für gegebenenfalls substituiertes Amino steht, T einen zweiwertigen, über die Ringkohlenstoffatome mit Am und dem Kohlenstoffatom der Gruppe der Formel $-C(=N-OR_4)-$ verbundenen Thiadiazolrest bedeutet, und $R_4$ Wasserstoff, gegebenenfalls

substituiertes Niederalkyl oder Cycloalkyl oder gegebenenfalls N-substituiertes Carbamoyl darstellt, und Salze von solchen Verbindungen, die eine salzbildende Gruppierung aufweisen.

3. Verbindungen der Formel I gemäss Anspruch 1, worin n für 0 steht, $R_1$ für Wasserstoff, Niederalkoxy, Halogen, Niederalkylthio, Heterocyclylthio, worin Heterocyclyl für einen fünf- oder sechsgliedrigen monocyclischen,gegebenenfalls partiell gesättigten, aromatischen Heterocyclylrest steht, der mindestens 1 Ringstickstoffatom und gegebenenfalls 1-3 zusätzliche Ringstickstoffatome und/oder ein Ringsauerstoff- oder Ringschwefelatom enthält, und der durch gegebenenfalls Substituenten, wie Hydroxy, Amino, Diniederalkylamino, Carboxy, Carbamoyl oder Sulfo enthaltendes Niederalkyl, durch gegebenenfalls Substituenten, z.B. Niederalkyl, Niederalkoxy und/oder Halogen, enthaltendes Phenyl, oder durch Thienyl substituiert sein kann, oder für den Rest der Formel $-CH_2-R_2$ steht,worin $R_2$ Niederalkanoyl, Carboxy, Niederalkoxycarbonyl oder gegebenenfalls durch Carbamoyl substituiertes Pyridinio bedeutet, $R_3$ Carboxy, Niederalkanoyloxymethoxycarbonyl , (2-Amino-niederalkanoyl)-oxymethoxycarbonyl oder geschütztes Carboxy darstellt, Am für Amino, Niederalkylamino , Diniederalkylamino, oder für geschütztes Amino steht, T einen zweiwertigen, über die beiden Ringkohlenstoffatome gebundenen Thiadiazolrest bedeutet, und $R_4$ Wasserstoff, gegebenenfalls durch Niederalkoxy, Amino, Diniederalkylamino oder Carboxy substituiertes Niederalkyl oder Cycloalkyl, oder gegebenenfalls N-niederalkyliertes Carbamoyl steht, wobei der Rest der Formel $=N-O-R_4$ die syn(oder Z)- oder anti(oder E)-Form aufweisen kann, sowie Salze von solchen Verbindungen der Formel I, die salzbildende Gruppen aufweisen.

4. Verbindungen der Formel I gemäss Anspruch 2, worin n für 0 oder 1 steht, $R_1$ für Wasserstoff, Niederalkoxy, Halogen, Niederalkylthio, Heterocyclylthio, worin Heterocyclyl für einen fünf- oder sechsgliedrigen monocyclischen, gegebenenfalls partiell gesättigten, aromatischen Heterocyclylrest steht, der mindestens 1 Ringstickstoffatom und gegebenenfalls 1-3 zusätzliche Ringstickstoffatome und/oder ein Ringsauerstoff- oder Ringschwefelatom enthält, und der durch gegebenenfalls Substituenten enthaltendes Niederalkyl oder Phenyl, oder durch Thienyl substituiert sein kann, oder für den Rest der Formel $-CH_2-R_2$ steht, worin $R_2$ Niederalkanoyl, Carboxy, Niederalkoxycarbonyl oder gegebenenfalls durch Carbamoyl substituiertes Pyridinio bedeutet, $R_3$ Carboxy, Niederalkanoyloxymethoxycarbonyl, (2-Amino-niederalkanoyl)-oxymethoxycarbonyl oder geschütztes Carboxy darstellt, Am für Amino, Niederalkylamino, Diniederalkylamino oder für geschütztes Amino steht, T einen zweiwertigen, über die beiden Ringkohlenstoffatome gebundenen Thiadiazolrest bedeutet, und $R_4$ für Wasserstoff, gegebenenfalls durch Niederalkoxy, Diniederalkylamino oder Carboxy substituiertes Niederalkyl oder Cycloalkyl, oder gegebenenfalls N-niederalkyliertes Carbamoyl steht, wobei der Rest der Formel $=N-O-R_4$ die syn(oder Z)- oder anti(oder E)-Form aufweisen kann, sowie Salze von solchen Verbindungen der Formel I, die salzbildende Gruppen aufweisen.

5. Verbindungen der Formel I gemäss Anspruch 1, worin n für O steht, $R_1$ für Wasserstoff, Niederalkoxy, z.B. Methoxy. Halogen, z.B. Chlor, Heterocyclylthio,worin Heterocyclyl gegebenenfalls durch Niederalkyl, z.B. Methyl, substituiertes Oxadiazolyl oder Thiadiazolyl, wie 1,3,4-Oxadiazol-2-yl oder 1,3,4-Thiadiazol-2-yl, gegebenenfalls durch Niederalkyl, z.B. Methyl, substituiertes Triazolyl, wie 1,2,3-Triazol-4-yl, oder gegebenenfalls durch Niederalkyl, z.B. Methyl oder Aethyl, das Diniederalkylamino, z.B. Dimethylamino, Carboxy, Carbamoyl oder Sulfo als Substituenten enthalten kann, substituiertes 1H-Tetrazol-5-yl bedeutet, oder einen Rest der Formel $-CH_2-R_2$ steht, worin $R_2$ gegebenenfalls durch Carbamoyl substituiertes Pyridinio bedeutet, $R_3$ Carboxy, Niederalkanoyloxymethoxycarbonyl, z.B. Pivaloyloxymethoxycarbonyl, oder geschütztes Carboxy darstellt, Am für Amino, Methylamino, oder geschütztes Amino steht, T den zweiwertigen, über die beiden Ringkohlenstoffatome gebundenen 1,2,4-Thiadiazolrest bedeutet, wobei das Ringkohlenstoffatom der 5-Stellung vorzugsweise mit der Gruppe Am verbunden ist, und $R_4$ Was-

serstoff, Niederalkyl, z.B. Methyl, Carboxyniederalkyl, z.B. 2-Carboxy-2-propyl, Carbamoyl, Niederalkylcarbamoyl, z.B. N-Methylcarbamoyl, oder Aminoniederalkyl, z.B. 2-Aminoäthyl, darstellt, wobei der Rest der Formel $=N-O-R_4$ vorzugsweise die syn(oder Z)-Form aufweist, sowie Salze, insbesondere pharmazeutisch verwendbare Salze von solchen Verbindungen der Formel I, die salzbildende Gruppen aufweisen.

6. Verbindungen der Formel I gemäss Anspruch 2, worin n für 0 steht, $R_1$ für Wasserstoff, Niederalkoxy, Halogen, Heterocyclylthio, worin Heterocyclyl gegebenenfalls durch Niederalkyl substituiertes Oxadiazolyl oder Thiadiazolyl, gegebenenfalls durch Niederalkyl substituiertes Triazolyl oder gegebenenfalls durch Niederalkyl, das Diniederalkylamino, Carboxy, Carbamoyl oder Sulfo als Substituenten enthalten kann, substituiertes 1H-Tetrazol-5-yl bedeutet, oder für einen Rest der Formel $-CH_2-R_2$ steht, worin $R_2$ gegebenenfalls durch Carbamoyl substituiertes Pyridinio bedeutet, $R_3$ Carboxy, Niederalkanoyloxymethoxycarbonyl oder geschütztes Carboxy darstellt, Am für Amino oder geschütztes Amino steht, T den zweiwertigen, über die beiden Ringkohlenstoffatome gebundenen 1,2,4-Thiadiazolrest bedeutet, wobei das Ringkohlenstoffatom der 5-Stellung vorzugsweise mit der Gruppe Am verbunden ist, und $R_4$ Wasserstoff oder Niederalkyl darstellt, sowie Salze von solchen Verbindungen der Formel I, die salzbildende Gruppen aufweisen.

7. Verbindungen der Formel I gemäss Anspruch 1, in welcher n für 0 steht, $R_1$ für Wasserstoff, Niederalkoxy, insbesondere Methoxy, Halogen, insbesondere Chlor oder den Rest der Formel $-CH_2R_2$ steht, worin $R_2$ gegebenenfalls Carbamoyl enthaltendes Pyridinio, insbesondere 4-Carbamoylpyridinio, bedeutet, $R_3$ Carboxy oder Niederalkanoyloxymethoxycarbonyl, insbesondere Pivaloyloxymethoxycarbonyl bedeutet, Am Amino oder Methylamino ist, T den zweiwertigen 1,2,4-Thiadiazolrest darstellt, der mit dem Ringkohlenstoffatom der 5-Stellung

mit der Aminogruppe Am und mit dem Ringkohlenstoffatom der 3-Stellung mit der Gruppe der Formel $-C(=N-O-R_4)-$ verbunden ist, und $R_4$ für Wasserstoff, Niederalkyl, insbesondere Methyl, Carboxyniederalkyl, z.B. Carboxy-2-propyl, Carbamoyl oder Niederalkylcarbamoyl, z.B. N-Methylcarbamoyl, steht, wobei die Gruppe der Formel $=N-O-R_4$ die syn(oder Z)-Form aufweist, sowie Salze von solchen Verbindungen der Formel I.

8. Verbindungen der Formel I gemäss Anspruch 2, in welcher n für 0 steht, $R_1$ für Wasserstoff, Niederalkoxy, Halogen, 1-Niederalkyl-1H-5-tetrazolylthio oder den Rest der Formel $-CH_2-R_2$ steht, worin $R_2$ gegebenenfalls Carbamoyl enthaltendes Pyridinio bedeutet, $R_3$ Carboxy oder Niederalkanoyloxymethoxycarbonyl bedeutet, Am Amino ist, T den zweiwertigen 1,2,4-Thiazolrest darstellt, der mit dem Ringkohlenstoffatom der 5-Stellung mit der Aminogruppe Am und mit dem Ringkohlenstoffatom der 3-Stellung mit der Gruppe der Formel $-C(=N-O-R_4)-$ verbunden ist, und $R_4$ für Niederalkyl steht, wobei die Gruppe der Formel $=N-O-R_4$ die syn(oder Z)-Form aufweist, sowie Salze von solchen Verbindungen der Formel I.

9. 7β-[2-(5-Amino-1,2,4-thiadiazol-3-yl)-2-Z-methoxyimino-acetylamino]-3-cephem-4-carbonsäure und Salze davon gemäss Anspruch 2.

10. 7β-[2-(5-Amino-1,2,4-thiadiazol-3-yl) -2-Z-(2-carboxyprop-2-yloxyimino)acetylamino]-3-(4-carbamoylpyridiniomethyl)-3-cephem-4-carboxylat gemäss Anspruch 1.

11. 7β-[2-(5-Amino-1,2,4-thiadiazol-3-yl)-2-Z-methoxyiminoacetylamino]-3-methoxy-3-cephem-4-carbonsäure und Salze davon gemäss Anspruch 1.

12. 7β-[2-(5-Amino-1,2,4-thiadiazol-3-yl)-2-Z-methylcarbamoyloxyiminoacetylamino]-3-cephem-4-carbonsäure und Salze davon gemäss Anspruch 1.

13. 7β-[2-(5-Amino-1,2,4-thiadiazol-3-yl)-2-Z-methylcarbamoyloxyiminoacetylamino]-3-(4-carbamoylpyridiniomethyl)-3-cephem-4-carboxylat gemäss Anspruch 1.

14. 7β-[2-(5-Amino-1,2,4-thiadiazol-3-yl)-2-Z-hydroxyimino-acetylamino]-3-cephem-4-carbonsäure und Salze davon gemäss Anspruch 1.

15. 7β-[2-(5-N-Methylamino-1,2,4-thiadiazol-3-yl)-2-Z-methoxy-imino-acetylamino]-3-cephem-4-carbonsäure und Salze davon gemäss Anspruch 1.

16. Pharmazeutisch verwendbare Präparate enthaltend Verbindungen der Formel I gemäss Anspruch 1 oder pharmazeutisch verwendbare Salze von solchen Verbindungen mit salzbildenden Gruppen.

17. Pharmazeutisch verwendbare Präparate enthaltend Verbindungen der Formel I gemäss Anspruch 2 oder pharmazeutisch verwendbare Salze von solchen Verbindungen mit salzbildenden Gruppen.

18. Verwendung von Verbindungen der Formel I gemäss Anspruch 1 und pharmazeutisch verwendbaren Salzen von solchen Verbindungen mit salzbildenden Gruppen zur Bekämpfung von Mikroorganismen.

19. Verwendung von Verbindungen der Formel I gemäss Anspruch 2 und pharmazeutisch verwendbaren Salzen von solchen Verbindungen mit salzbildenden Gruppen zur Bekämpfung von Mikroorganismen.

20. Verfahren zur Herstellung von Verbindungen der Formel I gemäss Anspruch 1 und Salzen von solchen Verbindungen, die eine salzbildende Gruppe aufweisen, dadurch gekennzeichnet, dass man

a) in einer Verbindung der Formel

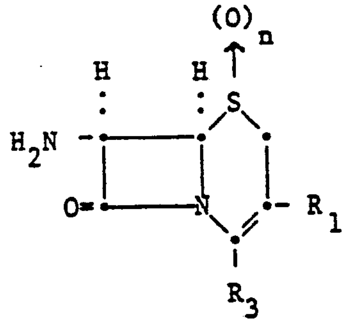

(II),

worin die 7β-Aminogruppe gegebenenfalls durch eine, die Acylierung erlaubende Gruppe geschützt ist, und worin weitere gegebenenfalls vorhandene funktionelle Gruppen geschützt sein können, die 7β-Aminogruppe durch Behandeln mit einem, den Acylrest einer Carbonsäure der Formel

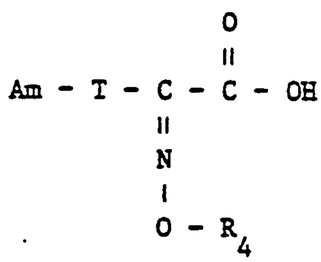

(III)

einführenden Acylierungsmittel, worin weitere, gegebenenfalls vorhandene funktionelle Gruppen in geschützter Form vorliegen können, acyliert, oder

b) eine 2-Cephemverbindung der Formel

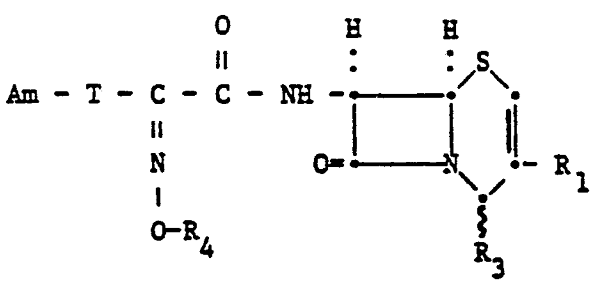

(IV)

worin gegebenenfalls vorhandene funktionelle Gruppen in geschützter Form vorliegen können, zur entsprechenden 3-Cephemverbindung isomerisiert, oder

c) eine Verbindung der Formel

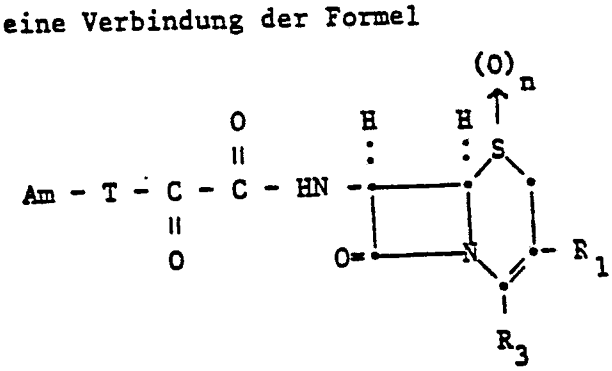

(V)

worin gegebenenfalls vorhandene funktionelle Gruppen in geschützter Form vorliegen können, mit einem gegebenenfalls O-substituierten Hydroxylamin der Formel $H_2N-O-R_4$ (VI) umsetzt, oder

d) zur Herstellung einer Verbindung der Formel I, worin $R_4$ Wasserstoff bedeutet, eine Verbindung der Formel

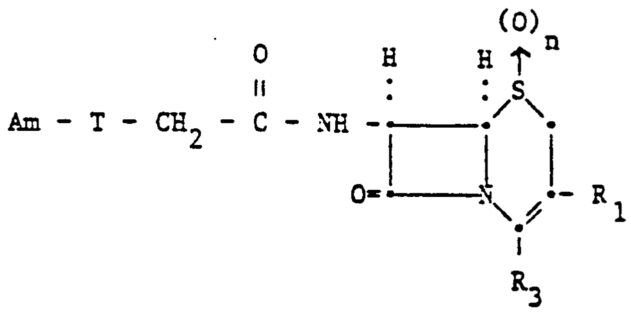

(VII),

worin gegebenenfalls vorhandene funktionelle Gruppen in geschütz- Form vorliegen können, mit einem Nitrosierungsmittel behandelt, oder

e) zur Herstellung einer Verbindung der Formel I, worin Am eine freie primäre oder sekundäre Aminogruppe bedeutet, und T für den zweiwertigen 1,2,4-Thiadiazolrest steht, dessen 5-Stellung mit der Aminogruppe Am und dessen 3-Stellung mit der Gruppe der Formel $-C(=N-O-R_4)-$ verbunden ist, eine Verbindung der Formel

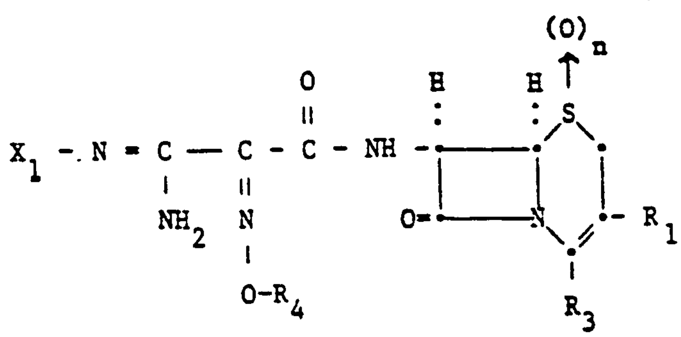

(VIII)

worin weitere, gegebenenfalls vorhandene funktionelle Gruppen in geschützter Form vorliegen können, und worin $X_1$ Halogen oder Hydroxy bedeutet, mit einem Salz der Rhodanwasserstoffsäure bzw. mit einem Isorhodanwasserstoffsäureester, oder worin $X_1$ Wasserstoff ist, mit Rhodan behandelt, oder

f) zur Herstellung einer Verbindung der Formel I, worin $R_1$ für Wasserstoff steht,

fa) in einer Verbindung der Formel

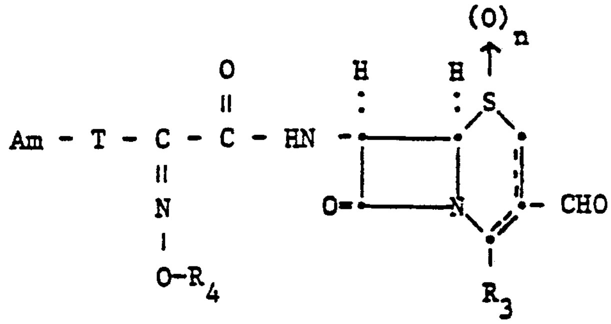

(IX)

worin die Doppelbindung in 2,3- oder in 3,4-Stellung sein kann, und worin gegebenenfalls vorhandene funktionelle Gruppen in geschützter Form vorliegen können, die Formylgruppe mittels Decarbonylierung durch Wasserstoff ersetzt, oder

fb) in einer Verbindung der Formel

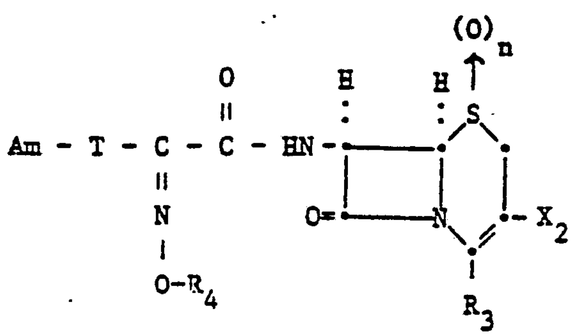

(X)

worin $X_2$ Hydroxy oder gegebenenfalls substituiertes Amino darstellt, und worin weitere gegebenenfalls vorhandene funktionelle Gruppen in geschützter Form vorliegen können, oder eine entsprechende tautomere Verbindung davon, $X_2$ durch Wasserstoff ersetzt, oder

fc) eine Verbindung der Formel

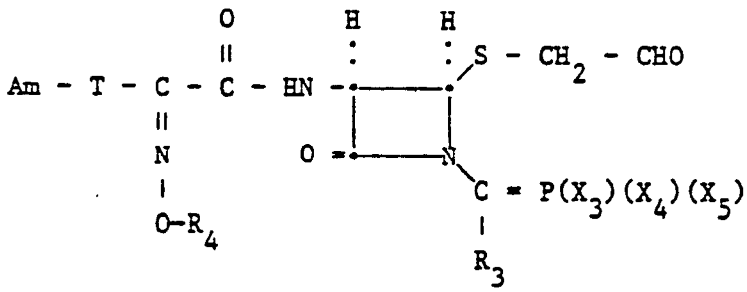

(XI)

worin $X_3$, $X_4$ und $X_5$ jeweils gegebenenfalls substituierte Kohlenwasserstoffreste darstellen, und worin weitere gegebenenfalls vorhandene funktionelle Gruppen in geschützter Form vorliegen, unter Abspaltung einer Verbindung der Formel $O = P(X_3)(X_4)(X_5)$ (XII) ringschliesst, oder

g) zur Herstellung einer Verbindung der Formel I, worin $R_1$ für veräthertes oder verestertes Hydroxy steht,

ga) eine Verbindung der Formel

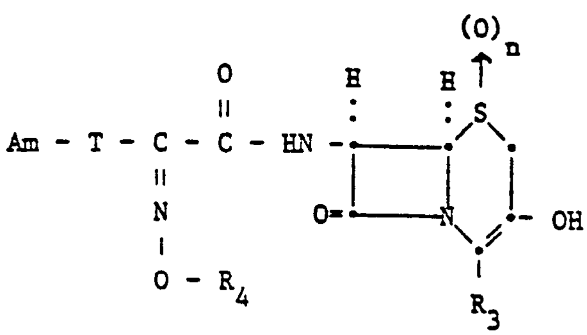

(XIII)

oder die entsprechende Tautomere 3-Oxo-cepham-Verbindung, worin $R_3$ funktionell abgewandeltes Carboxyl darstellt, und weitere gegebenenfalls vorhandene funktionelle Gruppen in geschützter Form vorliegen können, mit einem veräthernden oder veresternden Mittel behandelt, oder

gb) eine Verbindung der Formel

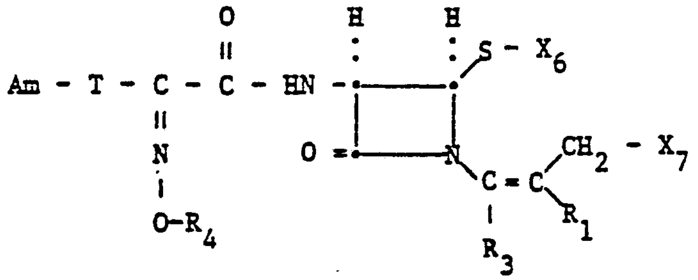

(XIV)

worin $X_6$ eine Abgangsgruppe darstellt, $X_7$ Wasserstoff oder Halogen bedeutet, und $R_1$ für veräthertes oder verestertes Hydroxy steht, und worin weitere gegebenenfalls vorhandene funktionelle Gruppen in geschützter Form vorliegen können, unter Abspaltung der Gruppen $X_6$ und $X_7$ ringschliesst, oder

h) zur Herstellung einer Verbindung der Formel I, worin $R_1$ den Rest der Formel $-CH_2-R_2$ darstellt, worin $R_2$ eine Ammoniogruppe bedeutet, eine Verbindung der Formel

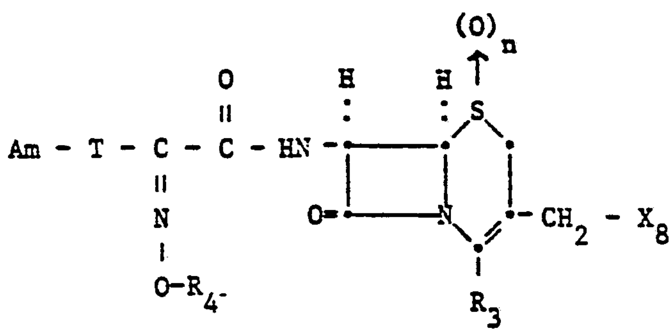

(XV)

worin $X_8$ einen durch nucleophile Substitution ersetzbaren Rest darstellt, und worin gegebenenfalls vorhandene funktionelle Gruppen in geschützter Form vorliegen können, mit einer organischen tertiären Aminbase umsetzt, oder

i) zur Herstellung einer Verbindung der Formel I, worin $R_1$ für den Rest der Formel $-CH_2-R_2$ darstellt, worin $R_2$ Acyl oder gegebenenfalls funktionell abgewandeltes Carboxy darstellt, eine Verbindung der Formel XIII oder eine entsprechende tautomere 3-Oxo-cepham-Ver-

bindung, worin die Gruppe $R_3$ als funktionell abgewandelte Carboxylgruppe vorliegt, und worin andere, gegebenenfalls vorhandene funktionelle Gruppen in geschützter Form vorliegen können, mit einem Ylid der Formel $X_9^{\oplus}-^{\ominus}CH-R_2$ (XVI), worin $X_9^{\oplus}$ eine dreifach substituierte Phosphoniumgruppe oder eine zweifach veresterte Phosphonogruppe zusammen mit einem Kation bedeutet, und worin $R_2$ für Acyl oder eine gegebenenfalls funktionell abgewandelte Carboxylgruppe steht, umsetzt,

j) zur Herstellung von Verbindungen der Formel I, worin Am eine primäre, sekundäre oder tertiäre Aminogruppe ist, eine Verbindung der Formel

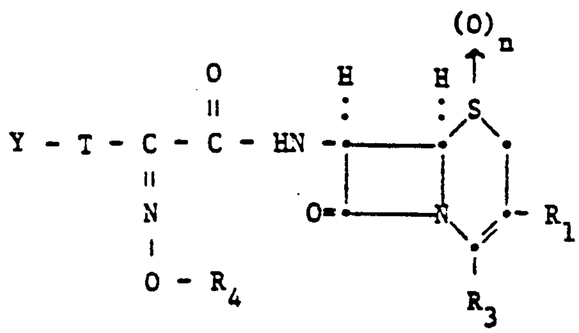

(XVII) ,

worin Y Halogen ist und $R_1$, $R_3$ und $R_4$ die oben angegebenen Bedeutungen haben, mit Ammoniak oder einem primären oder sekundären Amin Am-H, oder einem Metallamid davon, umsetzt, und, wenn erwünscht, eine erfindungsgemäss erhältliche Verbindung der Formel I in eine andere Verbindung der Formel I umwandelt, und/oder, wenn erwünscht oder notwendig, in einer erfindungsgemäss erhältlichen Verbindung der Formel I eine in geschützter Form vorliegende funktionelle Gruppe in die freie funktionelle Gruppe überführt, und/oder,
wenn erwünscht, ein erfindungsgemäss erhältliches Salz in die freie Verbindung oder in ein anderes Salz überführt, und/oder, wenn erwünscht, eine erfindungsgemäss erhältliche freie Verbindung mit einer salzbildenden Gruppe in ein Salz überführt, und/oder, wenn erwünscht, ein erfindungsgemäss erhältliches Gemisch von isomeren Verbindungen der Formel I in die einzelnen Isomeren auftrennt.

21. Verfahren zur Herstellung von Verbindungen der Formel I gemäss Anspruch 2 und Salzen von solchen Verbindungen mit salzbildenden Gruppen, dadurch gekennzeichnet, dass man

a) in einer Verbindung der Formel

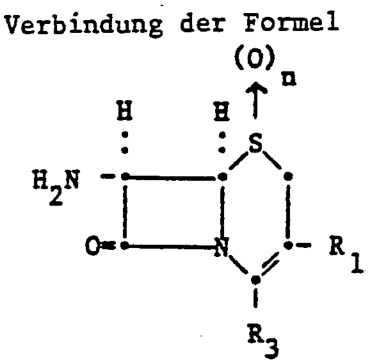

(II),

worin die 7β-Aminogruppe gegebenenfalls durch eine, die Acylierung erlaubende Gruppe geschützt ist, und worin weitere gegebenenfalls vorhandene funktionelle Gruppen geschützt sein können, die 7β-Aminogruppe durch Behandeln mit einem, den Acylrest einer Carbonsäure der Formel

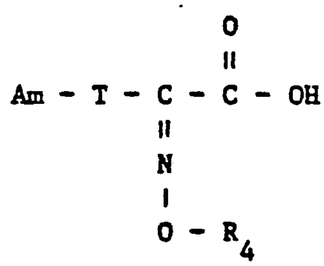

(III)

einführenden Acylierungsmittel, worin weitere, gegebenenfalls vorhandene funktionelle Gruppen in geschützter Form vorliegen können, acyliert, oder

b) eine 2-Cephemverbindung der Formel

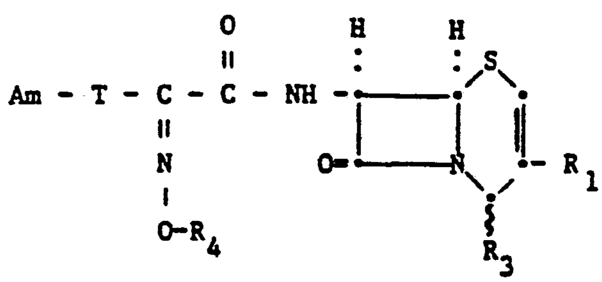

(IV)

worin gegebenenfalls vorhandene funktionelle Gruppen in geschützter Form vorliegen können, zur entsprechenden 3-Cephem-Verbindung isomerisiert, oder

c) eine Verbindung der Formel

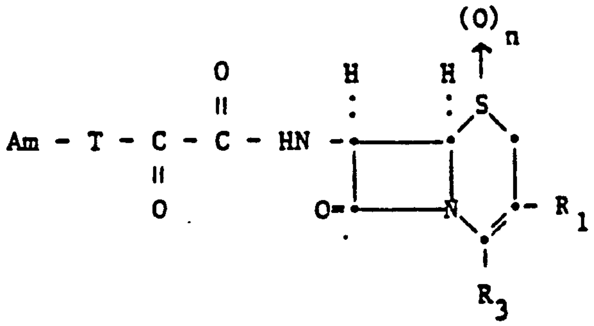

(V)

worin gegebenenfalls vorhandene funktionelle Gruppen in geschützter Form vorliegen können, mit einem gegebenenfalls O-substituierten Hydroxylamin der Formel $H_2N-O-R_4$ (VI) umsetzt, oder

d) zur Herstellung einer Verbindung der Formel I, worin $R_4$ Wasserstoff bedeutet, eine Verbindung der Formel

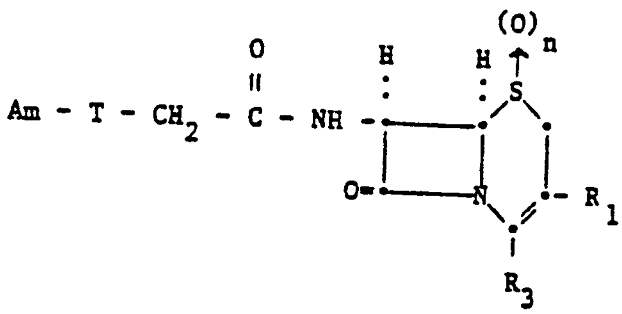

(VII),

worin gegebenenfalls vorhandene funktionelle Gruppen in geschütz- Form vorliegen können, mit einem Nitrosierungsmittel behandelt, oder

e) zur Herstellung einer Verbindung der Formel I, worin Am eine freie Aminogruppe bedeutet, und T für den zweiwertigen 1,2,4-Thiadiazolrest darstellt, dessen 5-Stellung mit der Aminogruppe Am und dessen 3-Stellung mit der Gruppe der Formel $-C(=N-O-R_4)-$ verbunden ist, eine Verbindung der Formel

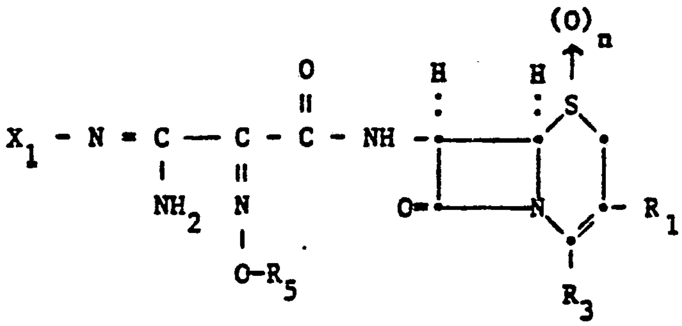

(VIII)

worin weitere, gegebenenfalls vorhandene funktionelle Gruppen in geschützter Form vorliegen können, und worin $X_1$ Halogen bedeutet, mit einem Salz der Rhodanwasserstoffsäure, oder worin $X_1$ Wasserstoff ist, mit Rhodan behandelt, oder

f) zur Herstellung einer Verbindung der Formel I, worin $R_1$ für Wasserstoff steht,

fa) in einer Verbindung der Formel

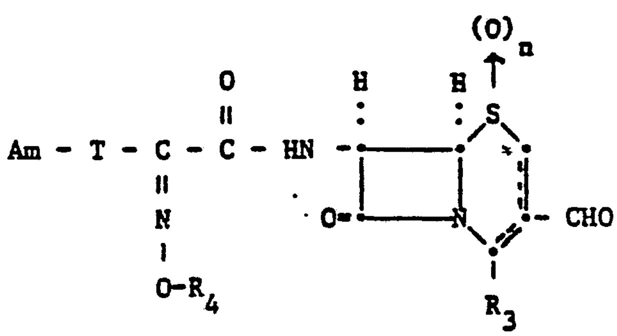

(IX)

worin die Doppelbindung in 2,3- oder in 3,4-Stellung sein kann, und worin gegebenenfalls vorhandene funktionelle Gruppen in geschützter Form vorliegen können, die Formylgruppe mittels Decarbonylierung durch Wasserstoff ersetzt, oder

fb) in einer Verbindung der Formel

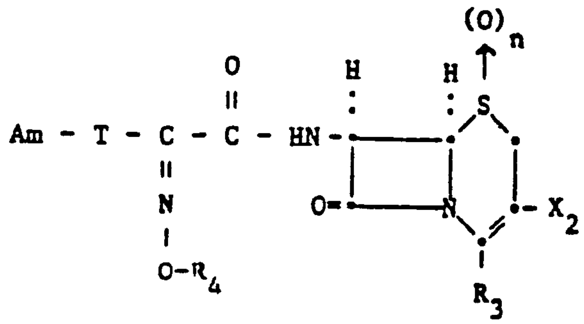

(X)

worin $X_2$ Hydroxy oder gegebenenfalls substituiertes Amino darstellt, und worin weitere gegebenenfalls vorhandene funktionelle Gruppen in geschützter Form vorliegen können, oder eine entsprechende tautomere Verbindung davon, $X_2$ durch Wasserstoff ersetzt, oder

fc) eine Verbindung der Formel

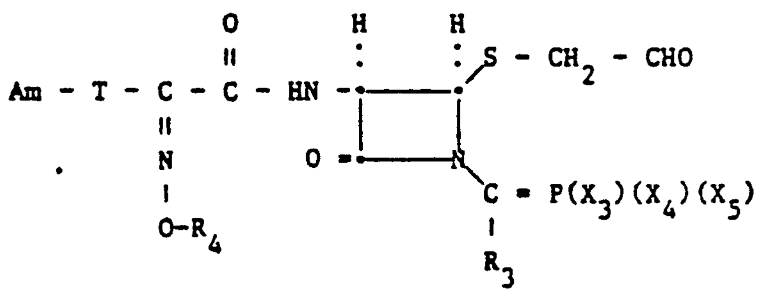

(XI)

worin $X_3$, $X_4$ und $X_5$ jeweils gegebenenfalls substituierte Kohlenwasserstoffreste darstellen, und worin weitere gegebenenfalls vorhandene funktionelle Gruppen in geschützter Form vorliegen, unter Abspaltung einer Verbindung der Formel $O = P(X_3)(X_4)(X_5)$ (XII) ringschliesst, oder

g) zur Herstellung einer Verbindung der Formel I, worin $R_1$ für veräthertes oder verestertes Hydroxy steht,

ga) eine Verbindung der Formel

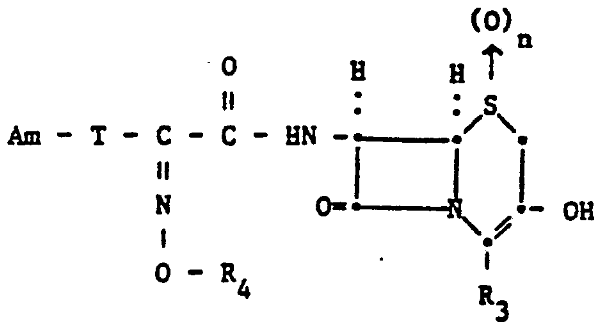

(XIII)

oder die entsprechende Tautomere 3-Oxo-cepham-Verbindung, worin $R_3$ funktionell abgewandeltes Carboxyl darstellt, und weitere gegebenenfalls vorhandene funktionelle Gruppen in geschützter Form vorliegen können, mit einem veräthernden oder veresternden Mittel behandelt, oder

gb) eine Verbindung der Formel

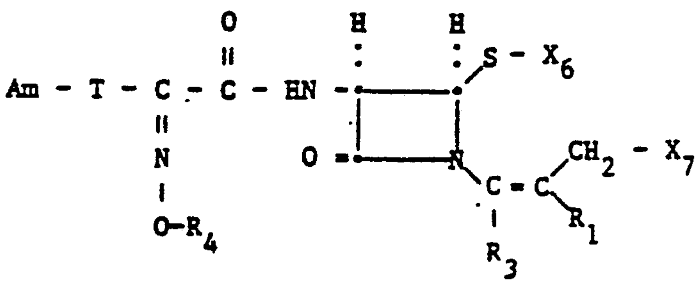

(XIV)

worin $X_6$ eine Abgangsgruppe darstellt, $X_7$ Wasserstoff oder Halogen bedeutet, und $R_1$ für veräthertes oder verestertes Hydroxy steht, und worin weitere gegebenenfalls vorhandene funktionelle Gruppen in geschützter Form vorliegen können, unter Abspaltung der Gruppen $X_6$ und $X_7$ ringschliesst, oder

h) zur Herstellung einer Verbindung der Formel I, worin $R_1$ den Rest der Formel $-CH_2-R_2$ darstellt, worin $R_2$ eine Ammoniogruppe bedeutet, eine Verbindung der Formel

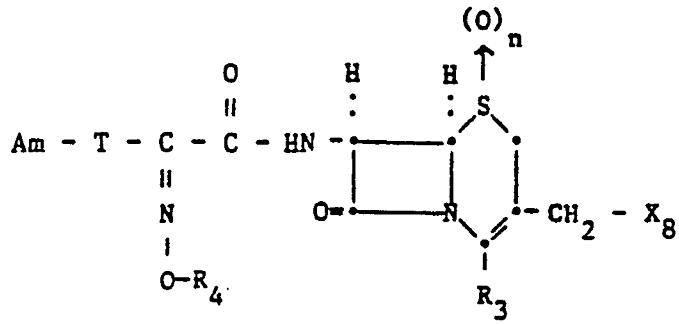

(XV)

worin $X_8$ einen durch nucleophile Substitution ersetzbaren Rest darstellt, und worin gegebenenfalls vorhandene funktionelle Gruppen in geschützter Form vorliegen können, mit einer organischen tertiären Aminbase umsetzt, oder

i) zur Herstellung einer Verbindung der Formel I, worin $R_1$ für den Rest der Formel $-CH_2-R_2$ darstellt, worin $R_2$ Acyl oder gegebenenfalls funktionell abgewandeltes Carboxy darstellt, eine Verbindung der Formel XIII oder eine entsprechende tautomere 3-Oxo-cepham-Verbindung, worin die Gruppe $R_3$ als funktionell abgewandelte Carboxylgruppe vorliegt, und worin andere, gegebenenfalls vorhandene funktionelle Gruppen in geschützter Form vorliegen können, mit einem Ylid der Formel $X_9^{\oplus}\text{-}^{\ominus}CH\text{-}R_2$ (XVI), worin $X_9^{\oplus}$ eine dreifach substituierte Phosphoniumgruppe oder eine zweifach veresterte Phosphonogruppe zusammen mit einem Kation bedeutet, und worin $R_2$ für Acyl oder eine gegebenenfalls funktionell abgewandelte Carboxylgruppe steht, umsetzt, und wenn erwünscht, eine erfindungsgemäss erhältliche Verbindung der Formel I in eine andere Verbindung der Formel I umwandelt, und/oder, wenn erwünscht oder notwendig, in einer erfindungsgemäss erhältlichen Verbindung der Formel I eine in geschützter Form vorliegende funktionelle Gruppe in die freie funktionelle Gruppe überführt, und/oder, wenn erwünscht, ein erfindungsgemäss erhältliches Salz in die freie Verbindung oder in ein anderes Salz überführt, und/oder, wenn erwünscht, eine erfindungsgemäss erhältliche freie Verbindung mit einer salzbildenden Gruppe in ein Salz überführt, und/oder, wenn erwünscht, ein erfindungsgemäss erhältliches Gemisch von isomeren Verbindungen der Formel I in die einzelnen Isomeren auftrennt.

Patentansprüche: (für Oesterreich)

1. Verfahren zur Herstellung von 7β-Aminothiadiazolylacetylamino-3-cephem-4-carbonsäureverbindungen der Formel

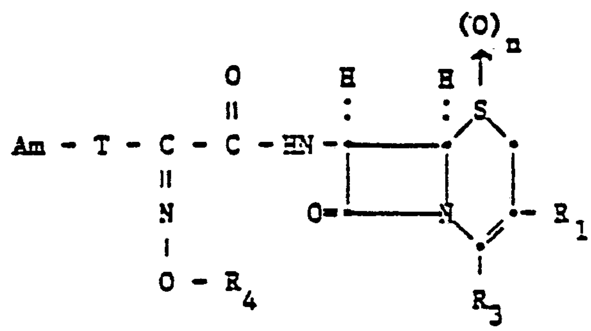

(I),

worin n für 0 oder 1 steht, $R_1$ für Wasserstoff, veräthertes oder verestertes Hydroxy, veräthertes Mercapto oder den Rest der Formel $-CH_2-R_2$ steht, worin $R_2$ Acyl, gegebenenfalls funktionell abgewandeltes Carboxy, eine Ammoniogruppe oder Azido darstellt, $R_3$ Carboxy oder in physiologisch spaltbarer oder geschützter Form vorliegendes funktionell abgewandeltes Carboxy bedeutet, Am für gegebenenfalls substituiertes Amino steht, T einen zweiwertigen, über die Ringkohlenstoffatome mit Am und dem Kohlenstoffatom der Gruppe der Formel $-C(=N-OR_4)-$ verbundenen Thiadiazolrest bedeutet, und $R_4$ Wasserstoff, gegebenenfalls substituiertes Niederalkyl oder Cycloalkyl oder gegebenenfalls N-substituiertes Carbamoyl darstellt, und Salzen von solchen Verbindungen, die eine salzbildende Gruppierung aufweisen, dadurch gekennzeichnet, dass man

a) in einer Verbindung der Formel

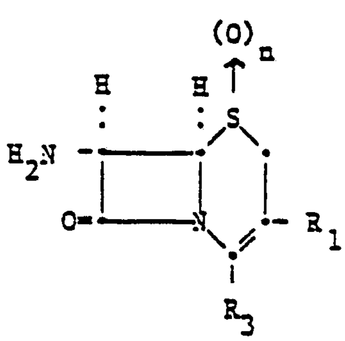

(II),

worin die 7β-Aminogruppe gegebenenfalls durch eine, die Acylierung erlaubende Gruppe geschützt ist, und worin weitere gegebenenfalls vorhandene funktionelle Gruppen geschützt sein können, die 7β-Aminogruppe durch Behandeln mit einem, den Acylrest einer Carbonsäure der Formel

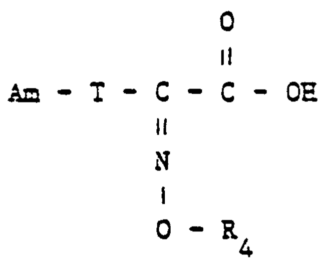

(III)

einführenden Acylierungsmittel, worin weitere, gegebenenfalls vorhandene funktionelle Gruppen in geschützter Form vorliegen können, acyliert, oder

b) eine 2-Cephemverbindung der Formel

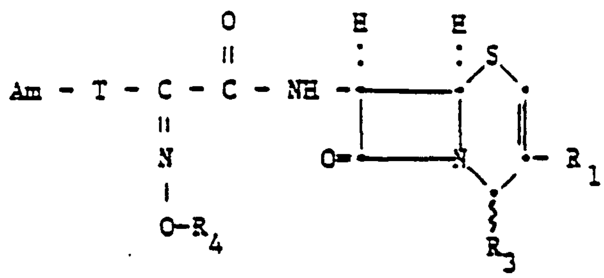

(IV)

worin gegebenenfalls vorhandene funktionelle Gruppen in geschützter Form vorliegen können, zur entsprechenden 3-Cephemverbindung isomerisiert, oder

c) eine Verbindung der Formel

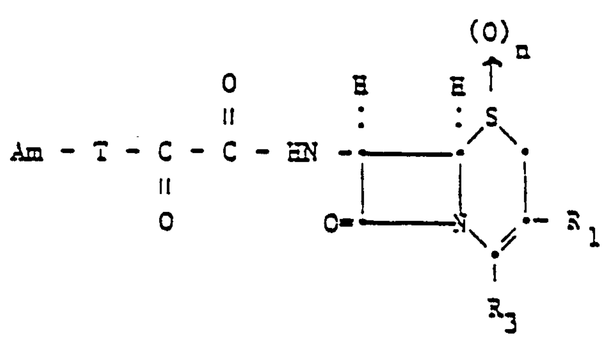

(V)

worin gegebenenfalls vorhandene funktionelle Gruppen in geschützter Form vorliegen können, mit einem gegebenenfalls O-substituierten Hydroxylamin der Formel $H_2N-O-R_4$ (VI) umsetzt, oder

d) zur Herstellung einer Verbindung der Formel I, worin $R_4$ Wasserstoff bedeutet, eine Verbindung der Formel

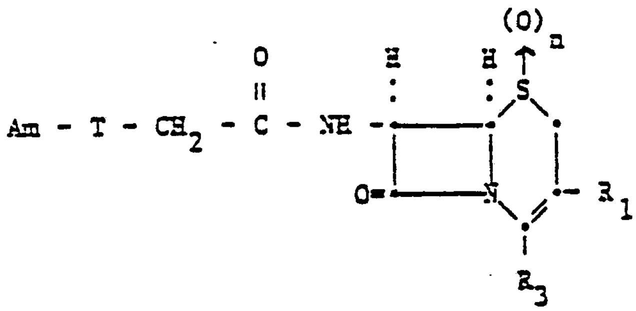

(VII),

worin gegebenenfalls vorhandene funktionelle Gruppen in geschütz- Form vorliegen können, mit einem Nitrosierungsmittel behandelt, oder

e) zur Herstellung einer Verbindung der Formel I, worin Am eine freie primäre oder sekundäre Aminogruppe bedeutet, und T für den zweiwertigen 1,2,4-Thiadiazolrest steht, dessen 5-Stellung mit der Aminogruppe Am und dessen 3-Stellung mit der Gruppe der Formel $-C(=N-O-R_4)-$ verbunden ist, eine Verbindung der Formel

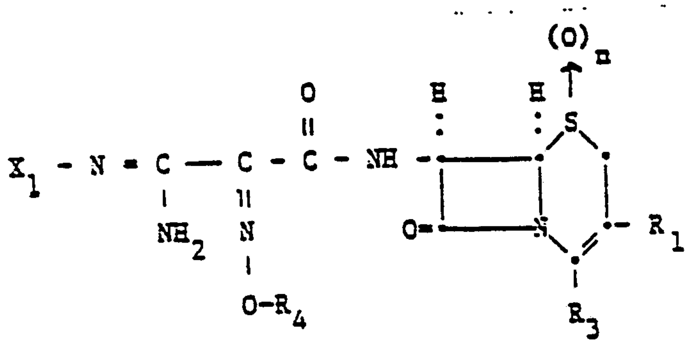

(VIII)

worin weitere, gegebenenfalls vorhandene funktionelle Gruppen in geschützter Form vorliegen können, und worin $X_1$ Halogen oder Hydroxy bedeutet, mit einem Salz der Rhodanwasserstoffsäure bzw. mit einem Isorhodanwasserstoffsäureester, oder worin $X_1$ Wasserstoff ist, mit Rhodan behandelt, oder

f) zur Herstellung einer Verbindung der Formel I, worin $R_1$ für Wasserstoff steht,

fa) in einer Verbindung der Formel

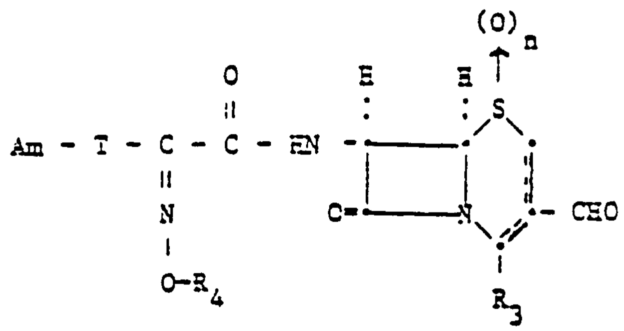

(IX)

worin die Doppelbindung in 2,3- oder in 3,4-Stellung sein kann, und worin gegebenenfalls vorhandene funktionelle Gruppen in geschützter Form vorliegen können, die Formylgruppe mittels Decarbonylierung durch Wasserstoff ersetzt, oder

fb) in einer Verbindung der Formel

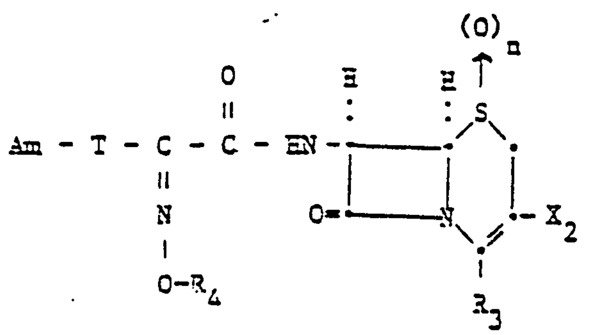

(X)

worin $X_2$ Hydroxy oder gegebenenfalls substituiertes Amino darstellt, und worin weitere gegebenenfalls vorhandene funktionelle Gruppen in geschützter Form vorliegen können, oder eine entsprechende tautomere Verbindung davon, $X_2$ durch Wasserstoff ersetzt, oder

fc) eine Verbindung der Formel

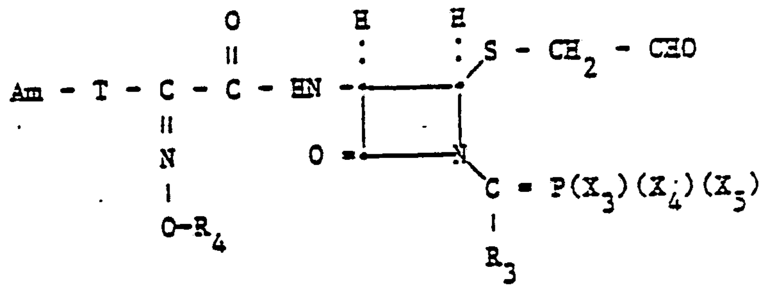

(XI)

worin $X_3$, $X_4$ und $X_5$ jeweils gegebenenfalls substituierte Kohlenwasserstoffreste darstellen, und worin weitere gegebenenfalls vorhandene funktionelle Gruppen in geschützter Form vorliegen, unter Abspaltung einer Verbindung der Formel $O = P(X_3)(X_4)(X_5)$ (XII) ringschliesst, oder

g) zur Herstellung einer Verbindung der Formel I, worin $R_1$ für veräthertes oder verestertes Hydroxy steht,

ga) eine Verbindung der Formel

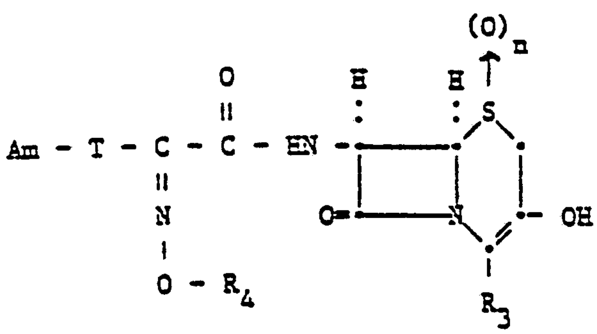

(XIII)

oder die entsprechende Tautomere 3-Oxo-cepham-Verbindung, worin $R_3$ funktionell abgewandeltes Carboxyl darstellt, und weitere gegebenenfalls vorhandene funktionelle Gruppen in geschützter Form vorliegen können, mit einem veräthernden oder veresternden Mittel behandelt, oder

gb) eine Verbindung der Formel

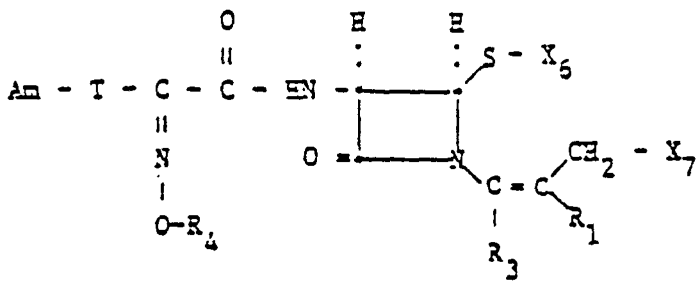

(XIV)

worin $X_6$ eine Abgangsgruppe darstellt, $X_7$ Wasserstoff oder Halogen bedeutet, und $R_1$ für veräthertes oder verestertes Hydroxy steht, und worin weitere gegebenenfalls vorhandene funktionelle Gruppen in geschützter Form vorliegen können, unter Abspaltung der Gruppen $X_6$ und $X_7$ ringschliesst, oder

h) zur Herstellung einer Verbindung der Formel I, worin $R_1$ den Rest der Formel $-CH_2-R_2$ darstellt, worin $R_2$ eine Ammoniogruppe bedeutet, eine Verbindung der Formel

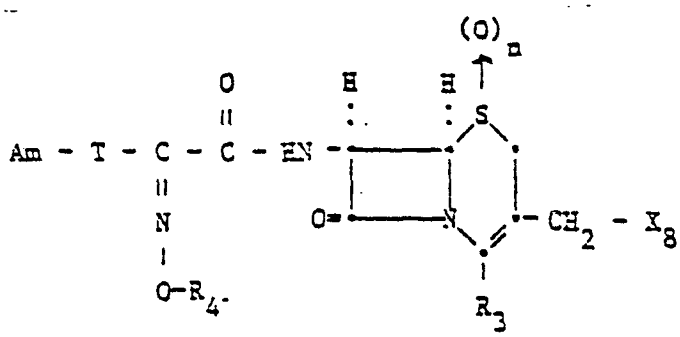

(XV)

worin $X_8$ einen durch nucleophile Substitution ersetzbaren Rest darstellt, und worin gegebenenfalls vorhandene funktionelle Gruppen in geschützter Form vorliegen können, mit einer organischen tertiären Aminbase umsetzt, oder

i) zur Herstellung einer Verbindung der Formel I, worin $R_1$ für den Rest der Formel $-CH_2-R_2$ darstellt, worin $R_2$ Acyl oder gegebenenfalls funktionell abgewandeltes Carboxy darstellt, eine Verbindung der Formel XIII oder eine entsprechende tautomere 3-Oxo-cepham-Ver-

bindung, worin die Gruppe $R_3$ als funktionell abgewandelte Carboxylgruppe vorliegt, und worin andere, gegebenenfalls vorhandene funktionelle Gruppen in geschützter Form vorliegen können, mit einem Ylid der Formel $X_9^{\ominus}-^{\ominus}CH-R_2$ (XVI), worin $X_9^{\ominus}$ eine dreifach substituierte Phosphoniumgruppe oder eine zweifach veresterte Phosphonogruppe zusammen mit einem Kation bedeutet, und worin $R_2$ für Acyl oder eine gegebenenfalls funktionell abgewandelte Carboxylgruppe steht, umsetzt,

j) zur Herstellung von Verbindungen der Formel I, worin Am eine primäre, sekundäre oder tertiäre Aminogruppe ist, eine Verbindung der Formel

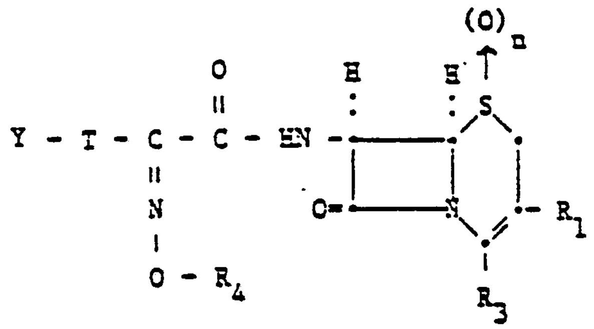

(XVII) ,

worin Y Halogen ist und $R_1$, $R_3$ und $R_4$ die oben angegebenen Bedeutungen haben, mit Ammoniak oder einem primären oder sekundären Amin Am-H, oder einem Metallamid davon, umsetzt, und, wenn erwünscht, eine erfindungsgemäss erhältliche Verbindung der Formel I in eine andere Verbindung der Formel I umwandelt, und/oder, wenn erwünscht oder notwendig, in einer erfindungsgemäss erhältlichen Verbindung der Formel I eine in geschützter Form vorliegende funktionelle Gruppe in die freie funktionelle Gruppe überführt, und/oder,
wenn erwünscht, ein erfindungsgemäss erhältliches Salz in die freie Verbindung oder in ein anderes Salz überführt, und/oder, wenn erwünscht, eine erfindungsgemäss erhältliche freie Verbindung mit einer salzbildenden Gruppe in ein Salz überführt, und/oder, wenn erwünscht, ein erfindungsgemäss erhältliches Gemisch von isomeren Verbindungen der Formel I in die einzelnen Isomeren auftrennt.

2. Verfahren zur Herstellung von Verbindungen der Formel I gemäss Anspruch 1, worin n für 0 oder 1 steht, $R_1$ für Wasserstoff, veräthertes oder verestertes Hydroxy, veräthertes Mercapto oder den Rest der Formel $-CH_2-R_2$ steht, worin $R_2$ Acyl, gegebenenfalls funktionell abgewandeltes Carboxy oder eine Ammoniogruppe darstellt, $R_3$ Carboxy oder in physiologisch spaltbarer oder geschützter Form vorliegendes funktionell abgewandeltes Carboxy bedeutet, Am für gegebenenfalls substituiertes Amino steht, T einen zweiwertigen, über die Ringkohlenstoffatome mit Am und dem Kohlenstoffatom der Gruppe der Formel $-C(=N-OR_4)-$ verbundenen Thiadiazolrest bedeutet, und $R_4$ Wasserstoff, gegebenenfalls substituiertes Niederalkyl oder Cycloalkyl oder gegebenenfalls N-substituiertes Carbamoyl darstellt, und Salzen von solchen Verbindungen, die eine salzbildende Gruppierung aufweisen, dadurch gekennzeichnet, dass man

a) in einer Verbindung der Formel

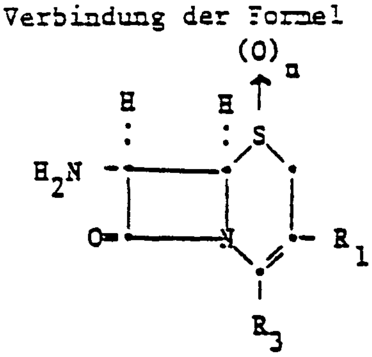

(II),

worin die 7β-Aminogruppe gegebenenfalls durch eine, die Acylierung erlaubende Gruppe geschützt ist, und worin weitere gegebenenfalls vorhandene funktionelle Gruppen geschützt sein können, die 7β-Aminogruppe durch Behandeln mit einem, den Acylrest einer Carbonsäure der Formel

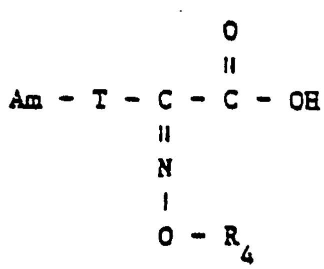

(III)

einführenden Acylierungsmittel, worin weitere, gegebenenfalls vorhandene funktionelle Gruppen in geschützter Form vorliegen können, acyliert, oder

b) eine 2-Cephemverbindung der Formel

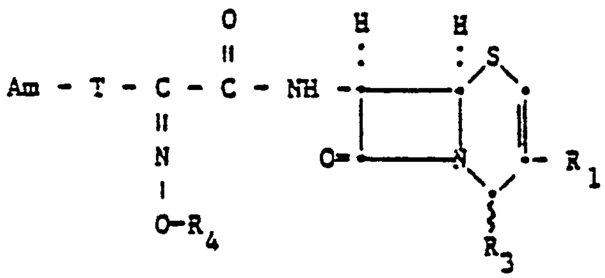

(IV)

worin gegebenenfalls vorhandene funktionelle Gruppen in geschützter Form vorliegen können, zur entsprechenden 3-Cephem-Verbindung isomerisiert, oder

c) eine Verbindung der Formel

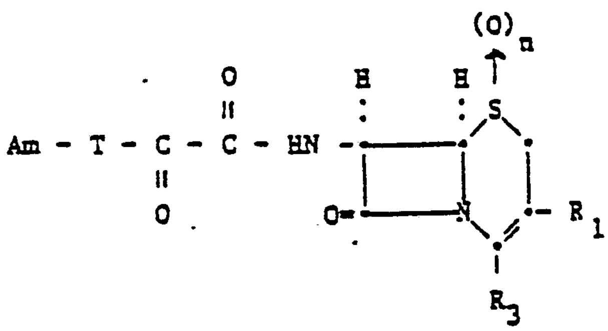

(V)

worin gegebenenfalls vorhandene funktionelle Gruppen in geschützter Form vorliegen können, mit einem gegebenenfalls O-substituierten Hydroxylamin der Formel $H_2N-O-R_4$ (VI) umsetzt, oder

d) zur Herstellung einer Verbindung der Formel I, worin $R_4$ Wasserstoff bedeutet, eine Verbindung der Formel

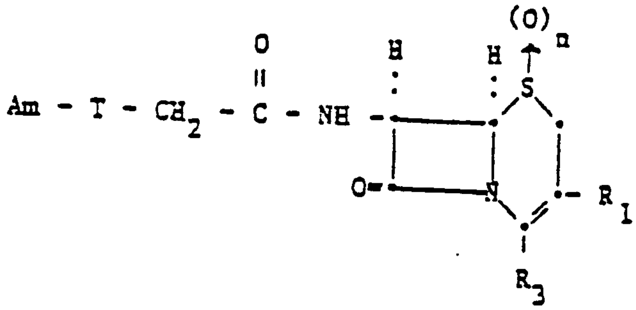

(VII),

worin gegebenenfalls vorhandene funktionelle Gruppen in geschütz- Form vorliegen können, mit einem Nitrosierungsmittel behandelt, oder

e) zur Herstellung einer Verbindung der Formel I, worin Am eine freie Aminogruppe bedeutet, und T für den zweiwertigen 1,2,4-Thiadiazolrest darstellt, dessen 5-Stellung mit der Aminogruppe Am und dessen 3-Stellung mit der Gruppe der Formel $-C(=N-O-R_4)-$ verbunden ist, eine Verbindung der Formel

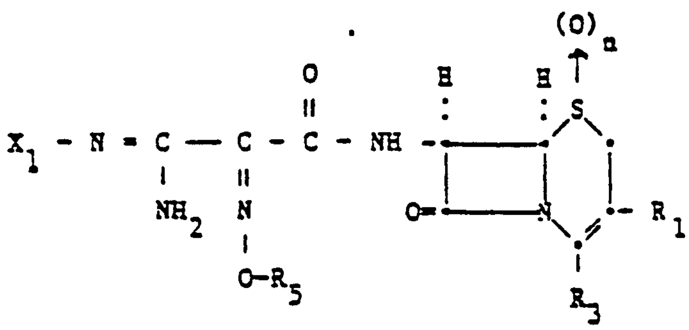

(VIII)

worin weitere, gegebenenfalls vorhandene funktionelle Gruppen in geschützter Form vorliegen können, und worin $X_1$ Halogen bedeutet, mit einem Salz der Rhodanwasserstoffsäure, oder worin $X_1$ Wasserstoff ist, mit Rhodan behandelt, oder

f) zur Herstellung einer Verbindung der Formel I, worin $R_1$ für Wasserstoff steht,

fa) in einer Verbindung der Formel

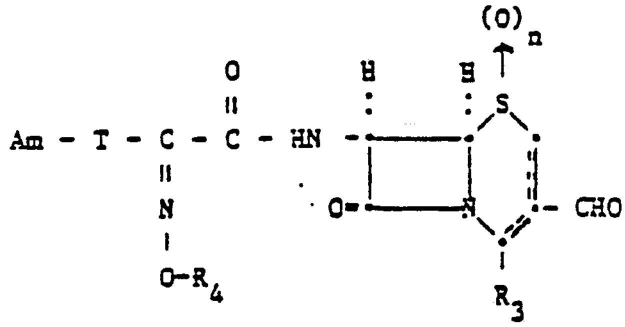

(IX)

worin die Doppelbindung in 2,3- oder in 3,4-Stellung sein kann, und worin gegebenenfalls vorhandene funktionelle Gruppen in geschützter Form vorliegen können, die Formylgruppe mittels Decarbonylierung durch Wasserstoff ersetzt, oder

fb) in einer Verbindung der Formel

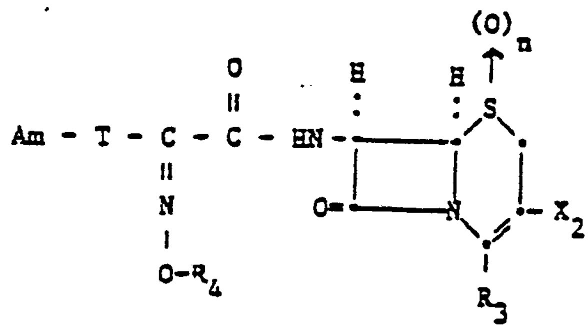

(X)

worin $X_2$ Hydroxy oder gegebenenfalls substituiertes Amino darstellt, und worin weitere gegebenenfalls vorhandene funktionelle Gruppen in geschützter Form vorliegen können, oder eine entsprechende tautomere Verbindung davon, $X_2$ durch Wasserstoff ersetzt, oder

fc) eine Verbindung der Formel

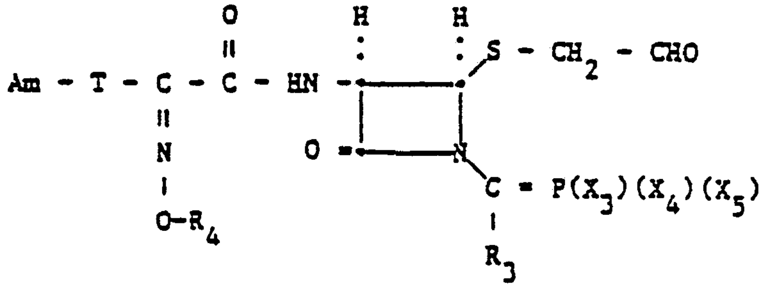

(XI)

worin $X_3$, $X_4$ und $X_5$ jeweils gegebenenfalls substituierte Kohlenwasserstoffreste darstellen, und worin weitere gegebenenfalls vorhandene funktionelle Gruppen in geschützter Form vorliegen, unter Abspaltung einer Verbindung der Formel $O = P(X_3)(X_4)(X_5)$ (XII) ringschliesst, oder

g) zur Herstellung einer Verbindung der Formel I, worin $R_1$ für veräthertes oder verestertes Hydroxy steht,

ga) eine Verbindung der Formel

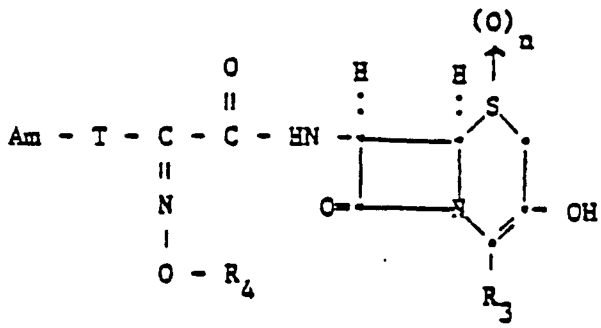

(XIII)

oder die entsprechende Tautomere 3-Oxo-cepham-Verbindung, worin $R_3$ funktionell abgewandeltes Carboxyl darstellt, und weitere gegebenenfalls vorhandene funktionelle Gruppen in geschützter Form vorliegen können, mit einem veräthernden oder veresternden Mittel behandelt, oder

gb) eine Verbindung der Formel

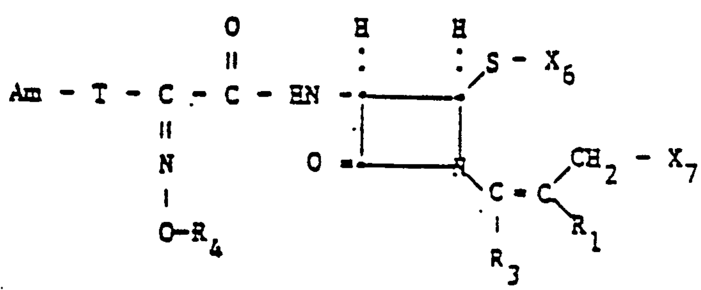

(XIV)

worin $X_6$ eine Abgangsgruppe darstellt, $X_7$ Wasserstoff oder Halogen bedeutet, und $R_1$ für veräthertes oder verestertes Hydroxy steht, und worin weitere gegebenenfalls vorhandene funktionelle Gruppen in geschützter Form vorliegen können, unter Abspaltung der Gruppen $X_6$ und $X_7$ ringschliesst, oder

h) zur Herstellung einer Verbindung der Formel I, worin $R_1$ den Rest der Formel $-CH_2-R_2$ darstellt, worin $R_2$ eine Ammoniogruppe bedeutet, eine Verbindung der Formel

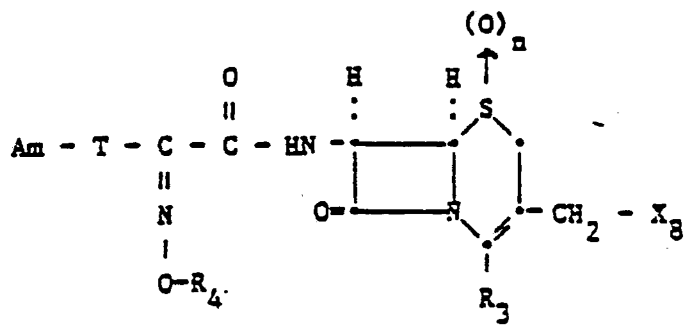

(XV)

worin $X_8$ einen durch nucleophile Substitution ersetzbaren Rest darstellt, und worin gegebenenfalls vorhandene funktionelle Gruppen in geschützter Form vorliegen können, mit einer organischen tertiären Aminbase umsetzt, oder

i) zur Herstellung einer Verbindung der Formel I, worin $R_1$ für den Rest der Formel $-CH_2-R_2$ darstellt, worin $R_2$ Acyl oder gegebenenfalls funktionell abgewandeltes Carboxy darstellt, eine Verbindung der Formel XIII oder eine entsprechende tautomere 3-Oxo-cepham-Verbindung, worin die Gruppe $R_3$ als funktionell abgewandelte Carboxylgruppe vorliegt, und worin andere, gegebenenfalls vorhandene funktionelle Gruppen in geschützter Form vorliegen können, mit einem Ylid der Formel $X_9^{\oplus}-{}^{\ominus}CH-R_2$ (XVI), worin $X_9^{\oplus}$ eine dreifach substituierte Phosphoniumgruppe oder eine zweifach veresterte Phosphonogruppe zusammen mit einem Kation bedeutet, und worin $R_2$ für Acyl oder eine gegebenenfalls funktionell abgewandelte Carboxylgruppe steht, umsetzt, und wenn erwünscht, eine erfindungsgemäss erhältliche Verbindung der

Formel I in eine andere Verbindung der Formel I umwandelt, und/oder, wenn erwünscht oder notwendig, in einer erfindungsgemäss erhältlichen Verbindung der Formel I eine in geschützter Form vorliegende funktionelle Gruppe in die freie funktionelle Gruppe überführt, und/oder, wenn erwünscht, ein erfindungsgemäss erhältliches Salz in die freie Verbindung oder in ein anderes Salz überführt, und/oder, wenn erwünscht, eine erfindungsgemäss erhältliche freie Verbindung mit einer salzbildenden Gruppe in ein Salz überführt, und/oder, wenn erwünscht, ein erfindungsgemäss erhältliches Gemisch von isomeren Verbindungen der Formel I in die einzelnen Isomeren auftrennt.

3. Verfahren gemäss Anspruch 1 zur Herstellung von Verbindungen der Formel I, worin n für 0 steht, $R_1$ für Wasserstoff, Niederalkoxy, Halogen, Niederalkylthio, Heterocyclylthio, worin Heterocyclyl für einen fünf- oder sechsgliedrigen monocyclischen, gegebenenfalls partiell gesättigten, aromatischen Heterocyclylrest steht, der mindestens 1 Ringstickstoffatom und gegebenenfalls 1-3 zusätzliche Ringstickstoffatome und/oder ein Ringsauerstoff- oder Ringschwefelatom enthält, und der durch gegebenenfalls Substituenten, wie Hydroxy, Amino, Diniederalkylamino, Carboxy, Carbamoyl oder Sulfo enthaltendes Niederalkyl, durch gegebenenfalls Substituenten, z.B. Niederalkyl, Niederalkoxy und/oder Halogen, enthaltendes Phenyl, oder durch Thienyl substituiert sein kann, oder für den Rest der Formel $-CH_2-R_2$ steht, worin $R_2$ Niederalkanoyl, Carboxy, Niederalkoxycarbonyl oder gegebenenfalls durch Carbamoyl substituiertes Pyridinio bedeutet, $R_3$ Carboxy, Niederalkanoyloxymethoxycarbonyl , (2-Amino-niederalkanoyl)-oxymethoxycarbonyl oder geschütztes Carboxy darstellt, Am für Amino, Niederalkylamino , Diniederalkylamino, oder für geschütztes Amino steht, T einen zweiwertigen, über die beiden Ringkohlenstoffatome gebundenen Thiadiazolrest bedeutet, und $R_4$ Wasserstoff, gegebenenfalls durch Niederalkoxy, Amino, Diniederalkylamino oder Carboxy substituiertes Niederalkyl oder Cycloalkyl, oder gegebenenfalls N-niederalkyliertes Carbamoyl steht, wobei der Rest der Formel $=N-O-R_4$ die syn(oder Z)- oder anti(oder E)-Form aufweisen kann, sowie von Salzen solcher Verbindungen der Formel I, die salzbildende Gruppen aufweisen.

4. Verfahren gemäss Anspruch 2 zur Herstellung von Verbindungen der Formel I, worin n für 0 oder 1 steht, $R_1$ für Wasserstoff, Niederalkoxy, Halogen, Niederalkylthio, Heterocyclylthio, worin Heterocyclyl für einen fünf- oder sechsgliedrigen monocyclischen, gegebenenfalls partiell gesättigten, aromatischen Heterocyclylrest steht, der mindestens 1 Ringstickstoffatom und gegebenenfalls 1-3 zusätzliche Ringstickstoffatome und/oder ein Ringsauerstoff- oder Ringschwefelatom enthält, und der durch gegebenenfalls Substituenten enthaltendes Niederalkyl oder Phenyl, oder durch Thienyl substituiert sein kann, oder für den Rest der Formel $-CH_2-R_2$ steht, worin $R_2$ Niederalkanoyl, Carboxy, Niederalkoxycarbonyl oder gegebenenfalls durch Carbamoyl substituiertes Pyridinio bedeutet, $R_3$ Carboxy, Niederalkanoyloxymethoxycarbonyl (2-Amino-niederalkanoyl)-oxymethoxycarbonyl oder geschütztes Carboxy darstellt, Am für Amino, Niederalkylamino, Diniederalkylamino oder für geschütztes Amino steht, T einen zweiwertigen, über die beiden Ringkohlenstoffatome gebundenen Thiadiazolrest bedeutet, und $R_4$ für Wasserstoff, gegebenenfalls durch Niederalkoxy, Diniederalkylamino oder Carboxy substituiertes Niederalkyl oder Cycloalkyl, oder gegebenenfalls N-niederalkyliertes Carbamoyl steht, wobei der Rest der Formel $=N-O-R_4$ die syn(oder Z)- oder anti (oder E)-Form aufweisen kann, sowie von Salzen solcher Verbindungen der Formel I, die salzbildende Gruppen aufweisen.

5. Verfahren gemäss Anspruch 1, zur Herstellung von Verbindungen der Formel I, worin n für 0 steht, $R_1$ für Wasserstoff, Niederalkoxy, z.B. Methoxy. Halogen, z.B. Chlor, Heterocyclylthio, worin Heterocyclyl gegebenenfalls durch Niederalkyl, z.B. Methyl, substituiertes Oxadiazolyl oder Thiadiazolyl, wie 1,3,4-Oxadiazol-2-yl oder 1,3,4-Thiadiazol-2-yl, gegebenenfalls durch Niederalkyl, z.B. Methyl, substituiertes Triazolyl, wie 1,2,3-Triazol-4-yl, oder gegebenenfalls durch Niederalkyl, z.B. Methyl oder Aethyl, das Diniederalkylamino, z.B. Dimethylamino, Carboxy, Carbamoyl oder Sulfo als Substituenten enthalten kann, substituiertes 1H-Tetrazol-5-yl bedeutet, oder einen Rest der Formel $-CH_2-R_2$ steht, worin $R_2$ gegebenenfalls durch Carbamoyl substituiertes Pyridinio bedeutet, $R_3$ Carboxy, Niederalkanoyloxymethoxycarbonyl, z.B. Pivaloyloxymethoxycarbonyl, oder geschütztes Carboxy darstellt, Am für Amino, Methylamino, oder geschütztes Amino steht, T den zweiwertigen, über die beiden Ringkohlenstoffatome gebundenen 1,2,4-Thiadiazolrest bedeutet, wobei das Ringkohlenstoffatom der 5-Stellung vorzugsweise mit der Gruppe Am verbunden ist, und $R_4$ Wasserstoff, Niederalkyl, z.B. Methyl, Carboxyniederalkyl, z.B. 2-Carboxy-2-propyl, Carbamoyl, Niederalkylcarbamoyl, z.B. N-Methylcarbamoyl, oder Aminoniederalkyl, z.B. 2-Aminoäthyl, darstellt, wobei der Rest der Formel $=N-O-R_4$ vorzugsweise die syn(oder Z)-Form aufweist, sowie von Salzen solcher Verbindungen der Formel I, die salzbildende Gruppen aufweisen.

6. Verfahren gemäss Anspruch 2 zur Herstellung von Verbindungen der Formel I, worin n für 0 steht, $R_1$ für Wasserstoff, Niederalkoxy, Halogen, Heterocyclylthio, worin Heterocyclyl gegebenenfalls durch Niederalkyl substituiertes Oxadiazolyl oder Thiadiazolyl, gegebenenfalls durch Niederalkyl substituiertes Triazolyl oder gegebenenfalls durch Niederalkyl, das Diniederalkylamino, Carboxy, Carbamoyl oder Sulfo als Substituenten enthalten kann, substituiertes 1H-Tetrazol-5-yl bedeutet, oder für einen Rest der Formel $-CH_2-R_2$ steht, worin $R_2$ gegebenenfalls durch Carbamoyl substituiertes Pyridinio bedeutet, $R_3$ Carboxy, Niederalkanoyloxymethoxycarbonyl oder geschütztes Carboxy darstellt, Am für Amino oder geschütztes Amino steht, T den zweiwertigen, über die beiden Ringkohlenstoffatome gebunden 1,2,4-Thiadiazolrest bedeutet, wobei das Ringkohlenstoffatom der 5-Stellung vorzugsweise mit der Gruppe Am verbunden ist, und $R_4$ Wasserstoff oder Niederalkyl darstellt, sowie von Salzen solcher Verbindungen der Formel I, die salzbildende Gruppen aufweisen.

7. Verfahren gemäss Anspruch 1 zur Herstellung von Verbindungen der Formel I, in welcher n für 0 steht, $R_1$ für Wasserstoff, Niederalkoxy, insbesondere Methoxy, Halogen, insbesondere Chlor oder den Rest der Formel $-CH_2R_2$ steht, worin $R_2$ gegebenenfalls Carbamoyl enthaltendes Pyridinio, insbesondere 4-Carbamoylpyridinio, bedeutet, $R_3$ Carboxy oder Niederalkanoyloxymethoxycarbonyl, insbesondere Pivaloyloxymethoxycarbonyl bedeutet, Am Amino oder Methylamino ist, T den zweiwertigen 1,2,4-Thiadiazolrest darstellt, der mit dem Ringkohlenstoffatom der 5-Stellung mit der Aminogruppe Am und mit dem Ringkohlenstoffatom der 3-Stellung mit der Gruppe der Formel $-C(=N-O-R_4)-$ verbunden ist, und $R_4$ für Wasserstoff, Niederalkyl, insbesondere Methyl, Carboxyniederalkyl, z.B. 2-Carboxy-2-Propyl, Carbamoyl oder Niederalkylcarbamoyl, z.B. N-Methylcarbamoyl, steht, wobei die Gruppe der Formel $=N-O-R_4$ die syn(oder Z)-Form aufweist, sowie von Salzen solcher Verbindungen der Formel I.

8. Verfahren gemäss Anspruch 2 zur Herstellung von Verbindungen der Formel I, in welcher n für 0 steht, $R_1$ für Wasserstoff, Niederalkoxy, Halogen, 1-Niederalkyl-1H-5-tetrazolylthio oder den Rest der Formel $-CH_2-R_2$ steht, worin $R_2$ gegebenenfalls Carbamoyl enthaltendes Pyridinio bedeutet, $R_3$ Carboxy oder Niederalkanoyloxymethoxycarbonyl bedeutet, Am Amino ist, T den zweiwertigen 1,2,4-Thiazolrest darstellt, der mit dem Ringkohlenstoffatom der 5-Stellung mit der Aminogruppe Am und mit dem Ringkohlenstoffatom der 3-Stellung mit der Gruppe der Formel $-C(=N-O-R_4)-$ verbunden ist, und $R_4$ für Niederalkyl steht, wobei die Gruppe der Formel $=N-O-R_4$ die syn(oder Z)-Form aufweist, sowie von Salzen solcher Verbindungen der Formel I.

9. Verfahren zur Herstellung von 7β-[2-(5-Amino-1,2,4-Thiadiazol-3-yl)-2-Z-methoxyiminoacetylamino]-3-cephem-4-carbonsäure und Salzen davon gemäss Anspruch 2.

10. Verfahren zur Herstellung von 7β-[2-(5-Amino-1,2,4-thiadiazol-3-yl)-2-Z-(2-carboxyprop-2-yl moylpyridiniomethyl)-3-cephem-4-carboxylat gemäss Anspruch 1.

11. Verfahren zur Herstellung von 7β-[2-(5-Amino-1,2,4-thiadiazol-3-yl)-2-Z-methoxyiminoacetylamino]-3-methoxy-3-cephem-4-carbonsäure und Salzen davon gemäss Anspruch 1.

12. Verfahren zur Herstellung von 7β-[2-(5-Amino-1,2,4-thiadiazol-3-yl)-2-Z-methylcarbamoyloxyiminoacetylamino]-3-cephem-4-carbonsäure und Salzen davon gemäss Anspruch 1.

13. Verfahren zur Herstellung von 7β-[2-(5-Amino-1,2,4-thiadiazol-3-yl)-2-Z-methylcarbamoyloxyiminoacetylamino]-3-(4-carbamoylpyridiniomethyl)-3-cephem-4-carboxylat gemäss Anspruch 1.

14. Verfahren zur Herstellung von 7β-[2-(5-Amino-1,2,4-thiadiazol-3-yl)-2-Z-hydroxyimino-acetylamino]-3-cephem-4-carbonsäure und Salzen davon gemäss Anspruch 1.

15. Verfahren zur Herstellung von 7β-[2-(5-N-Methylamino-1,2,4-thiadiazol-3-yl)-2-Z-methoxyimino-acetylamino]-3-cephem-4-carbonsäure und Salzen davon gemäss Anspruch 1.

0025017

Europäisches Patentamt

# EUROPÄISCHER RECHERCHENBERICHT

Nummer der Anmeldung
EP 80 81 0261

| EINSCHLÄGIGE DOKUMENTE | | | KLASSIFIKATION DER ANMELDUNG (Int Cl 3) |
|---|---|---|---|
| Kategorie | Kennzeichnung des Dokuments mit Angabe, soweit erforderlich der maßgeblichen Teile | betrifft Anspruch | |
| | FR - A - 2 384 780 (FUJISAWA)<br>* Seite 5, Zeilen 16-22; Seiten 56-62; Ansprüche *<br>-- | 1-21 | C 07 D 501/20<br>501/24<br>501/59<br>A 61 K 31/545//<br>C 07 D 285/08 |
| | FR - A - 2 383 951 (FUJISAWA)<br>* Seiten 141-165; Ansprüche 1-113 *<br>-- | 1-21 | |
| P | EP - A - 0 007 470 (FUJISAWA)<br>* Seiten 123-132; Ansprüche *<br>-- | 1-21 | RECHERCHIERTE SACHGEBIETE (Int Cl 3)<br>C 07 D 501/20<br>501/24<br>501/59<br>501/00 |
| P | EP - A - 0 013 762 (FUJISAWA)<br>* Seiten 1-17; Ansprüche 1-19 *<br>----- | 1-21 | |

KATEGORIE DER GENANNTEN DOKUMENTE
- X: von besonderer Bedeutung
- A: technologischer Hintergrund
- O: nichtschriftliche Offenbarung
- P: Zwischenliteratur
- T: der Erfindung zugrunde liegende Theorien oder Grundsätze
- E: kollidierende Anmeldung
- D: in der Anmeldung angeführtes Dokument
- L: aus andern Gründen angeführtes Dokument
- &: Mitglied der gleichen Patentfamilie, übereinstimmendes Dokument

X Der vorliegende Recherchenbericht wurde für alle Patentansprüche erstellt

| Recherchenort | Abschlußdatum der Recherche | Prüfer |
|---|---|---|
| Den Haag | 13-11-1980 | LUYTEN |